# EUROPEAN PATENT APPLICATION

(11) **EP 4 585 603 A1**
(43) Date of publication of application: **16.07.2025**
(21) Application number: 23950499.6
(22) Date of filing: 01.12.2023
(51) Int. Cl.: C07H 15/18, A61K 47/54, A61K 31/713, A61K 31/7105, A61K 31/7088, A61P 3/06, A61P 1/16, A61P 9/10, A61P 29/00

(54) **GALNAC COMPOUND COMPRISING RIBOSE RING OR DERIVATIVE STRUCTURE THEREOF, AND OLIGONUCLEOTIDE CONJUGATE THEREOF**

(30) Priority: 01.09.2023 CN 202311118366
(71) Applicant: Hangzhou Tianlong Pharmaceutical Co., Ltd., Hangzhou, Zhejiang 310009 (CN)
(72) Inventor: SONG, Gengshen, Beijing 100176 (CN); HUANG, Zeao, Beijing 100176 (CN); LI, Zhenmin, Beijing 100176 (CN); TIAN, Zhikang, Beijing 100176 (CN); YANG, Shuo, Beijing 100176 (CN); YAO, Peng, Beijing 100176 (CN); GAO, Zhongcai, Beijing 100176 (CN); YU, Xiaowen, Beijing 100176 (CN); HUANG, Dawei, Beijing 100176 (CN); PANG, Xue, Beijing 100176 (CN); HOU, Yuhua, Beijing 100176 (CN); WANG, Jie, Beijing 100176 (CN); LIN, Fang, Beijing 100176 (CN); CHANG, Yanan, Beijing 100176 (CN)
(74) Representative: Potter Clarkson
(86) International application number: PCT/CN2023/135929
(87) International publication number: WO 2025/043931

(57) **Abstract**

Provided are a GalNAc compound containing a ribose ring or its derivative structure and an oligonucleotide conjugate thereof. The oligonucleotide conjugate can achieve efficient liver-targeted delivery and improve the efficacy of the drug.

## Description

This application claims the priority of Chinese Patent Application No. 202311118366.4, filed with the China National Intellectual Property Administration on September 1, 2023, and titled with "GALNAC COMPOUND CONTAINING RIBOSE RING OR ITS DERIVATIVE STRUCTURE AND OLIGONUCLEOTIDE CONJUGATE THEREOF", which is hereby incorporated by reference in its entirety.

### FIELD

The present disclosure relates to the field of biomedicine, and in particular to a GalNAc compound having a ribose ring structure and a GalNAc oligonucleotide conjugate prepared therefrom.

### BACKGROUND

Nucleic acid drugs, particularly oligonucleotide drugs, are widely employed due to their straightforward synthesis and high activity. Oligonucleotide drugs typically encompass antisense oligonucleotides (ASOs), small interfering RNA (siRNA), microRNA (miRNA), and nucleic acid aptamers.

Oligonucleotides are a class of short DNA or RNA molecules or oligomers that readily bind to their respective complementary oligonucleotides, DNA or RNA in a sequence-specific manner to form duplexes, or less commonly, higher order hybrids. This fundamental characteristic makes oligonucleotides widely used in genetic testing, as well as in research and medicine related to targeted gene therapy. These small fragments of nucleic acid can be prepared into single-stranded molecules with any specified sequence. In nature, oligonucleotides are typically small RNA molecules that regulate gene expression or intermediates derived from the degradation of large nucleic acid molecules.

RNA interference is a natural defense mechanism against foreign genes. siRNA can knock out target genes by recognizing specific sequences and degrading target mRNA.

N-acetylgalactosamine (GalNAc) is a ligand that binds to the asialoglycoprotein receptor (ASGPR) on the liver surface. ASGPR is an endocytic receptor that is specifically expressed on the surface of hepatocytes. In recent years, there has been some progress in the liver-targeted delivery of nucleic acid drugs using GalNAc, a high-affinity ligand of ASGPR, as a targeting molecule. For instance, Alnylam Pharmaceuticals, Inc. reported that siRNA based on GalNAc conjugation technology exhibited gene silencing activity in mice (Nair JK, et al. J. Am. Chem. Soc. 2014, 136, 16958). The article indicated that the conjugate of GalNAc and siRNA exhibited satisfactory delivery efficacy in both *in vivo* and *in vitro* experiments. In the *in vivo* experiments where mice were subcutaneously administered, the ED₅₀ of a single dose was determined to be 1 mg/kg, with the single injection dose being less than 1 mL. In long-term administration experiments, subcutaneous injection once a week has been demonstrated to result in stable interference activity for up to nine months. Studies have found that tetraantennary and triantennary GalNAc compounds exhibit a markedly higher affinity for ASGPR than biantennary and monoantennary GalNAc compounds.

Proprotein convertase subtilisin/kexin type 9 (PCSK9), a glycoprotein composed of 692 amino acids, is the ninth member of the proprotein convertase (PCs) family and a secretory serine protease. It is mainly expressed in tissues such as liver and intestines, and subsequently secreted into the blood. Once entering the blood circulation, PCSK9 can specifically bind to the epidermal growth factor-like domain of the low-density lipoprotein receptor (LDL-R) on the surface of hepatocytes, and guide it into the hepatocytes to reach the lysosomes, resulting in the degradation of LDL-R in the lysosomes, thereby reducing LDL-R on the surface of hepatocytes, which impairs the liver's ability to bind and clear LDL-C and ultimately leads to elevated LDL-C levels in the blood. Consequently, the treatment of hypercholesterolemia may be achieved through the inhibition of PCSK9. Furthermore, recent studies have demonstrated that elevated PCSK9 is closely associated with obesity and type 2 diabetes, as well as with chronic kidney diseases such as nephrotic syndrome and proteinuria. Therefore, the inhibition of PCSK9 represents a significant avenue for the prevention and treatment of these related diseases.

Different GalNAc ligand structures exert markedly disparate effects on nucleic acid delivery. In order to enhance the delivery effect of liver-targeted therapeutics, such as PCSK9 inhibitors, there is a continued need in the field to develop novel GalNAc ligand compounds.

### SUMMARY

The present disclosure has designed a series of new GalNAc compounds. Through solid-phase synthesis methods, the new GalNAc compounds of the present disclosure can be efficiently conjugated with oligonucleotides. The GalNAc oligonucleotide conjugates prepared therefrom significantly improve liver-targeted delivery efficiency compared with GalNAc compounds with similar structures in the prior art.

In a first aspect, the present disclosure provides a compound represented by formula (I) or a pharmaceutically acceptable salt thereof: 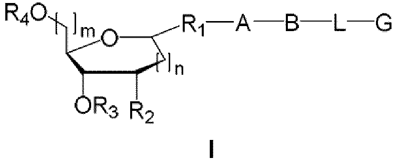 wherein,
R₁ is oxygen or sulfur, preferably oxygen;
R₂ is hydrogen, C₁₋₄ alkyl, C₁₋₄ alkoxy or halogen;
R₃ is hydrogen, a hydroxyl protecting group, a phosphorus-containing active reactive group, 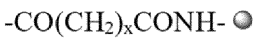 or -CO(CH₂)ₓCOOH, wherein x is an integer selected from 1 to 10, and 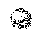 is controlled pore glass or polystyrene;
R₄ is hydrogen or a hydroxyl protecting group;
A is -(CH₂)ₐ-, -(CH₂CH₂O)_{b}-, -((CH₂)_{c}NHCO)_{d}-, or -((CH₂)_{c}CONH)_{d}-, wherein a is an integer selected from 1 to 15, preferably an integer selected from 3 to 13, b is an integer selected from 1 to 7, preferably an integer selected from 2 to 5, c is an integer selected from 1 to 7, preferably an integer selected from 2 to 6, and d is an integer selected from 1 to 5, preferably an integer selected from 1 to 3;
B is -(CH₂)ₑ-, wherein e is an integer selected from 0-7, preferably an integer selected from 1 to 5;
L is -CONH- or -NHCO-;
G is 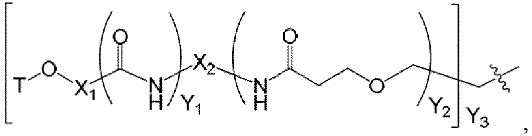
wherein,
T is N-acetyl-galactosamine with hydroxyl fully protected by acyl, galactose with hydroxyl fully protected by acyl, galactosamine with hydroxyl fully protected by acyl, N-formyl-galactosamine with hydroxyl fully protected by acyl, N-propionyl-galactosamine with hydroxyl fully protected by acyl, N-n-butyryl-galactosamine with hydroxyl fully protected by acyl, or N-isobutyryl-galactosamine with hydroxyl fully protected by acyl, preferably N-acetyl-galactosamine with hydroxyl fully protected by acyl, wherein the acyl is, for example, acetyl or benzoyl, preferably acetyl;
X₁ is -(CH₂)_{f}- or -(CH₂CH₂O)_{f}CH₂-, wherein f is an integer selected from 1 to 5;
X₂ is -(CH₂)_{g}-, wherein g is an integer selected from 1 to 6;
Y₁ is 0 or 1;
Y₂ is 0, 1 or 2;
Y₃ is 1, 2 or 3;
m is an integer selected from 0 to 4, preferably 0, 1 or 2; and
n is an integer selected from 0 to 4, preferably 0, 1 or 2.

In an embodiment, A is -(CH₂)₁₀-, -(CH₂)₇-, -(CH₂)₈-, -(CH₂)₉-, -(CH₂)₁₁-, -(CH₂)₁₂-, -(CH₂CH₂O)₃-, -(CH₂)₄NHCO-, or -(CH₂)₆NHCO-.

In an embodiment, B is -(CH₂)₀-, -CH₂-, -(CH₂)₂-, -(CH₂)₄-, or -(CH₂)₃-.

In an embodiment, R₁ is oxygen.

In an embodiment, R₁ has an α configuration or a β configuration.

In an embodiment, R₂ is hydrogen or -OCH₃.

In an embodiment, R₃ is a hydroxyl protecting group or a phosphorus-containing active reactive group. Preferably, R₃ is acyl, silyl, trityl, monomethoxytrityl, 4,4'-dimethoxytrityl or 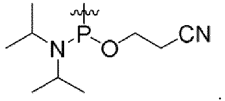 More preferably, R₃ is selected from the group consisting of acetyl, 1,1,3,3-tetraisopropyldisiloxanylidene (TIPDS), t-butyldimethylsilyl, phenyldimethylsilyl, 4,4'-dimethoxytrityl and

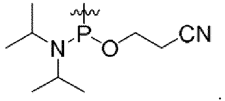

In another embodiment, R₃ is 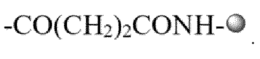 In yet another embodiment, R₃ is -CO(CH₂)₂COOH.

In an embodiment, R₄ is a hydroxy protecting group, preferably selected from the group consisting of trityl, monomethoxytrityl and 4,4'-dimethoxytrityl, more preferably 4,4'-dimethoxytrityl.

In an embodiment, m is 1.

In an embodiment, n is 0.

In an embodiment, G is 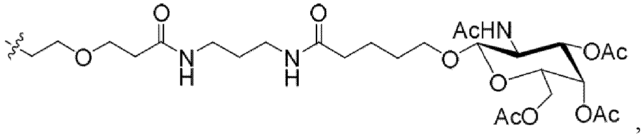 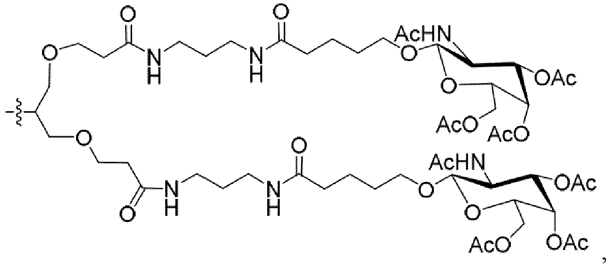 or

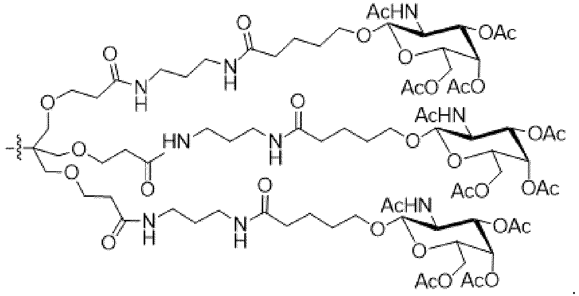

In an embodiment, the compound represented by formula (I) is YK-GAL-301, YK-GAL-302, YK-GAL-303, YK-GAL-304, YK-GAL-305, YK-GAL-306, YK-GAL-307, YK-GAL-308, YK-GAL-309, YK-GAL-310, YK-GAL-311, YK-GAL-312, or YK-GAL-313: 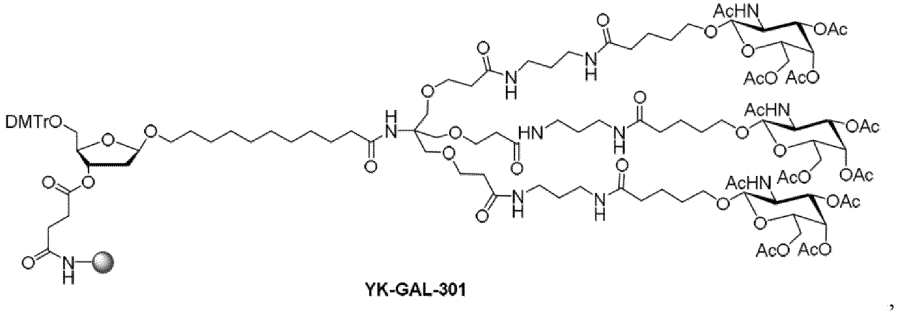 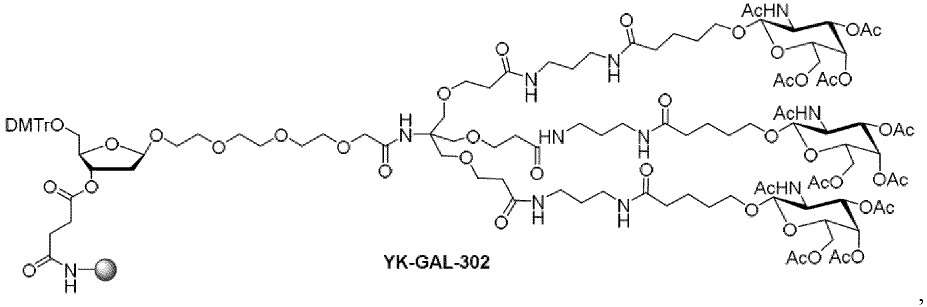 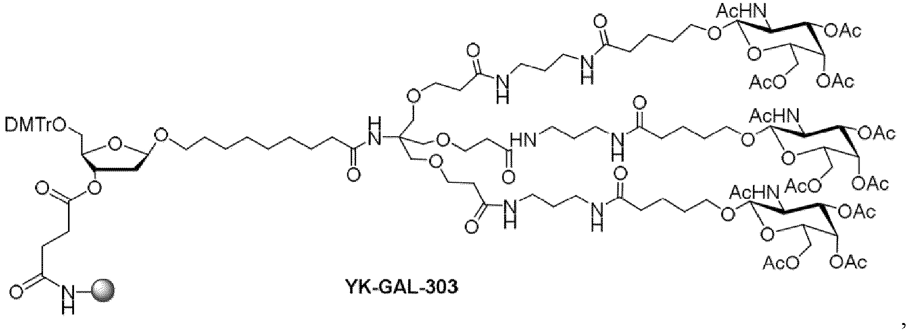 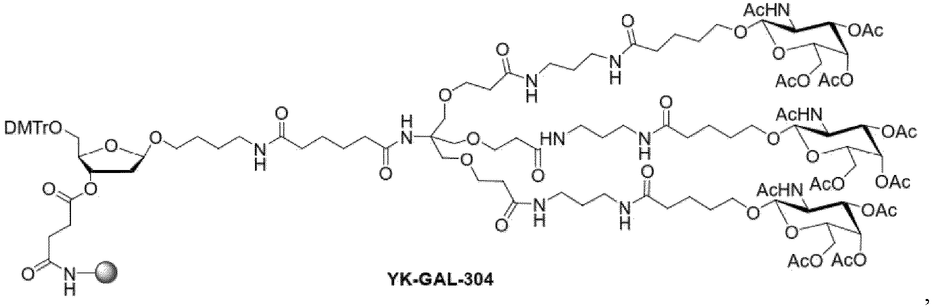 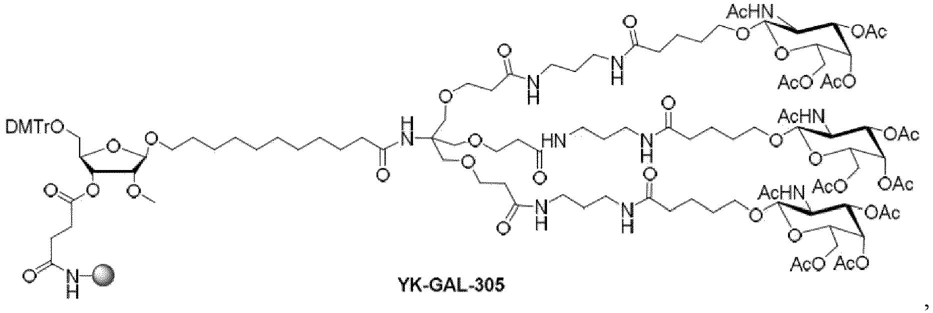 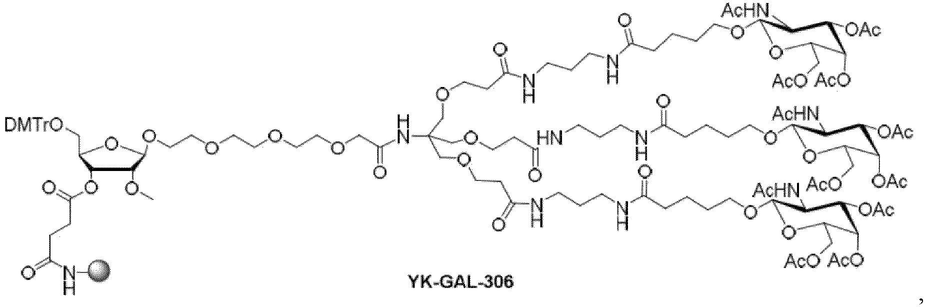 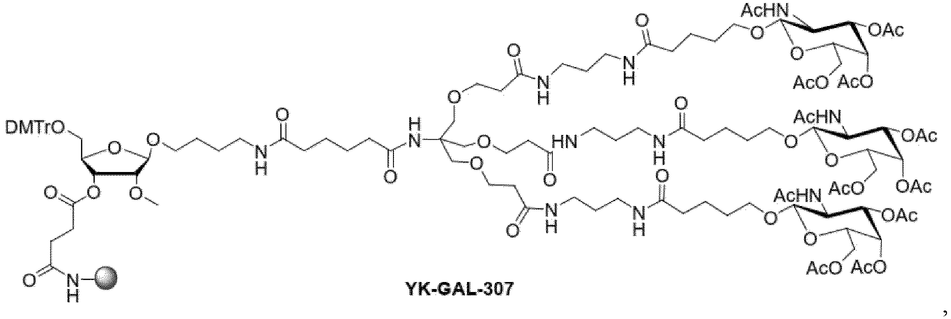 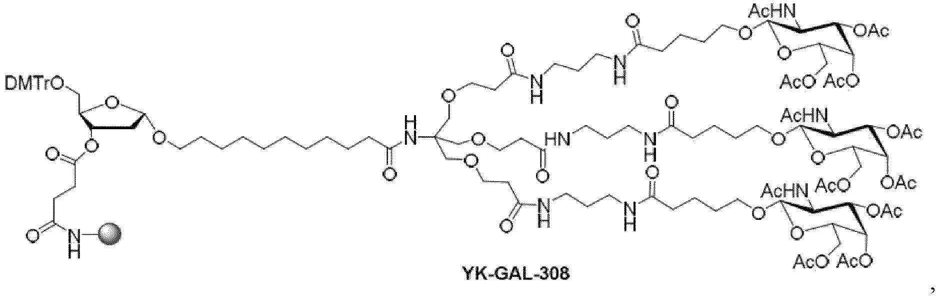 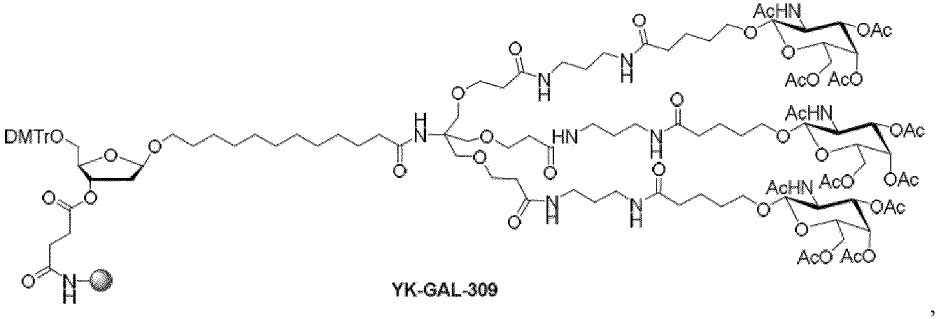 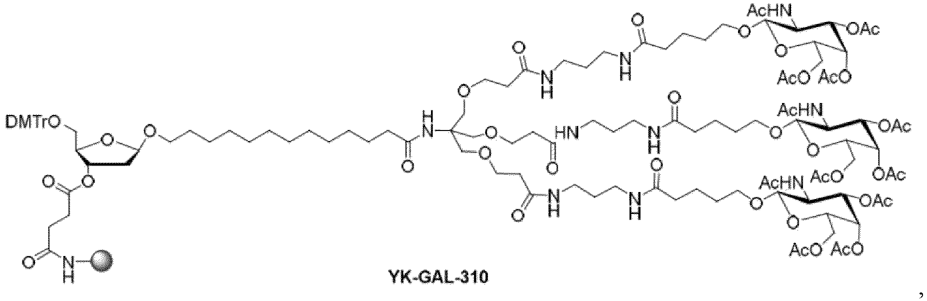 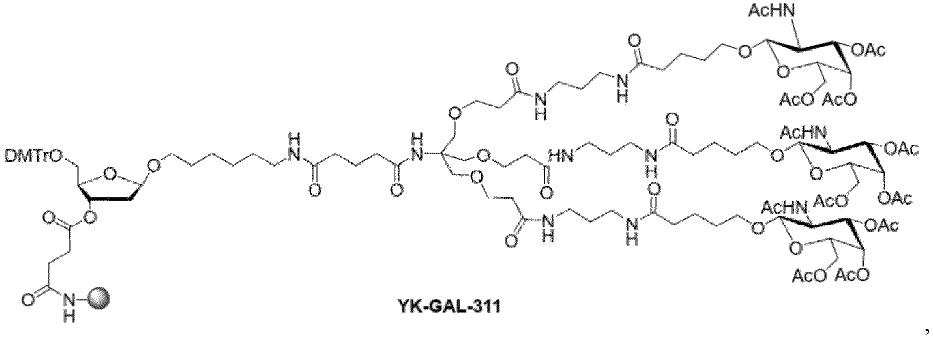 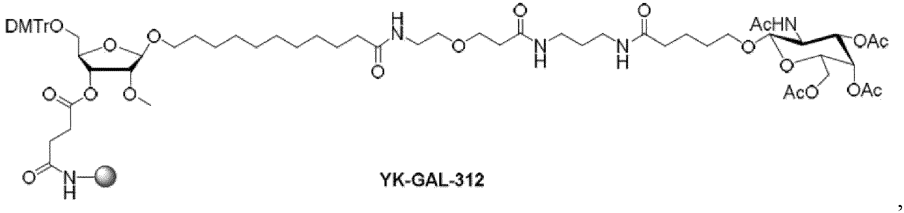 or 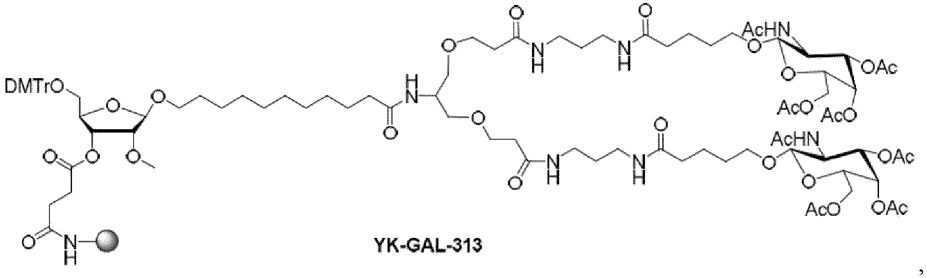 wherein 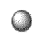 is controlled pore glass.

In another embodiment, the compound represented by formula (I) is YK-GAL-314, YK-GAL-315, YK-GAL-316, YK-GAL-317, YK-GAL-318, YK-GAL-319, YK-GAL-320, YK-GAL-321, YK-GAL-322, YK-GAL-323, YK-GAL-324, YK-GAL-325 or YK-GAL-326: 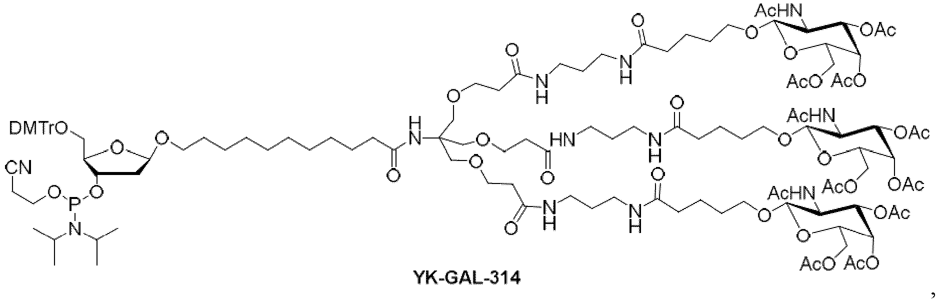 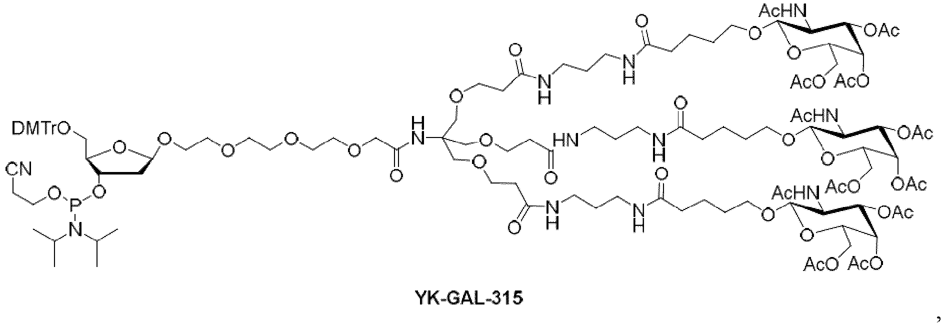 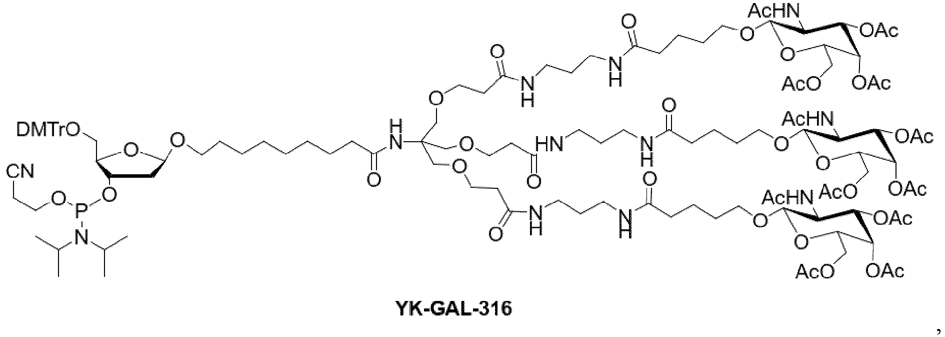 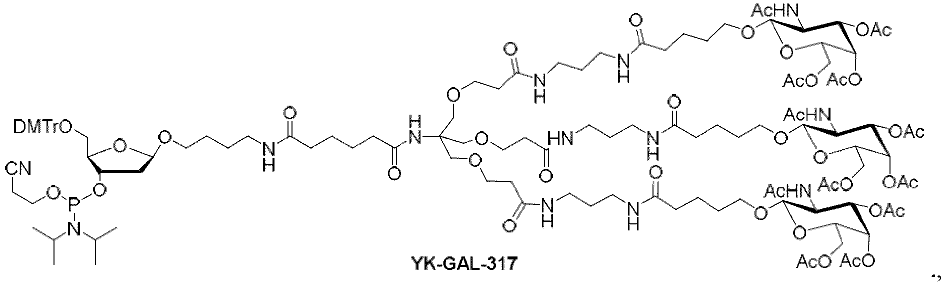 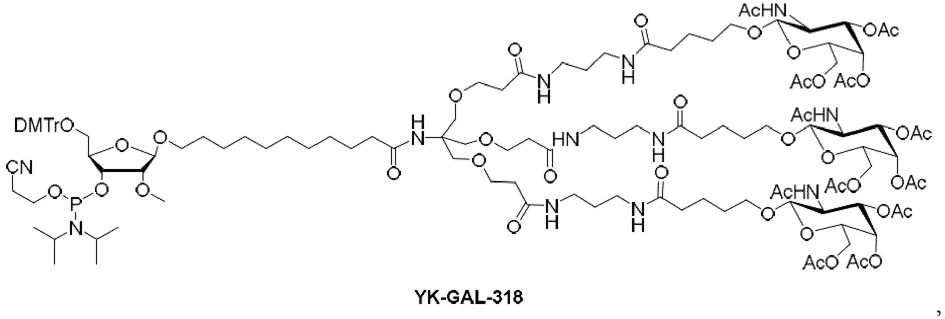 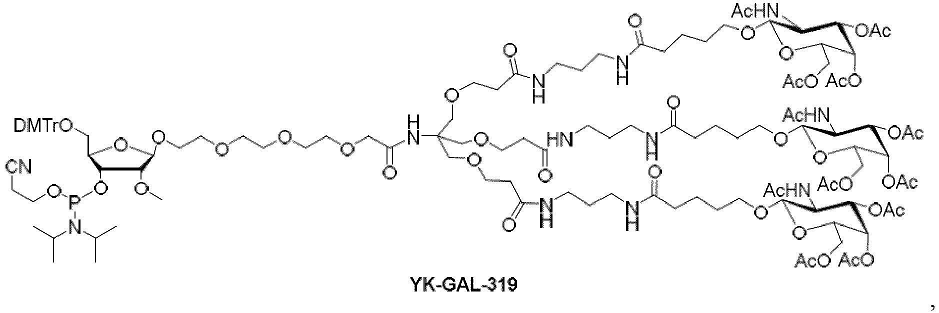 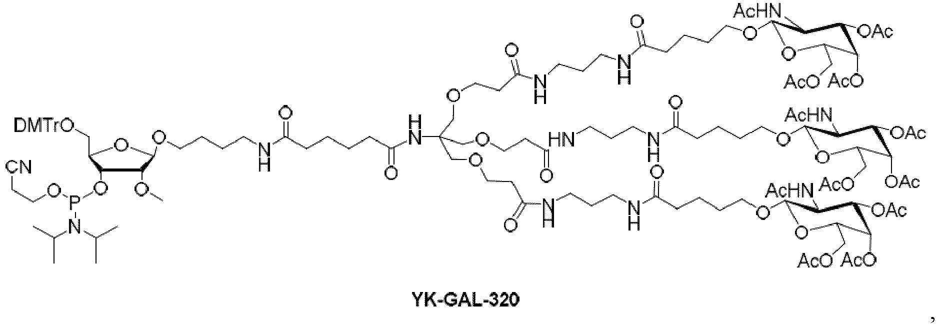 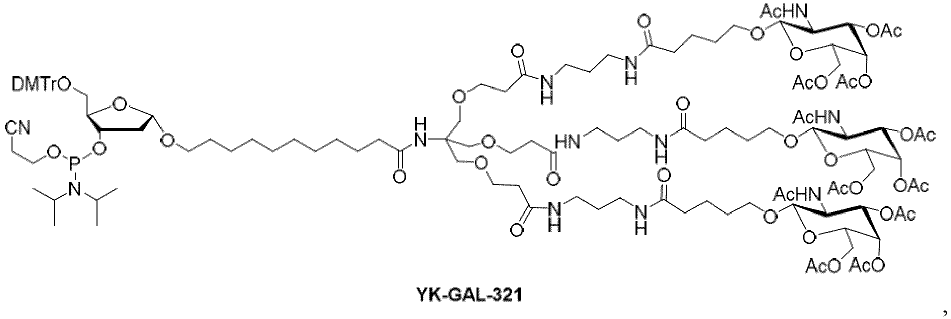 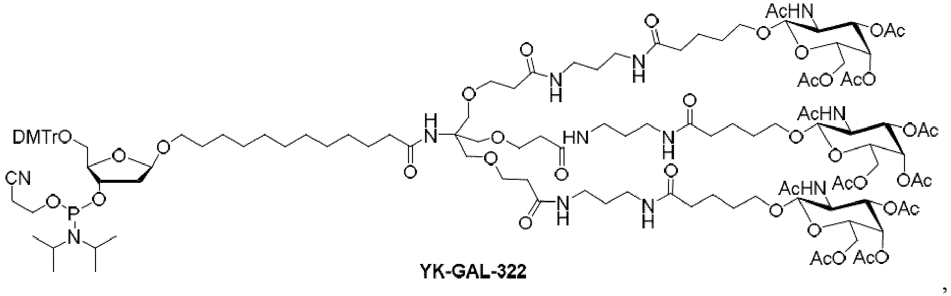 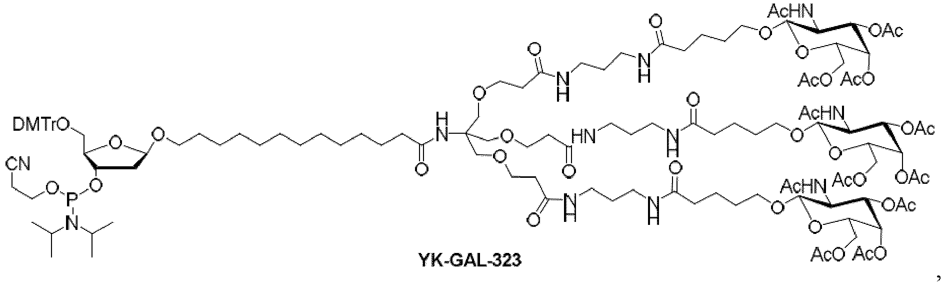 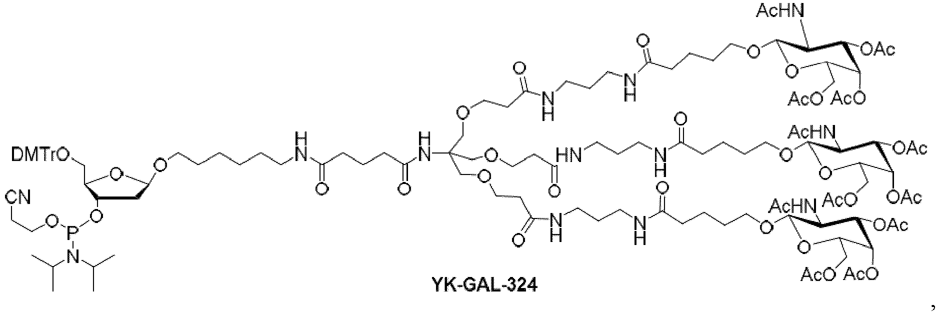 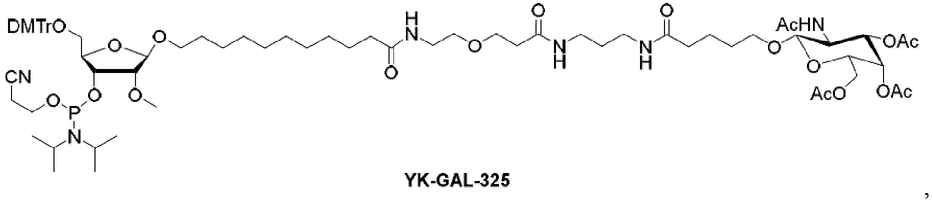 or

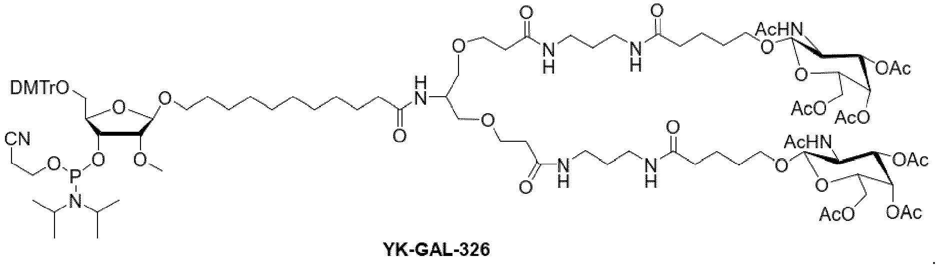

Optionally, the compound represented by formula (I) or the pharmaceutically acceptable salt thereof is capable of binding to asialoglycoprotein receptor (ASGPR).

In a second aspect, the present disclosure provides a conjugate comprising an oligonucleotide and a GalNAc moiety, wherein the conjugate has a structure of 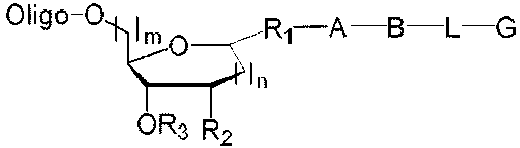 or 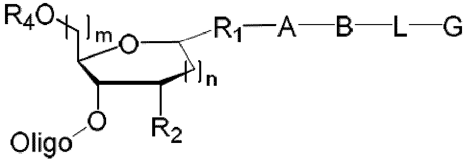 wherein, Oligo represents oligonucleotide, and R₁, R₂, R₃, R₄, A, B, L, G, m and n are as defined above.

Optionally, the hydroxyl protecting group acetyl in the N-acetyl-galactosamine of G in the conjugate is removed.

Optionally, the oligonucleotides include non-thio oligonucleotide and a thio oligonucleotide. In an embodiment, the non-thio oligonucleotide and the GalNAc moiety are linked by a phosphate bond. In another embodiment, the thio oligonucleotide and the GalNAc moiety are linked by a phosphorothioate bond.

Optionally, the oligonucleotides include a small interfering RNA (siRNA), a DNA, a micro RNA (miRNA), a small activating RNA (saRNA), a small guide RNA (sgRNA), a transfer RNA (tRNA), an antisense oligonucleotide (ASO), and an aptamer, preferably an antisense oligonucleotide (ASO) or a small interfering RNA (siRNA). In an embodiment, each nucleotide in the antisense oligonucleotide (ASO) or small interfering RNA (siRNA) is independently a modified or unmodified nucleotide.

Optionally, the oligonucleotide modulates expression of a target gene.

In a third aspect, the present disclosure provides a pharmaceutical composition comprising the conjugate according to the second aspect and at least one pharmaceutically acceptable excipient.

In a fourth aspect, the present disclosure provides use of the conjugate according to the second aspect or the pharmaceutical composition according to the third aspect in the manufacture of a medicament for treating and/or preventing a pathological condition or disease caused by the expression of a specific gene in a hepatocyte; optionally, the specific gene is selected from one of the following gene: hepatitis B virus gene, angiopoietin 3 gene or apolipoprotein C3 gene.

In an embodiment, the disease is selected from chronic liver disease, hepatitis, liver fibrosis, liver proliferative disease and dyslipidemia; optionally, the dyslipidemia is hypercholesterolemia, hypertriglyceridemia or atherosclerosis.

In a fifth aspect, the present disclosure provides a method for inhibiting the expression of a specific gene in a hepatocyte, comprising contacting the hepatocyte with an effective amount of the conjugate according to the second aspect or the pharmaceutical composition according to the third aspect.

Optionally, the specific gene is selected from one of the following gene: proprotein convertase subtilisin/kexin type 9 gene (PCSK9), Apo B, ApoC, ANGPTL3, SCD1, FVII, p53, HBV, and HCV.

Optionally, the specific gene is selected from proprotein convertase subtilisin/kexin type 9 gene (PCSK9), hepatitis B virus gene, angiopoietin-like protein 3 gene, or apolipoprotein C3 gene.

In a sixth aspect, the present disclosure provides a kit comprising the conjugate according to the second aspect.

### BRIEF DESCRIPTION OF DRAWINGS

In order to more clearly illustrate the technical solutions of the embodiments of the present disclosure, the drawings of the embodiments are briefly illustrated below. Apparently, the drawings in the following description only relate to some embodiments of the present disclosure, but are not intended to limit the present disclosure.
FIG. 1 shows the inhibition rate of PCSK9 protein expression in mouse serum on Day 7 and Day 14 by GalNAc-conjugated siRNA sequences including inc-G4, inc-G5, inc-G6, inc-G7, inc-G11, inc-G12, inc-G13, G18-inc, inc-L96, inc-NAG0052, inc-GalNAc 1b and inc-GalNAc 2.
FIG. 2 shows the inhibition rate of PCSK9 protein expression in mouse serum on Day 7 and Day 14 by GalNAc-conjugated siRNA sequences including inc-G1, inc-G2, inc-G3, inc-G8, inc-G9, inc-G10, inc-G25, G26-inc, inc-L96, inc-NAG0052, inc-GalNAc 1b and inc-GalNAc 2.
FIG. 3 shows the inhibition rate of PCSK9 gene expression in mouse liver on Day 14 by GalNAc-conjugated siRNA sequences including inc-G4, inc-G5, inc-G6, inc-G7, inc-G11, inc-G12, inc-G13, G18-inc, inc-L96, inc-NAG0052, inc-GalNAc 1b and inc-GalNAc 2.
FIG. 4 shows the inhibition rate of PCSK9 gene expression in mouse liver on Day 14 by GalNAc-conjugated siRNA sequences including inc-G1, inc-G2, inc-G3, inc-G8, inc-G9, inc-G10, inc-G25, G26-inc, inc-L96, inc-NAG0052, inc-GalNAc 1b and inc-GalNAc 2.
FIG. 5 shows the effects on LDL-C levels in mouse serum on Day 7 and Day 14 by GalNAc-conjugated siRNA sequences including inc-G4, inc-G5, inc-G6, inc-G7, inc-G11, inc-G12, inc-G13, G18-inc, inc-L96, inc-NAG0052, inc-GalNAc 1b and inc-GalNAc 2.
FIG. 6 shows the effects on LDL-C levels in mouse serum on Day 7 and Day 14 by GalNAc-conjugated siRNA sequences including inc-G1, inc-G2, inc-G3, inc-G8, inc-G9, inc-G10, inc-G25, G26-inc, inc-L96, inc-NAG0052, inc-GalNAc 1b and inc-GalNAc 2.
FIG. 7 shows the *in vivo* imaging of mice 8 hours after the administration of GalNAc-conjugated siRNA sequences including inc-G5, inc-G7, inc-L96 and inc-GalNAc 1b (a: inc-G5; b: inc-G7; c: inc-L96; d: inc-GalNAc 1b)
FIG. 8 shows in mouse liver the half-lives of GalNAc-conjugated siRNA sequences, including inc-G4, inc-G5, inc-G6, inc-G7, inc-G11, inc-G12, inc-G13, G18-inc, inc-L96, inc-NAG0052, inc-GalNAc 1b, and inc-GalNAc 2.
FIG. 9 shows in mouse liver the half-lives of GalNAc-conjugated siRNA sequences, including inc-G1, inc-G2, inc-G3, inc-G8, inc-G9, inc-G10, inc-G25, G26-inc, inc-L96, inc-NAG0052, inc-GalNAc 1b, and inc-GalNAc 2.

### DETAILED DESCRIPTION

In order to make the purpose, technical solution and advantages of the embodiments of the present disclosure clearer, the technical solution of the embodiments of the present disclosure will be clearly and completely described below. Obviously, the described embodiments are a part of the embodiments of the present disclosure, not all of the embodiments. Based on the described embodiments of the present disclosure, all other embodiments obtained by those of ordinary skill in the art without making inventive effort are within the protection scope of the present disclosure.

The present disclosure may be implemented in other specific forms without departing from the basic attributes of the present disclosure. It should be understood that, without conflict, any and all embodiments of the present disclosure may be combined with the technical features in any other embodiment or multiple other embodiments to obtain other embodiments. The present disclosure includes other embodiments obtained by such combination.

All publications and patents mentioned in the present disclosure are hereby incorporated into the present disclosure by reference in their entirety. If the purposes or terms used in any publications and patents incorporated by reference conflict with the purposes or terms used in the present disclosure, the purposes and terms of the present disclosure shall prevail.

The section headings used herein are for organizational purposes only and are not to be construed as limiting the subject matter described.

Unless otherwise specified, all technical and scientific terms used herein have the common meaning in the field to which the claimed subject matter belongs. If there are multiple definitions for a term, the definition herein shall prevail.

Except in the working examples or otherwise indicated, all numbers of quantitative properties such as dosage stated in the specification and claims should be understood to be modified by the term "about" in all cases. It should also be understood that any numerical range recited in the present disclosure is intended to include all subranges within the range and any combination of the respective endpoints of the range or subrange.

The words "include", "contain" or "comprise" and the like as used herein mean that the elements preceding the word include the elements listed after the word and their equivalents, without excluding unrecorded elements. The terms "contain" or "comprise (include)" used herein may be open, semi-closed and closed. In other words, the term also includes "substantially consisting of" or "consisting of".

The term "pharmaceutically acceptable" as used herein means that the compound or composition is chemically and/or toxicologically compatible with the other ingredients constituting the formulation and/or compatible with humans or mammals for the prevention or treatment of a disease or condition.

The term "pharmaceutically acceptable salt" refers to relatively non-toxic, inorganic or organic acid addition salts of compounds of the present disclosure. For example, see S. M. Berge et al., "Pharmaceutical Salts", J. Pharm. Sci. 1977, 66, 1-19. An inorganic acid is, for example, hydrochloric acid, hydrobromic acid, hydroiodic acid, sulfuric acid, phosphoric acid or nitric acid. An organic acid is, for example, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)-benzoic acid, camphoric acid, cinnamic acid, cyclopentanepropionic acid, digluconic acid, 3-hydroxy-2-naphthoic acid, nicotinic acid, pamoic acid, pectinic acid, 3-phenylpropionic acid, picric acid, pivalic acid, 2-hydroxyethanesulfonic acid, itaconic acid, aminosulfonic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, or sulfosalicylic acid. For example, HCl (or hydrochloric acid), HBr (or hydrobromic acid solution), methanesulfonic acid, sulfuric acid, tartaric acid or fumaric acid can be used to form a pharmaceutically acceptable salt with the compound represented by formula (I).

The term "alkyl" as used herein refers to a branched or linear saturated aliphatic monovalent hydrocarbon group having a specified number of carbon atoms. For example, "C₁₋₄alkyl" includes methyl, ethyl, propyl, isopropyl, n-butyl, isobutyl, sec-butyl and tert-butyl.

The term "alkoxy" refers to the formula -OR, wherein R is alkyl as defined herein. Non-limiting examples of alkoxy include methoxy, ethoxy, n-propoxy, 1-methylethoxy (isopropoxy), n-butoxy, isobutoxy, sec-butoxy, tert-butoxy, phenoxy, and benzoyloxy. In some cases, the alkoxy may be -OR, wherein R is unsubstituted C₁₋₄ alkyl. The alkoxy may be substituted or unsubstituted.

"Halogen" refers to fluorine (F), chlorine (Cl), bromine (Br) or iodine (I), preferably fluorine (F) or chlorine (Cl). In an embodiment, the halogen is fluorine.

The term "protecting group" as used herein refers to any atom or group of atoms introduced into a molecule to prevent existing groups in the molecule from undergoing undesired chemical reactions, which can be removed to leave an unprotected group.

A hydroxyl protecting group may be, for example, a protecting group commonly used to protect the hydroxyl group of the ribose structure in the synthesis of RNA or its derivatives, or may be referred to the protecting groups described in the literature of Protective Groups in Organic Synthesis, 3rd Edition, 1999, John Wiley & Sons, Inc. by Green et al., such as acetyl, phenoxyacetyl, pivaloyl, benzyl, 4-methoxybenzyl, benzoyl, triphenylmethyl, 4,4'-dimethoxytrityl (DMTr), monomethoxytrityl (MMTr), 9-phenyl-xanthen-9-yl, 9-(p-tolyl)-xanthen-9-yl, trimethylsilyl, tert-butyldimethylsilyl (TBDMS), cyanomethoxymethyl, 2-(cyanoethoxy)ethyl, or cyanoethoxymethyl, preferably 4,4'-dimethoxytrityl (DMTr).

The term "phosphorus-containing reactive group" as used herein refers to a phosphorus-containing group that can react with hydroxyl or amino contained in another molecule, particularly in another nucleotide unit or in another nucleotide analog, by a nucleophilic attack reaction. Typically, such a reaction produces an ester-type internucleoside bond connecting a nucleotide unit or a nucleotide analog unit to another nucleotide unit or a nucleotide analog unit. These phosphorus-containing reactive groups are known in the art and contain phosphorus atoms in P^{III} or P^{IV} valence state, and include, but are not limited to, phosphoramidites, H-phosphonates, phosphotriesters, and phosphorus-containing chiral auxiliary agents. Phosphorus-containing reactive groups are, for example: 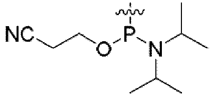 2-cyanoethoxy-N,N-diisopropylaminophosphine, 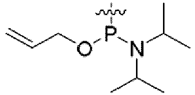 2-propyleneoxy--N,N-diisopropylaminophosphine, 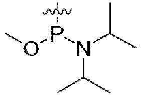 methoxy-N,N-diisopropylaminophosphine, or 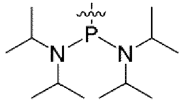 bis-diisopropylaminophosphine. In an embodiment, the phosphorus-containing reactive group is

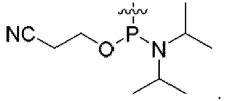

The controlled pore glass (CPG) and polystyrene (highly cross-linked polystyrene beads) described herein are solid supports for oligonucleotide synthesis, which are insoluble particles that bind to oligonucleotides during the synthesis process and are commercially available.

The term "nucleotide" as used herein includes naturally occurring nucleotides and chemically modified nucleotides. Chemically modified nucleotides are non-naturally occurring nucleotides, also referred to herein as "nucleotide analogs".

The "hydroxyl fully protected by acyl" described herein for "T" means that all hydroxyl groups in the galactose structure, except the hydroxyl group used for connecting to X₁, are protected with acyl, wherein the acyl is, for example, acetyl, chloroacetyl, trichloroacetyl, trifluoroacetyl, pivaloyl, isobutyryl or benzoyl.

The compound represented by formula (I) or a pharmaceutically acceptable salt thereof

The present disclosure provides a compound represented by formula (I) or a pharmaceutically acceptable salt thereof: 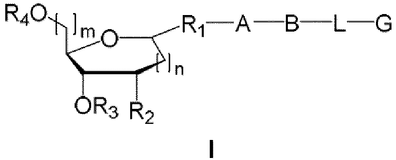 wherein,
R₁ is oxygen or sulfur, preferably oxygen;
R₂ is hydrogen, C₁₋₄ alkyl, C₁₋₄ alkoxy or halogen;
R₃ is hydrogen, a hydroxyl protecting group, a phosphorus-containing active reactive group, 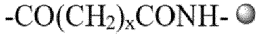 or -CO(CH₂)ₓCOOH, wherein x is an integer selected from 1 to 10, and 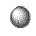 is controlled pore glass or polystyrene;
R₄ is hydrogen or a hydroxyl protecting group;
A is -(CH₂)ₐ-, -(CH₂CH₂O)_{b}-, -((CH₂)_{c}NHCO)_{d}-, or -((CH₂)_{c}CONH)_{d}-, wherein a is an integer selected from 1 to 15, preferably an integer selected from 3 to 13, b is an integer selected from 1 to 7, preferably an integer selected from 2 to 5, c is an integer selected from 1 to 7, preferably an integer selected from 2 to 6, and d is an integer selected from 1 to 5, preferably an integer selected from 1 to 3;
B is -(CH₂)ₑ-, wherein e is an integer selected from 0 to 7, preferably an integer selected from 1 to 5;
L is -CONH- or -NHCO-;
G is 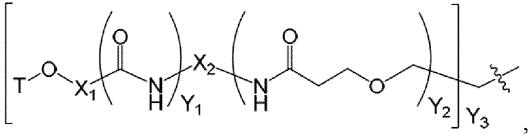
wherein,
T is N-acetyl-galactosamine with hydroxyl fully protected by acyl, galactose with hydroxyl fully protected by acyl, galactosamine with hydroxyl fully protected by acyl, N-formyl-galactosamine with hydroxyl fully protected by acyl, N-propionyl-galactosamine with hydroxyl fully protected by acyl, N-n-butyryl-galactosamine with hydroxyl fully protected by acyl, or N-isobutyryl-galactosamine with hydroxyl fully protected by acyl. For example, T is N-acetyl-galactosamine with hydroxyl fully protected by acyl, wherein the acyl is acetyl or benzoyl, preferably acetyl;
X₁ is -(CH₂)_{f}- or -(CH₂CH₂O)_{f}CH₂-, wherein f is an integer selected from 1 to 5;
X₂ is -(CH₂)_{g}-, wherein g is an integer selected from 1 to 6;
Y₁ is 0 or 1;
Y₂ is 0, 1 or 2;
Y₃ is 1, 2 or 3;
m is an integer selected from 0 to 4, preferably 0, 1 or 2; and
n is an integer selected from 0 to 4, preferably 0, 1 or 2.

In an embodiment, A is -(CH₂)₁₀-, -(CH₂)₇-, -(CH₂)₈-, -(CH₂)₉-, -(CH₂)₁₁-, -(CH₂)₁₂-, -(CH₂CH₂O)₃-, -(CH₂)₄NHCO-, or -(CH₂)₆NHCO-.

In an embodiment, B is -(CH₂)₀-, -CH₂-, -(CH₂)₂-, -(CH₂)₄-, or -(CH₂)₃-.

In an embodiment, R₁ is oxygen.

In an embodiment, R₁ has an α configuration or a β configuration.

In an embodiment, R₂ is hydrogen or -OCH₃.

In an embodiment, R₃ is a hydroxyl protecting group or a phosphorus-containing active reactive group. The hydroxyl protecting group may be, for example, acyl (e.g., acetyl, phenoxyacetyl, 4-isopropylphenoxyacetyl), silyl, trityl, monomethoxytrityl (MMTr), or 4,4'-dimethoxytrityl (DMTr). The phosphorus-containing reactive group may be, for example, 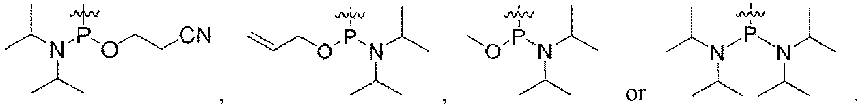 Preferably, R₃ is acetyl, 1,1,3,3-tetraisopropyldisiloxanylidene (TIPDS), t-butyldimethylsilyl, phenyldimethylsilyl, 4,4'-dimethoxytrityl or 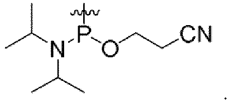 More preferably, R₃ is

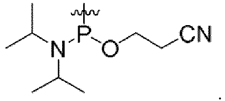

In another embodiment, R₃ is 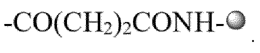 In yet another embodiment, R₃ is -CO(CH₂)₂COOH.

In an embodiment, R₄ is a hydroxy protecting group, preferably trityl, monomethoxytrityl or 4,4'-dimethoxytrityl, more preferably 4,4'-dimethoxytrityl.

In an embodiment, m is 1.

In an embodiment, n is 0.

In an embodiment, G is 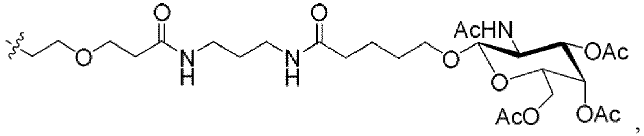 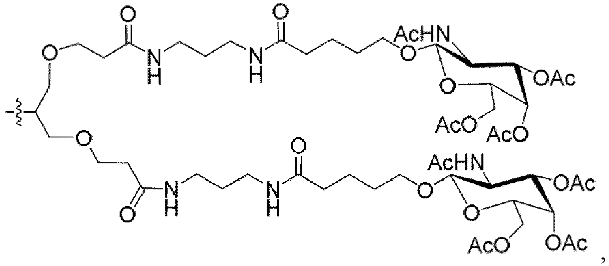 or

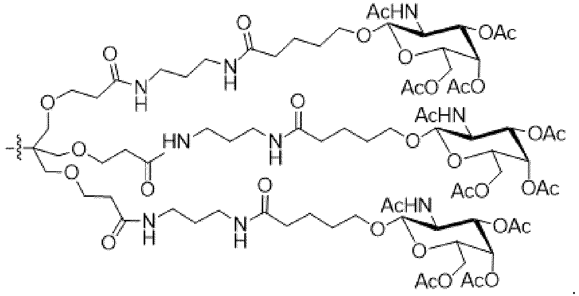

In an embodiment, the compound represented by formula (I) is YK-GAL-301, YK-GAL-302, YK-GAL-303, YK-GAL-304, YK-GAL-305, YK-GAL-306, YK-GAL-307, YK-GAL-308, YK-GAL-309, YK-GAL-310, YK-GAL-311, YK-GAL-312, or YK-GAL-313: 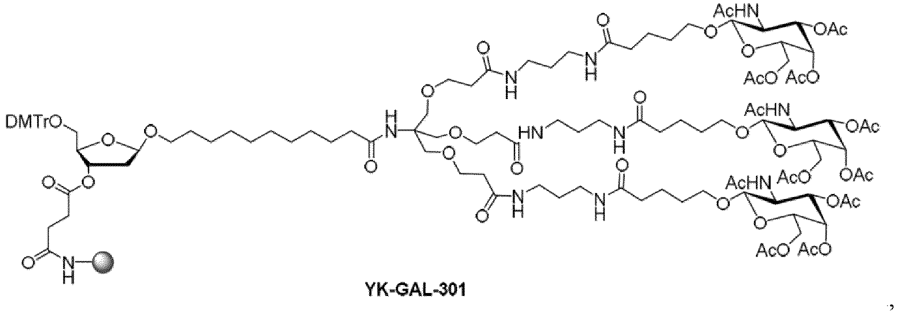 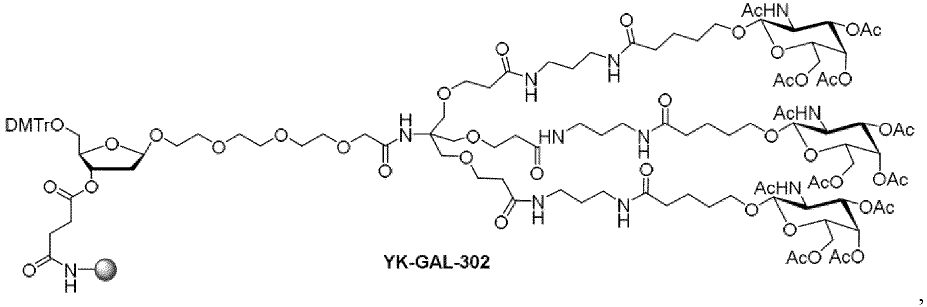 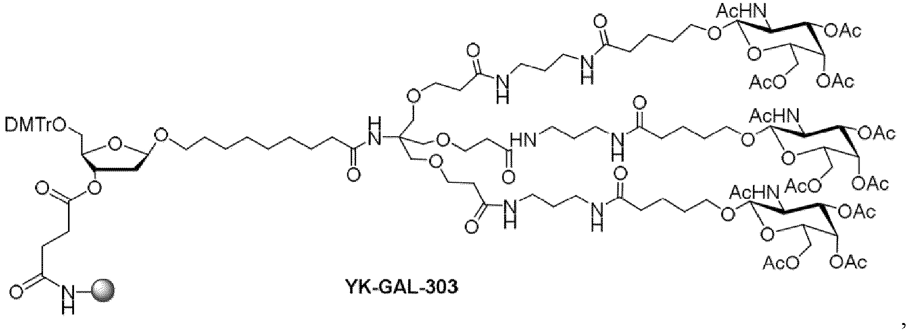 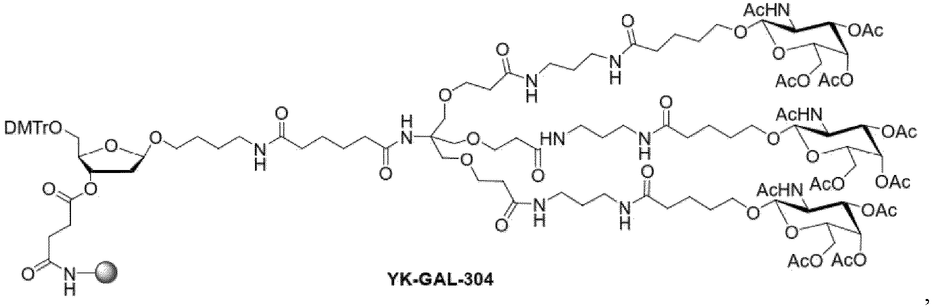 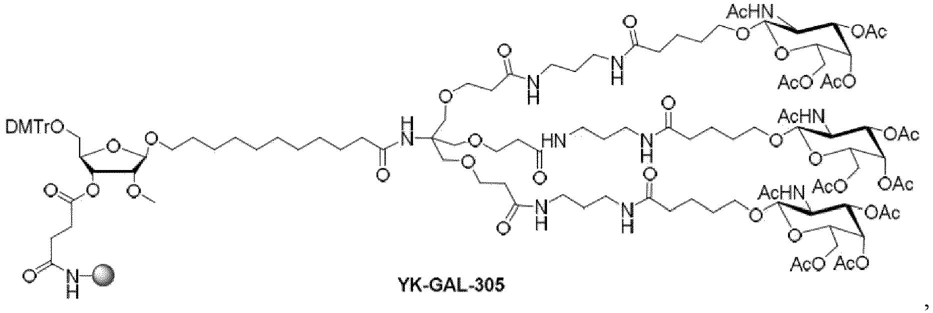 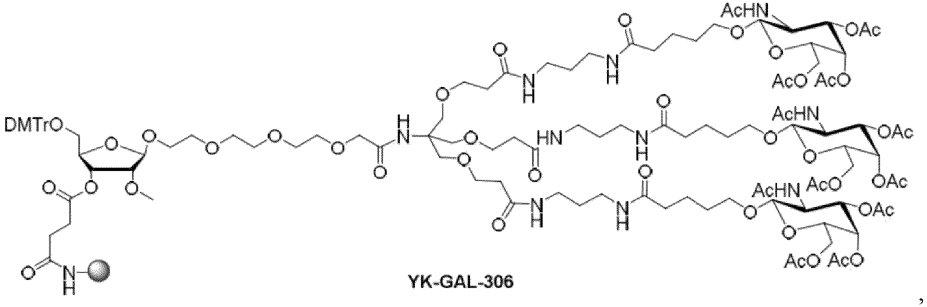 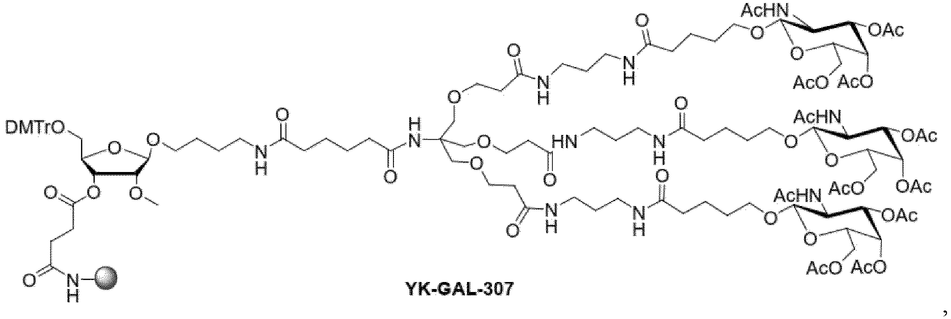 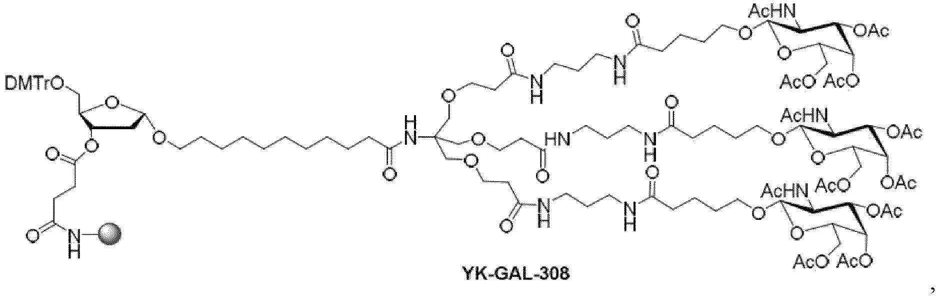 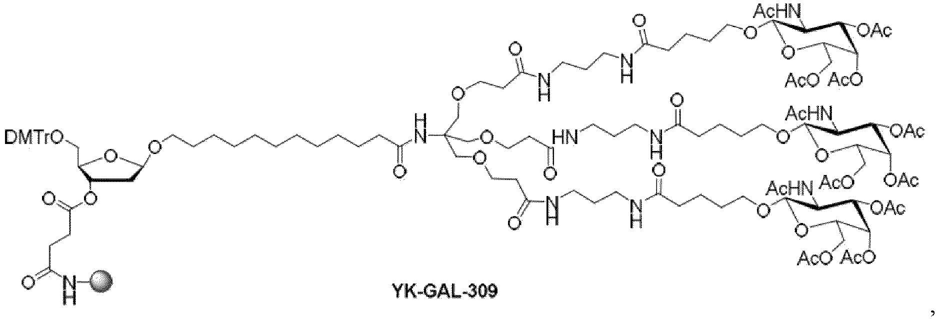 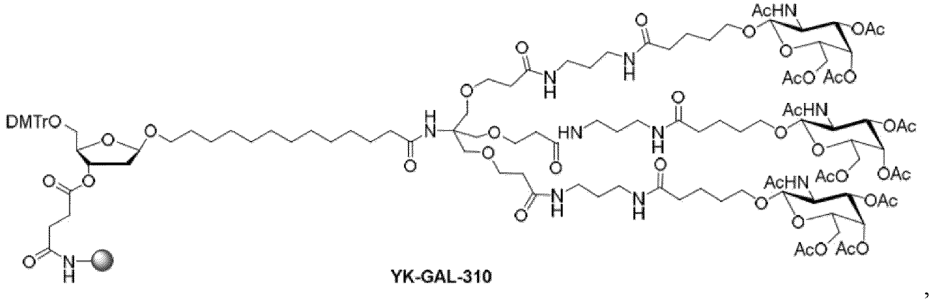 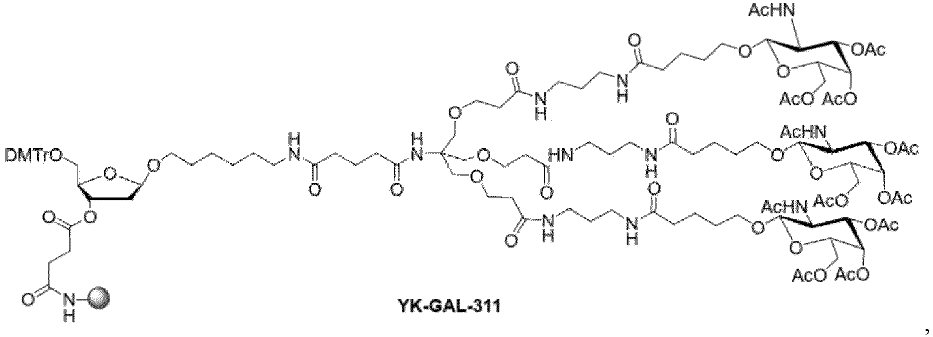 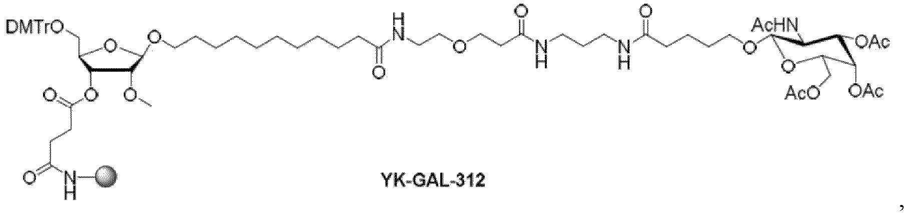 or 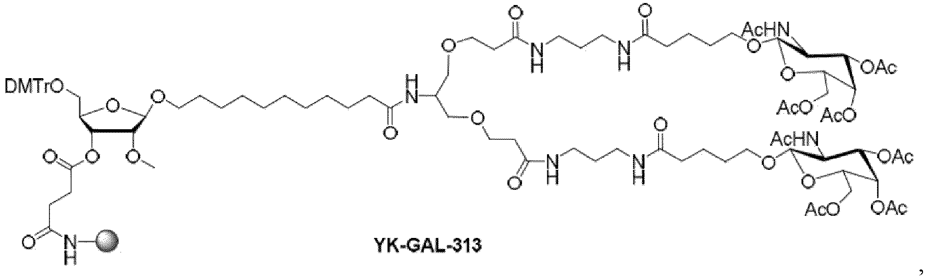 wherein 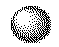 is controlled pore glass.

In another embodiment, the compound represented by formula (I) is YK-GAL-314, YK-GAL-315, YK-GAL-316, YK-GAL-317, YK-GAL-318, YK-GAL-319, YK-GAL-320, YK-GAL-321, YK-GAL-322, YK-GAL-323, YK-GAL-324, YK-GAL-325 or YK-GAL-326: 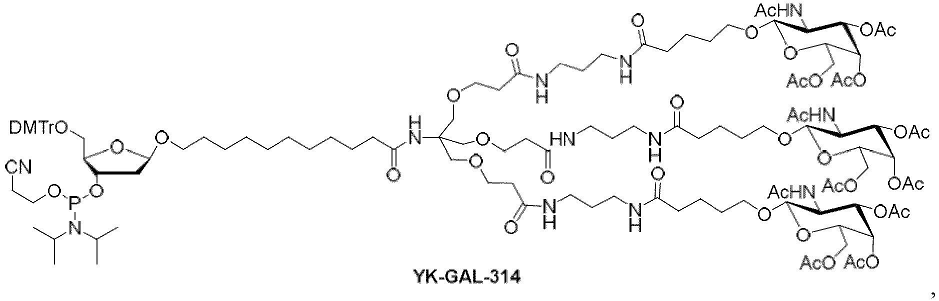 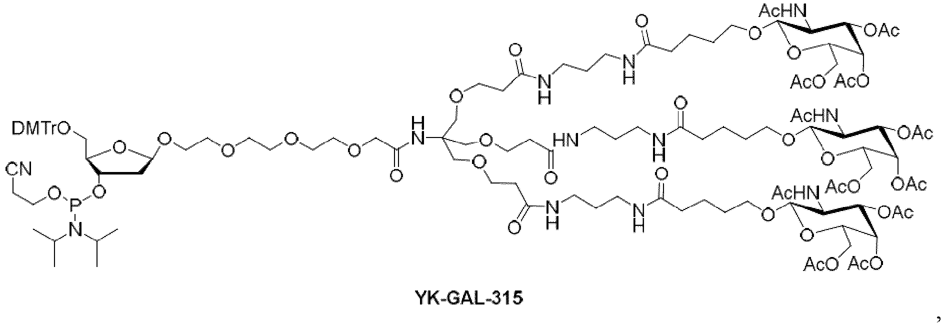 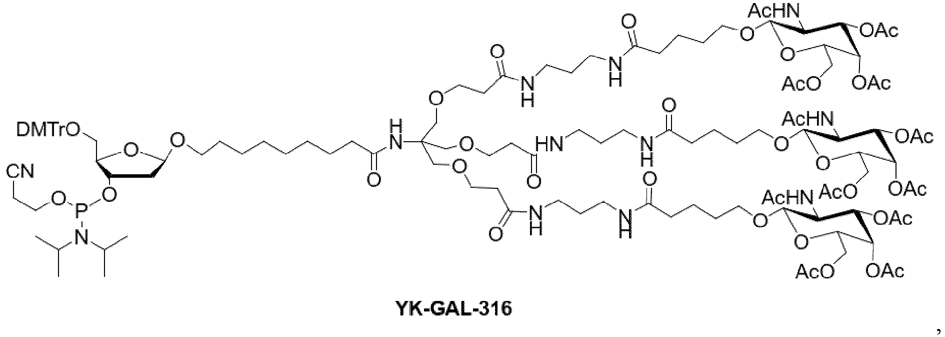 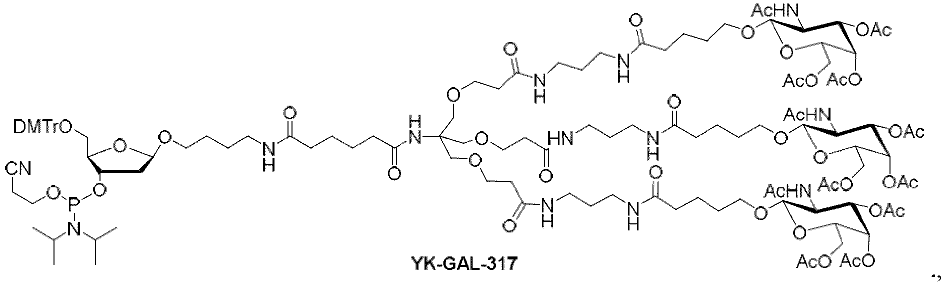 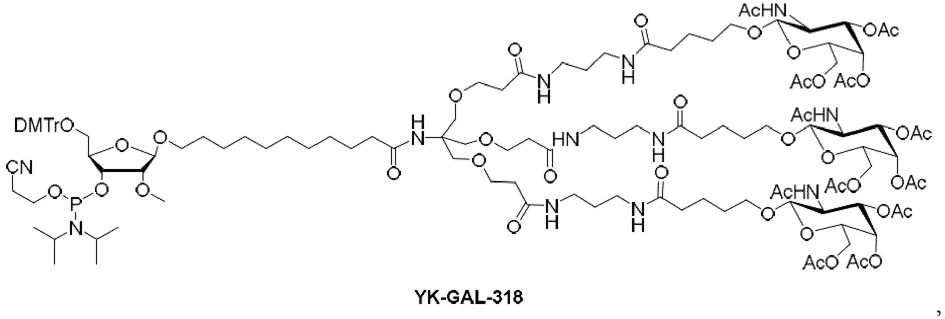 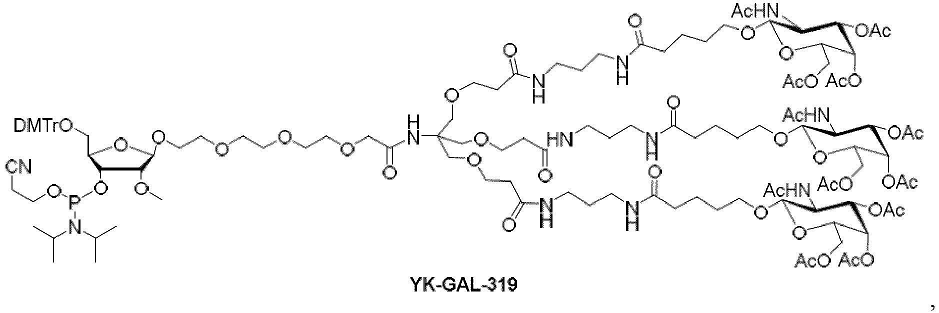 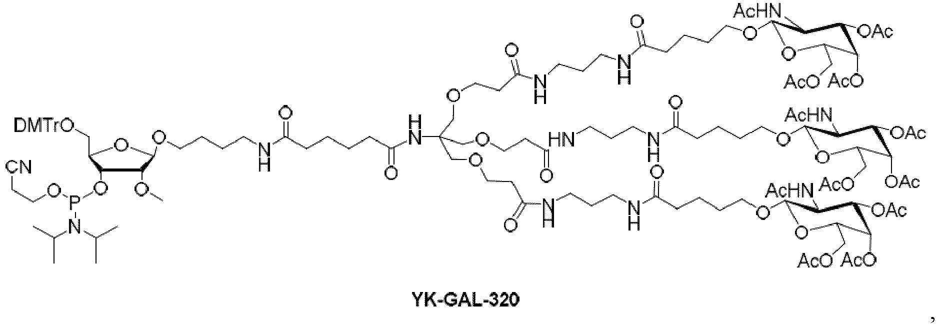 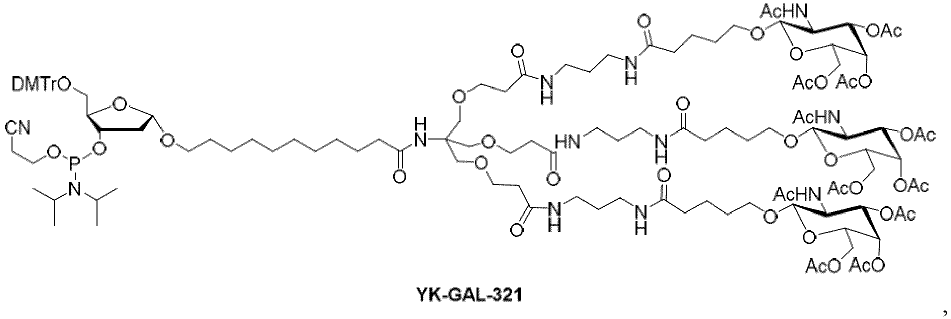 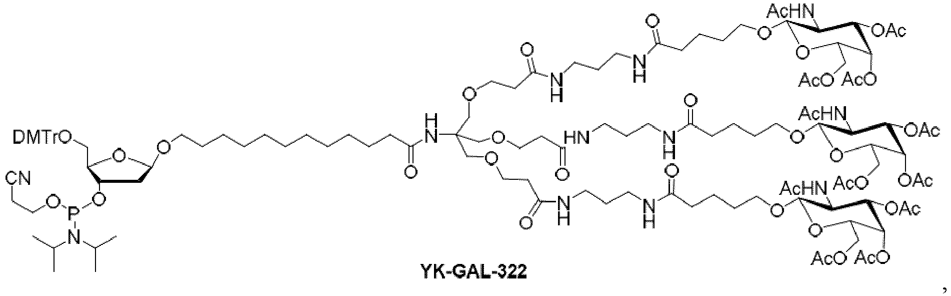 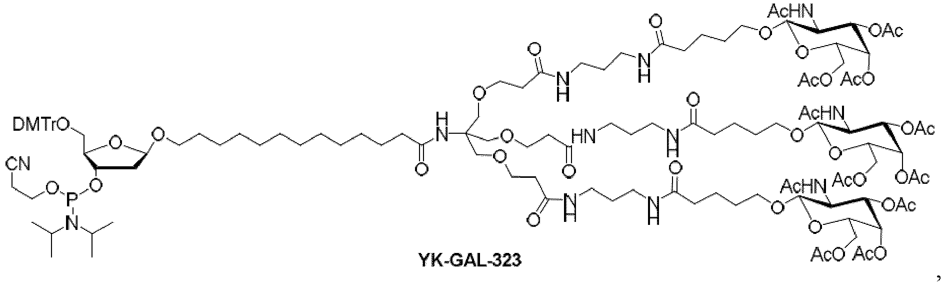 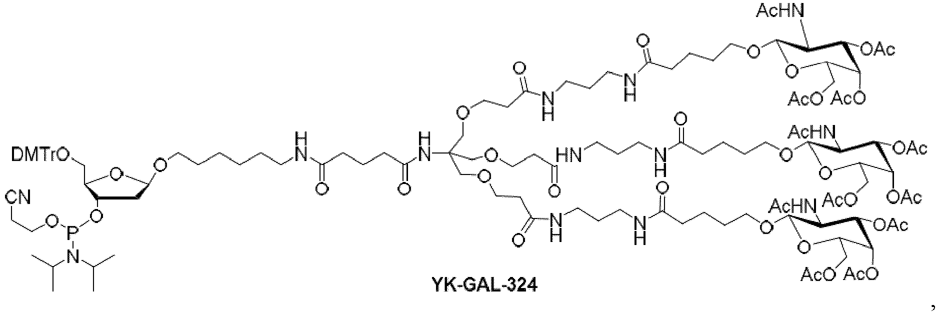 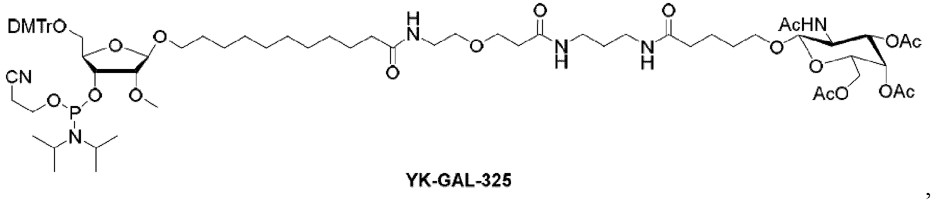 or

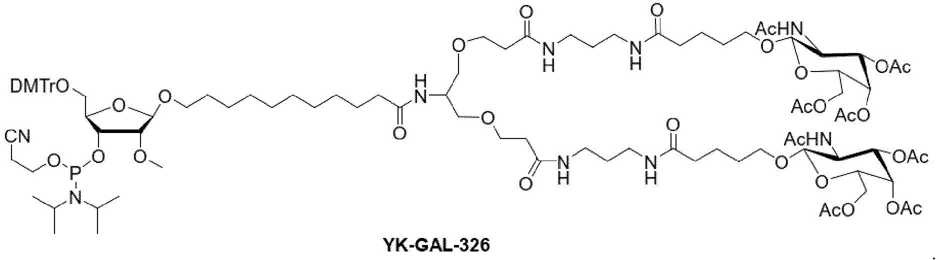

Optionally, the compound represented by formula (I) or the pharmaceutically acceptable salt thereof is capable of binding to asialoglycoprotein receptor (ASGPR).

### Conjugate

The present disclosure provides a conjugate comprising an oligonucleotide and a GalNAc moiety, wherein the conjugate has a structure of 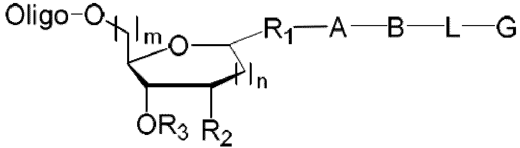 or 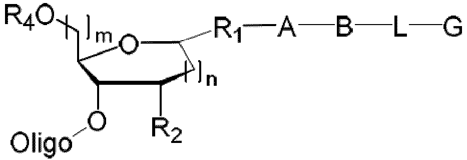 wherein, Oligo represents oligonucleotide, and R₁, R₂, R₃, R₄, A, B, L, G, m and n are as defined above.

The conjugate of the present disclosure can be prepared by solid phase synthesis reaction of the compound represented by formula (I) or a pharmaceutically acceptable salt thereof with an oligonucleotide. The conventional method for conjugating GalNAc with an oligonucleotide is a solid phase synthesis method. One method is to connect the GalNAc compound to a CPG (controlled pore glass) column and connect the GalNAc compound with the oligonucleotide by solid phase synthesis. Another method is to first prepare the GalNAc compound into a phosphoramidite monomer and then connect the GalNAc compound to any position of the oligonucleotide by solid phase synthesis.

The oligonucleotides in the present disclosure include single-stranded oligonucleotides (e.g., antisense nucleotides, referred to as ASOs) and double-stranded oligonucleotides (e.g., small interfering nucleotides, referred to as siRNAs). In a preferred embodiment, the oligonucleotide comprises 7 to 30 nucleotides. The conjugates prepared by such oligonucleotides will have more therapeutic value.

In an embodiment, the oligonucleotide is a small interfering RNA (siRNA), a DNA, a micro RNA (miRNA), a small activating RNA (saRNA), a small guide RNA (sgRNA), a transfer RNA (tRNA), an antisense oligonucleotide, or an aptamer, preferably an antisense oligonucleotide or a small interfering RNA.

The oligonucleotides in the present disclosure include natural oligonucleotides and chemically modified oligonucleotides. Chemical modifications herein include nucleoside modifications (including sugar moiety modifications and nucleobase modifications) and internucleoside linkage modifications. Chemical modifications of oligonucleotides do not include situations where there are differences only in the nucleobase sequence. Natural herein refers to situations corresponding to naturally occurring RNA or DNA.

Natural oligonucleotides have difficulty entering cells and are easily degraded by intracellular nucleases, resulting in poor effects. Chemical modification of oligonucleotides can improve their properties and increase their bioavailability. Among the many modified oligonucleotides, thio-oligonucleotides are the most representative, in which one of the thio-modification methods is that a non-bridging oxygen atom in the phosphodiester bond is replaced by a sulfur atom, for example,

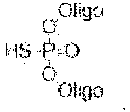

Thio-oligonucleotides can be commercially available or prepared by conventional solid phase synthesis methods. Xanthane Hydride can be used as a sulfurizing agent. For example, reference can be made to CN1479745A and CN113150041A for synthesizing thio-oligonucleotides.

Optionally, the hydroxyl protecting group acetyl in the N-acetyl-galactosamine of G in the conjugate is removed, for example, by hydrolysis in an alkaline solution to obtain hydroxyl.

In an embodiment, the GalNAc moiety of the conjugate has the structure of 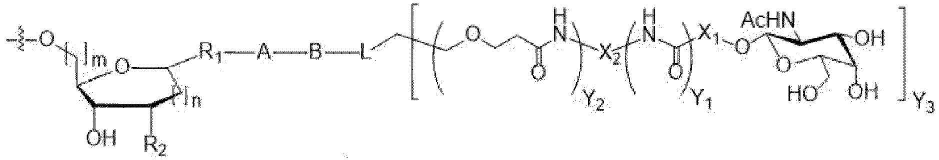 wherein R₁, R₂, A, B, L, X₁, X₂, Y₁, Y₂, Y₃, m and n are as defined above.

For example, the oligonucleotide and the GalNAc moiety are connected via a bond or a cleavable linker. The bond herein may include, but is not limited to, a phosphate bond and a phosphorothioate bond.

The cleavable linker used in the present disclosure refers to a linker that is cleaved under intracellular metabolism after internalization, for example, cleaved by hydrolysis, reduction or enzymatic reaction. Suitable linkers include, but are not limited to, acid-labile linkers, hydrolysis-labile linkers, enzymatically cleavable linkers and reduction-labile linkers. Acid-labile linkers can refer to the acid-labile linkers in ADC drugs (Mylotarg, Besponsas, Trodelvys).

Optionally, the oligonucleotide include a non-thio oligonucleotide and a thio oligonucleotide. In an embodiment, the non-thio oligonucleotide and the GalNAc moiety are linked by a phosphoester bond. In another embodiment, the thio oligonucleotide and the GalNAc moiety are linked by a Thiophosphoester bond.

Optionally, the oligonucleotide is a small interfering RNA (siRNA), a DNA, a micro RNA (miRNA), a small activating RNA (saRNA), a small guide RNA (sgRNA), a transfer RNA (tRNA), an antisense oligonucleotide (ASO), and an aptamer, preferably an antisense oligonucleotide (ASO) or a small interfering RNA (siRNA). In an embodiment, each nucleotide in the antisense oligonucleotide (ASO) or small interfering RNA (siRNA) is independently modified or unmodified.

Optionally, the oligonucleotide modulates expression of a target gene.

### Pharmaceutical composition

In addition to the conjugate, the composition provided herein may comprise any substance that can be used in a pharmaceutical composition. For example, the composition may comprise one or more pharmaceutically acceptable excipients or auxiliary ingredients, such as but not limited to one or more solvents, dispersion media, diluents, dispersing aids, suspension aids, granulation aids, disintegrants, fillers, glidants, liquid vehicles, adhesives, surfactants, isotonic agents, thickeners or emulsifiers, buffers, lubricants, oils, preservatives, flavoring agents, colorants, etc. Excipients are, for example, starch, lactose, or dextrin. Pharmaceutically acceptable excipients are well known in the art (see, for example, Remington's The Science and Practice of Pharmacy, 21st edition, A. R. Gennaro; Lippincott, Williams & Wilkins, Baltimore, MD, 2006).

Pharmaceutically acceptable diluents include phosphate buffered saline (PBS), for example, sterile phosphate buffered saline. In some embodiments, the conjugate is used in a pharmaceutically acceptable diluent at a concentration of 50-500 µM solution. WO 2007/031091 provides suitable pharmaceutically acceptable diluents, carriers and excipients, and also provides suitable dosages, formulations, routes of administration, compositions, dosage forms, combinations with other therapeutic agents, and prodrug preparations (the document is incorporated by reference).

The oligonucleotide conjugates of the present disclosure can be mixed with pharmaceutically acceptable active substances or inert substances to prepare pharmaceutical compositions or preparations, and can be sterilized by conventional sterilization techniques, or can be subjected to sterile filtration.

### Kit

The present disclosure further provides a kit comprising the conjugate as defined above. In some embodiments, the kit provided herein comprises a container comprising the conjugate as defined above. In some embodiments, the kit provided herein further comprises a pharmaceutically acceptable adjuvant, such as a stabilizer or a preservative. In some embodiments, the kit further comprises instructions for mixing the conjugate with a pharmaceutically acceptable adjuvant or other ingredients (if present).

### Examples

The present disclosure is further described below in conjunction with the examples. However, the present disclosure is not limited to the following examples. The implementation conditions used in the examples can be further adjusted according to the different requirements of specific use, and the implementation conditions not specified are conventional conditions in the industry. In the specific examples of the present disclosure, the raw materials used can be obtained commercially. Unless otherwise specified, all temperatures are given in degrees Celsius. The technical features involved in each embodiment of the present disclosure can be combined with each other as long as they do not conflict with each other.

### Example 1: Synthesis of GalNAc-CPG Compounds

The following abbreviations represent the following reagents: DCM: dichloromethane; PE: petroleum ether; EA: ethyl acetate; THF: tetrahydrofuran; DMF: N,N-dimethylformamide; ACN: acetonitrile; BF₃•Et₂O: boron trifluoride etherate; LiOH•H₂O: lithium hydroxide monohydrate; DMTrCl: 4,4'-dimethoxytriphenylmethane chloride; TEAB: aqueous triethylamine ammonium bicarbonate solution; HBTU: O-benzotriazole-tetramethyluronium hexafluorophosphate; DIPEA: N,N-diisopropylethylamine; DMAP: 4-dimethylaminopyridine; TMSOTf: trimethylsilyl trifluoromethanesulfonate; TIPDSCl₂: 1,3-dichloro-1,1,3,3-tetraisopropyldisiloxane; TEA•3HF: triethylamine trihydrofluoride; Proton sponge: 1,8-bis(dimethylaminonaphthalene).

### 1. Synthesis of YK-GAL-301

The synthetic route is as follows:

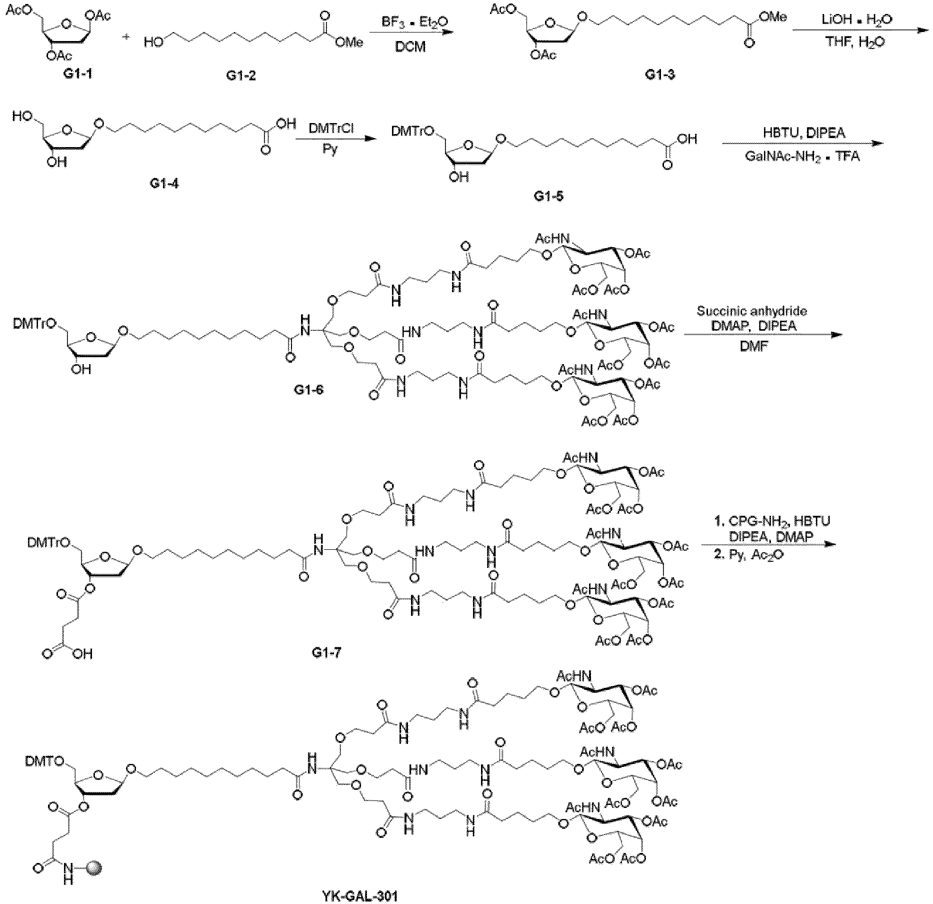

### Step 1: Synthesis of G1-3

A mixture of the dried compound **G1-1** (781 mg, 3.00 mmol), the dried compound **G1-2** (0.50 g, 2.31 mmol) and 3A molecular sieve (2.0 g) was added with dichloromethane (20 mL), cooled to 0°C, added with boron trifluoride etherate (656 mg, 4.62 mmol), and stirred for 2 h. After quenched by adding aqueous solution of saturated sodium bicarbonate (20 mL), the reaction solution was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a yellow oil **G1-3** (1.17 g, 2.81 mmol, α- and β- mixed isomers, 93.7%). Then, high-pressure preparative separation was performed to obtain **G8-1** (533.2 mg, 1.28 mmol, α configuration, 42.7%) and G1-3 (625.6 mg, 1.5 mmol, β configuration, 50.1%). ¹HNMR (400 MHz, CDCl₃) *δ* ppm 5.23 - 5.13 (m, 2H), 4.27 (dd, *J =* 11.0, 5.6 Hz, 1H), 4.23 - 4.15 (m, 1H), 4.09 (dd, *J* = 11.0, 6.4 Hz, 1H), 3.69 - 3.63 (m, 4H), 3.37 - 3.28 (m, 1H), 2.39 - 2.33 (m, 1H), 2.29 (t, *J* = 7.6 Hz, 2H), 2.19 - 1.94 (m, 8H), 1.65 - 1.56 (m, 2H), 1.53 - 1.48 (m, 2H), 1.32 - 1.21 (m, 11H). MS (ESI) m/z [M + Na]⁺ = 439.1.

### Step 2: Synthesis of G1-4

**G1-3** (0.12 g, 288 µmol) was dissolved in tetrahydrofuran (3.0 mL). added with 1.5 mL of aqueous solution of lithium hydroxide monohydrate (38.7 mg, 921 µmol), and stirred at 15°C for 12 h. The reaction solution was concentrated under reduced pressure to remove the solvent to obtain a yellow solid. The crude product **G1-4** (92 mg) was used directly in the next step. MS (ESI) m/z [M + Na]⁺ = 341.2.

### Step 3: Synthesis of G1-5

The crude product **G1-4** (92 mg, 288 µmol) and anhydrous pyridine (20 mL × 3) were co-rotary-evaporated to remove water. The dried **G1-4** and 4,4'-dimethoxytriphenylmethane chloride (195 mg, 576 µmol) were dissolved in pyridine (3.0 mL) and stirred at 15°C for 28 h under nitrogen protection. Methanol (10 mL) was added to quench the reaction, and the solvent was removed by concentration under reduced pressure to obtain a crude product, which was purified by preparative chromatography (H₂O (10 mM NH₄HCO₃)-ACN) to obtain a white solid **G1-5** (109.5 mg, 176.5 µmol) with a yield of 61.3%. ¹HNMR (400 MHz, CDCl₃) *δ* ppm 7.48 - 7.41 (m, 2H), 7.37 - 7.27 (m, 6H), 7.21 (dd, *J* = 8.2, 6.2 Hz, 1H), 6.88 - 6.78 (m, 4H), 5.15 (dd, *J* = 5.3, 2.2 Hz, 1H), 4.43 - 4.39 (m, 1H), 3.98 - 3.91 (m, 1H), 3.79 (s, 6H), 3.62 - 3.56 (m, 1H), 3.35 - 3.23 (m, 2H), 3.15 (dd, *J* = 9.4, 6.8 Hz, 1H), 2.33 (t, *J* = 7.4 Hz, 5H), 2.21 - 2.15 (m, 2H), 2.07 - 2.00 (m, 1H), 1.66 - 1.59 (m, 2H), 1.44 - 1.41 (m, 2H), 1.29 - 1.22 (m, 10H). MS (ESI) m/z [M + Na]⁺ = 643.4.

### Step 4: Synthesis of G1-6

**G1-5** (40 mg, 64.4 µmol), diisopropylethylamine (16.7 mg, 129 µmol) and O-benzotriazole-tetramethyluronium hexafluorophosphate (36.7 mg, 96.6 µmol) were dissolved in N,N-dimethylformamide (0.5 mL), added with GalNAc-NH₂.TFA (123 mg, 64.4 µmol, TFA) and stirred at 15°C for 1 h under nitrogen protection. The reaction solution was used directly in the next step. MS (ESI) m/z [M - 2H]²⁻ = 1197.4.

### Step 5: Synthesis of G1-7

**G1-7** (154 mg, 64.2 µmol), 4-dimethylaminopyridine (15.7 mg, 128 µmol), diisopropylethylamine (16.6 mg, 128 µmol), and succinic anhydride (64.4 mg, 642 µmol) were dissolved in N,N-dimethylformamide (2.0 mL) and stirred at 30°C for 48 h under nitrogen protection. Purification was performed by preparative chromatography (H₂O (10 mM TEAB)-ACN) to obtain a white solid **G1-7** (59 mg, 26.7 µmol) with a yield of 41.5%. ¹HNMR (400 MHz, CDCl₃) *δ* ppm 7.44 (d, *J=* 7.6 Hz, 2H), 7.32 (d, *J* = 8.5 Hz, 3H), 7.25 - 7.14 (m, 5H), 6.97 (t, *J* = 6.3 Hz, 3H), 6.78 (dd, *J =* 11.8, 8.6 Hz, 6H), 6.48 (s, 1H), 5.33 (d, *J* = 3.2 Hz, 3H), 5.19 - 5.15 (m, 5H), 4.59 (d, *J* = 8.3 Hz, 3H), 4.18 - 4.03 (m, 10H), 3.95 - 3.88 (m, 6H), 3.77 (s, 6H), 3.68 - 3.61 (m, 13H), 3.52 - 3.46 (m, 3H), 3.39 - 3.09 (m, 15H), 2.61 - 2.48 (m, 4H), 2.42 (t, *J* = 5.8 Hz, 5H), 2.31 - 2.09 (m, 20H), 2.03 - 1.99 (m, 16H), 1.98 - 1.93 (m, 21H), 1.72 - 1.54 (m, 16H), 1.41 (s, 2H), 1.24 - 1.21 (m, 10H). MS (ESI) m/z [M - 2H]²⁻ = 1247.4.

### Step 6: Synthesis of YK-GAL-301

**G1-7** (59 mg, 22.7 µmol), 4-dimethylaminopyridine (2.77 mg, 22.7 µmol), diisopropylethylamine (23.5 mg, 182 µmol), and O-benzotriazole-tetramethyluronium hexafluorophosphate (43.1 mg, 113 µmol) were dissolved in N,N-dimethylformamide (4.0 mL), added with CPG-NH₂ (425 mg), and stirred at 40°C for 12 h. The reaction solution was filtered, and the filtrate was washed with methanol followed by dichloromethane and dried in vacuum. The filtrate was then added to 4 mL of acetic anhydride/pyridine (1:5) solution and stirred at 40°C for 0.5 h. The mixture was filtered and washed with dichloromethane followed by methanol, and the filtrate was dried in vacuum for 12 h to obtain a white solid compound **YK-GAL-301** (338 mg, loading 27.0 µmol/g).

### 2. Synthesis of YK-GAL-302

The synthetic route is as follows:

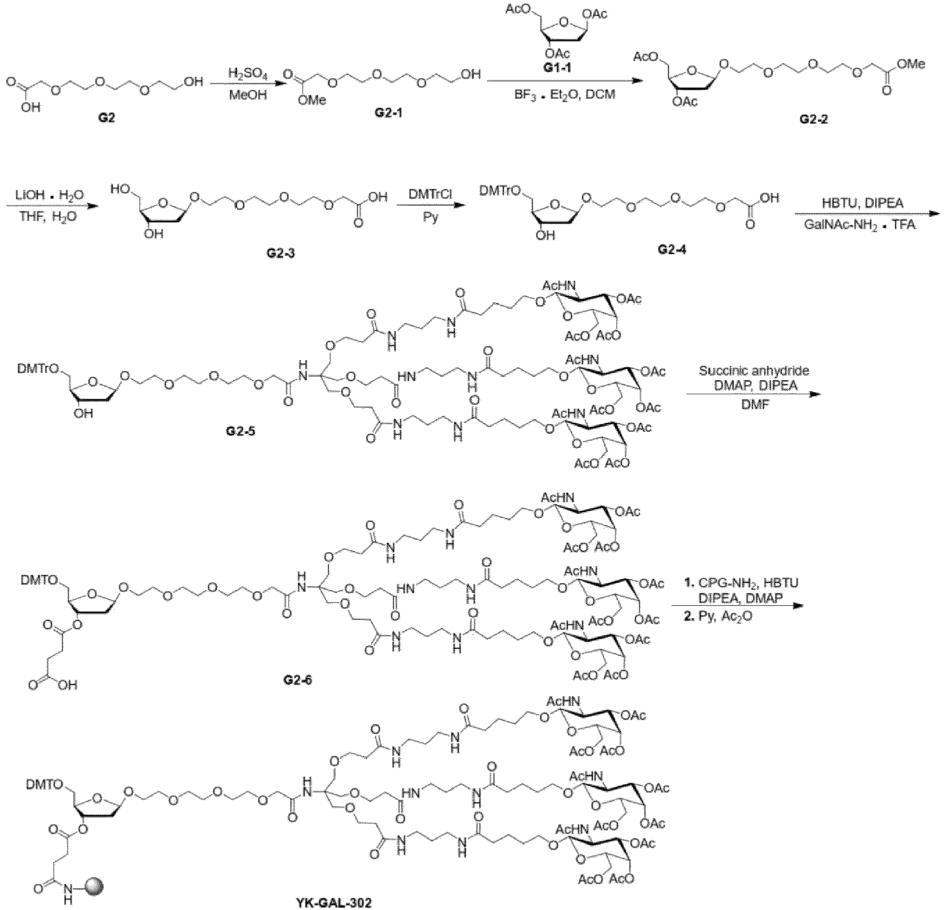

### Step 1: Synthesis of G2-1

Compound **G2** (800 mg, 3.84 mmol) was dissolved in methanol (16 mL), added with sulfuric acid (1.18 g, 11.7 mmol) and stirred at 70°C for 16 h. 10 mL of sodium sulfate was added to quench the reaction, and the solvent was removed by concentration under reduced pressure to obtain a crude product. Purification was performed by column chromatography (DCM/MeOH) to obtain a colorless oil **G2-1** (470 mg, 2.11 mmol) with a yield of 55.0%. ¹HNMR (400 MHz, CDCl₃) δ ppm 4.17 (s, 2H), 3.78 - 3.65 (m, 13H), 3.61 (dd, *J* = 5.4, 3.7 Hz, 2H), 2.30 (s, 1H)

### Step 2: Synthesis of G2-2

A mixture of compound **G1-1** (660 mg, 2.54 mmol), compound **G2-1** (470 mg, 2.11 mmol) and 4A molecular sieve (900 mg) was added with dichloromethane (9.0 mL), cooled to 0°C, added with boron trifluoride etherate (600 mg, 4.23 mmol), stirred for 2 h, and then stirred at 15°C for 3 h. After quenched by adding aqueous solution of saturated sodium bicarbonate (30 mL), the reaction solution was extracted with dichloromethane (30 mL × 3). The organic phase was dried over anhydrous sodium sulfate and concentrated under reduced pressure to remove the solvent to obtain a crude product, which was purified by column chromatography (PE/EA) to obtain a yellow oil **G2-2** (443.3 mg, 1.05 mmol) with a yield of 49.8%. ¹HNMR (400 MHz, CDCl₃) *δ* ppm 5.24 (d, *J* = 5.4 Hz, 1H), 5.04 - 5.00 (m, 1H), 4.36 - 4.23 (m, 2H), 4.19 - 4.14 (m, 3H), 3.87 - 3.80 (m, 1H), 3.75 (s, 3H), 3.73 - 3.68 (m, 5H), 3.67 - 3.64 (m, 6H), 2.45 - 2.38 (m, 1H), 2.11 - 2.01 (m, 7H). MS (ESI) m/z [M + Na]⁺ = 445.0.

### Step 3: Synthesis of G2-3

**G2-2** (320 mg, 757 µmol) was dissolved in tetrahydrofuran (2.5 mL), added with 2.5 mL of aqueous solution of lithium hydroxide monohydrate (95.3 mg, 2.27 mmol) and stirred at 15°C for 16 h. The reaction solution was concentrated under reduced pressure to remove the solvent to obtain a white solid. The crude product **G2-3** (245 mg) was used directly in the next step. MS (ESI) m/z [M + Na]⁺ = 346.9.

### Step 4: Synthesis of G2-4

The dried crude product **G2-3** (245 mg, 755 µmol) and 4,4'-dimethoxytriphenylmethane chloride (511 mg, 1.51 mmol) were dissolved in pyridine (3.0 mL) and stirred at 15°C for 6 h under nitrogen protection. Methanol (2 mL) was added to quench the reaction, and the solvent was removed by concentration under reduced pressure to obtain a crude product, which was purified by preparative chromatography (H₂O (10 mM NH₄HCO₃)-ACN) to obtain a white solid **G2-4** (309.1 mg, 494 µmol, 65.2% yield). ¹HNMR (400 MHz, CDCl₃) *δ* ppm 7.47 (d, *J* = 7.6 Hz, 1H), 7.39 - 7.33 (m, 4H), 7.20 - 7.16 (m, 1H), 6.82 - 6.80 (m, 4H), 5.13 (d, *J* = 4.8 Hz, 1H), 4.43 - 4.38 (m, 2H), 4.05 - 4.38 (m, 5H), 3.79 - 3.78(m, 8H), 3.67 - 3.52 (m, 10H), 3.46 - 3.13 (m, 4H), 2.13 - 2.16 (m, 1H), 2.03 - 1.98 (m, 1H). MS (ESI) m/z [M - H]⁻ = 625.4.

### Step 5: Synthesis of G2-5

**G2-4** (80.0 mg, 127 µmol), diisopropylethylamine (33.0 mg, 255 µmol, 44.4 µL) and O-benzotriazole-tetramethyluronium hexafluorophosphate (72.6 mg, 191 µmol) were dissolved in N,N-dimethylformamide (2.0 mL), added with GalNAc-NH₂.TFA (206 mg, 108 µmol, TFA) and stirred at 15°C for 4 h under nitrogen protection. The reaction solution was used directly in the next step. MS (ESI) m/z [M - 2H]²⁻ = 1200.3.

### Step 6: Synthesis of G2-6

**G2-5** (305 mg, 126 µmol), 4-dimethylaminopyridine (62.0 mg, 507 µmol), diisopropylethylamine (49.2 mg, 380 µmol), and succinic anhydride (114 mg, 1.14 mmol) were dissolved in N,N-dimethylformamide (3.0 mL) and stirred at 30°C for 48 h under nitrogen protection. Purification was performed by preparative chromatography (H₂O (10 mM TEAB)-ACN) to obtain a white solid **G2-6** (130 mg, 51.9 µmol) with a yield of 40.9%. ¹HNMR (400 MHz, CDCl₃) *δ* ppm 7.45 (s, 1H), 7.43 (s, 1H), 7.32 (d, *J =* 8.5 Hz, 4H), 7.25 - 7.16 (m, 4H), 6.95 (dd, *J =* 36.7, 13.2 Hz, 4H), 6.85 - 6.72 (m, 6H), 5.35 (d, *J* = 3.4 Hz, 2H), 5.22 (td, *J* = 11.0, 10.3, 4.0 Hz, 4H), 4.63 (dd, *J =* 8.3, 2.6 Hz, 2H), 4.12 (dtd, *J =* 27.6, 11.1, 4.6 Hz, 9H), 3.96 - 3.85 (m, 7H), 3.78 (d, *J =* 2.6 Hz, 5H), 3.71 - 3.62 (m, 14H), 3.54 (dd, *J =* 25.9, 6.2 Hz, 7H), 3.33 - 3.16 (m, 12H), 2.83 (s, 10H), 2.67 - 2.53 (m, 4H), 2.42 (t, *J* = 5.9 Hz, 4H), 2.28 - 2.14 (m, 14H), 2.11 - 1.93 (m, 26H), 1.67 (ddd, *J* = 39.3, 13.2, 7.3 Hz, 16H), 1.23 (d, *J =* 7.4 Hz, 15H). MS (ESI) m/z [M - 2H]²⁻ = 1250.3.

### Step 7: Synthesis of YK-GAL-302

**G2-6** (130 mg, 51.9 µmol), 4-dimethylaminopyridine (6.35 mg, 51.9 µmol), diisopropylethylamine (53.7 mg, 415 µmol) and O-benzotriazole-tetramethyluronium hexafluorophosphate (98.7 mg, 259.5 µmol) were dissolved in N,N-dimethylformamide (6.0 mL), then added with CPG-NH₂ (830 mg, 1.41 mmol) and stirred at 30°C for 16 h. The reaction solution was filtered, and the filtrate was washed with methanol followed by dichloromethane and dried in vacuum. The filtrate was then added to 12 mL of acetic anhydride/pyridine (1:5) solution and stirred at 40°C for 0.5 h. The mixture was filtered, washed with dichloromethane followed by methanol, and the filtrate was dried in vacuum for 12 h to obtain a white solid compound **YK-GAL-302** (840 mg, loading 34.9 µmol/g).

### 3. Synthesis of YK-GAL-303

The synthetic route is as follows:

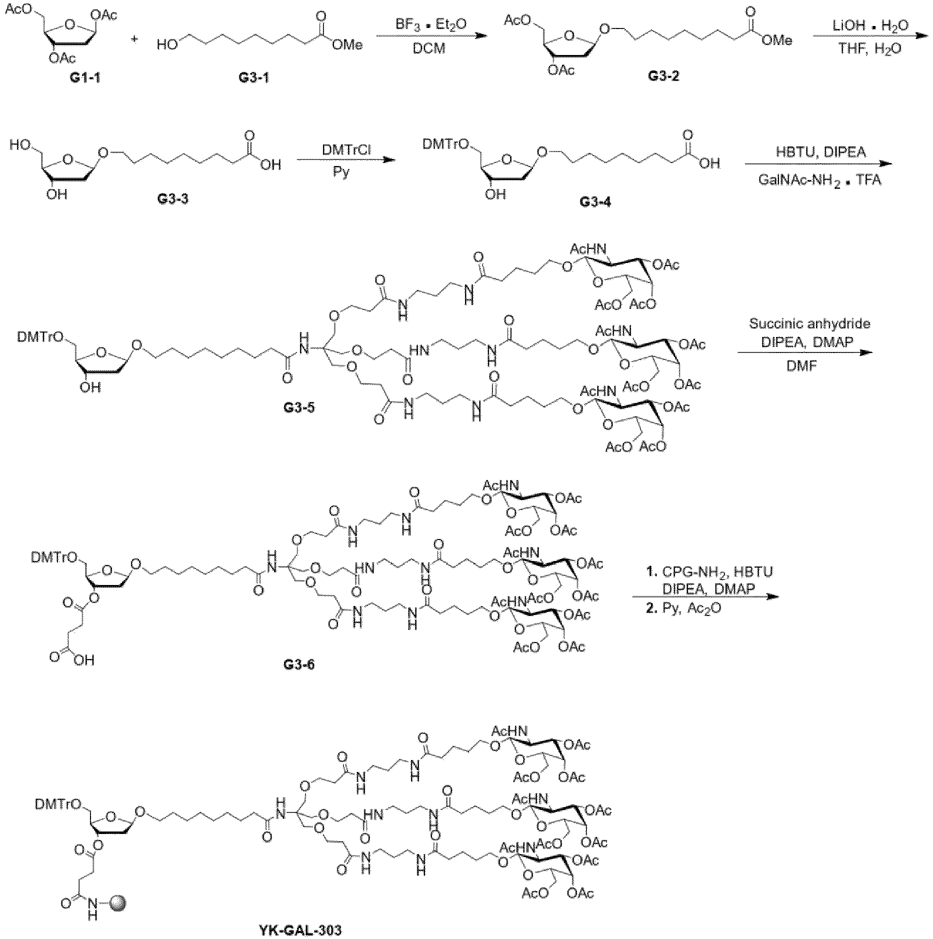

### Step 1: Synthesis of G3-2

A mixture of the dried compound **G1-1** (1.19 g, 4.58 mmol), the dried compound G3-1 (663.2 mg, 3.52 mmol) and 3A molecular sieve (2.0 g) was added with dichloromethane (26 mL), cooled to 0°C, added with boron trifluoride etherate (999.95 mg, 7.05 mmol), and then stirred at 25°C for 2 h. After quenched by adding aqueous solution of saturated sodium bicarbonate (30 mL), the reaction solution was extracted with dichloromethane (30 mL × 3). The organic phase was dried over anhydrous sodium sulfate and concentrated under reduced pressure to remove the solvent to obtain a crude product, which was purified by column chromatography (PE/EA) to obtain a yellow oil **G3-2** (612.2 mg, 1.58 mmol) with a yield of 44.8%. MS (ESI) m/z [M + Na]⁺ = 411.0.

### Step 2: Synthesis of G3-3

**G3-2** (130 mg, 335 µmol) was dissolved in tetrahydrofuran (2.0 mL), and 1.0 mL of aqueous solution of lithium hydroxide monohydrate (44.9 mg, 1.07 mmol) was added and stirred at room temperature for 16 h. The reaction solution was concentrated under reduced pressure to remove the solvent to obtain a yellow solid. The crude product **G3-3** (97 mg) was used directly in the next step. MS (ESI) m/z [M - H]⁻ = 289.3.

### Step 3: Synthesis of G3-4

Dried **G3-3** (97 mg, 335 µmol) and 4,4'-dimethoxytriphenylmethane chloride (406 mg, 1.2 mmol) were dissolved in pyridine (4.0 mL) and stirred at room temperature for 16 h under nitrogen protection. Methanol (10 mL) was added to quench the reaction, and the solvent was removed by concentration under reduced pressure to obtain a crude product, which was purified by column chromatography (DCM/MeOH) to obtain a white solid **G3-4** (126.7 mg, 213.9 µmol) with a yield of 63.86%. MS (ESI) m/z [M - H]⁻ = 591.5.

### Step 4: Synthesis of G3-5

**G3-4** (85 mg, 143 µmol), diisopropylethylamine (37.1 mg, 287 µmol, 49.9 µL) and O-benzotriazole-tetramethyluronium hexafluorophosphate (81.6 mg, 215 µmol) were dissolved in N,N-dimethylformamide (3.0 mL), added with GalNAc-NH₂.TFA (274 mg, 143 µmol, TFA) and stirred at room temperature for 2 h under nitrogen protection. The reaction solution was directly used in the next step. MS (ESI) m/z [M - 2H]²⁻ = 1183.2.

### Step 5: Synthesis of G3-6

**G3-5** (339 mg, 143 µmol), 4-dimethylaminopyridine (17.5 mg, 143 µmol), diisopropylethylamine (74.1 mg, 573 µmol) and succinic anhydride (71.7 mg, 717 µmol) were dissolved in N,N-dimethylformamide (3.0 mL) and stirred at 30°C for 16 h under nitrogen protection. Purification was performed by preparative chromatography (H₂O (10 mM TEAB)-ACN) to obtain a white solid **G3-6** (187 mg, 75.7 µmol) with a yield of 52.8%. ¹HNMR (400 MHz, CDCl₃) *δ* ppm 7.46 - 7.41 (m, 2H), 7.34 - 7.27 (m, 5H), 7.25 - 7.21 (m, 2H), 7.19 - 7.14 (m, 1H), 7.05 (t, *J =* 6.1 Hz, 2H), 6.96 (d, *J =* 9.0 Hz, 2H), 6.83 - 6.75 (m, 4H), 6.50 (d, *J=* 5.4 Hz, 1H), 5.34 - 5.31 (m, 2H), 5.18 (dt, *J* = 11.3, 3.9 Hz, 4H), 4.60 (dd, *J* = 8.4, 2.6 Hz, 3H), 4.50 (d, *J* = 8.0 Hz, 1H), 4.18 - 4.04 (m, 9H), 3.95 - 3.88 (m, 5H), 3.76 (s, 5H), 3.69 - 3.58 (m, 12H), 3.52 - 3.46 (m, 3H), 3.32 - 3.11 (m, 14H), 2.80 (q, *J* = 7.2 Hz, 8H), 2.57 - 2.54 (m, 1H), 2.48 (t, *J =* 6.5 Hz, 2H), 2.41 (t, *J =* 5.8 Hz, 5H), 2.29 - 2.06 (m, 18H), 2.05 - 1.91 (m, 24H), 1.76 - 1.51 (m, 18H), 1.40 (t, *J =* 6.7 Hz, 2H), 1.23 - 1.19 (m, 7H), 1.16 - 1.13 (m, 12H). MS (ESI) m/z [M - 2H]²⁻ = 1233.3.

### Step 6: Synthesis of YK-GAL-303

**G3-6** (50 mg, 19.1 µmol), 4-dimethylaminopyridine (2.34 mg, 19.1 µmol), diisopropylethylamine (19.8 mg, 153 µmol), and O-benzotriazole-tetramethyluronium hexafluorophosphate (36.3 mg, 95.7 µmol) were dissolved in N,N-dimethylformamide (4.0 mL), then added with CPG-NH₂ (332 mg) and stirred at 40°C for 16 h. The reaction solution was filtered, and the filtrate was washed with methanol followed by dichloromethane and dried in vacuum. The filtrate was then added to 4 mL of acetic anhydride/pyridine (1:4) solution and stirred at 40°C for 0.5 h. The mixture was filtered, washed with dichloromethane followed by methanol, and the filtrate was dried in vacuum for 12 h to obtain a white solid compound **YK-GAL-303** (292 mg, loading 31.7 µmol/g).

### 4. Synthesis of YK-GAL-304

The synthetic route is as follows:

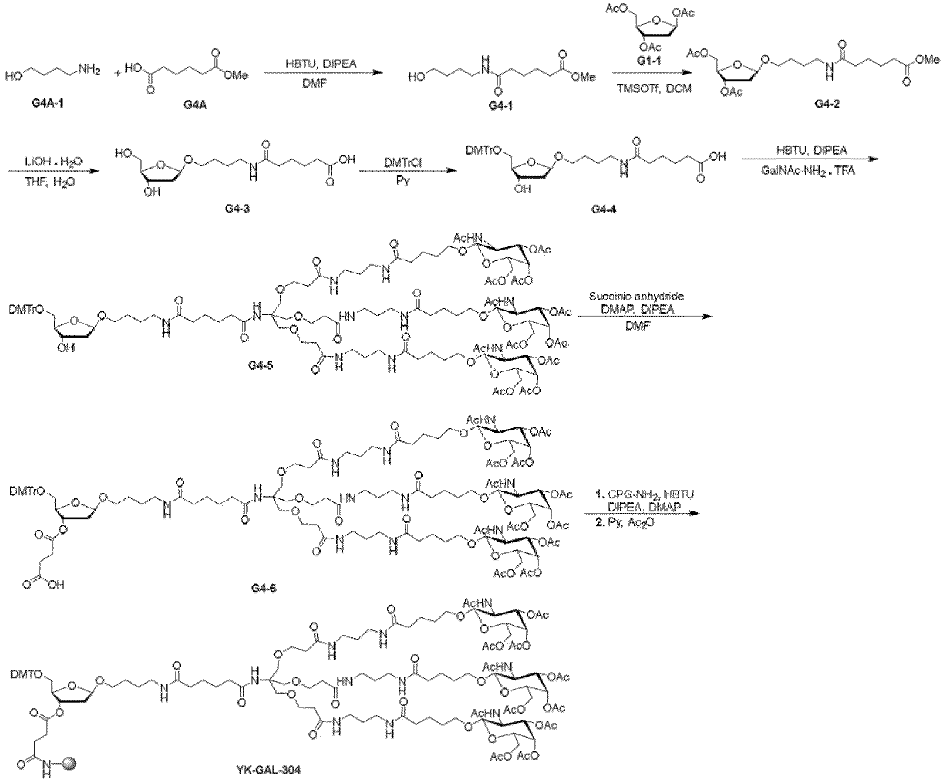

### Step 1: Synthesis of G4-1

**G4A** (3.00 g, 18.7 mmol), diisopropylethylamine (4.84 g, 37.4 mmol) and O-benzotriazole-tetramethyluronium hexafluorophosphate (7.81 g, 20.6 mmol) were dissolved in N,N-dimethylformamide (30.0 mL), added with **G4A-1** (1.50 g, 16.9 mmol) and stirred at 15°C for 2 h. Purification was performed by preparative chromatography (H₂O (0.1% TFA) - ACN) to obtain a white solid **G4-1** (1.70 g). ¹HNMR (400 MHz, DMSO-*d₆*) *δ* ppm 7.73 - 7.75 (m, 1H), 4.69 (s, 1H), 3.62 (s, 3H), 3.35 - 3.57 (m, 2H), 2.99 - 3.02 (m, 2H), 2.27 - 2.30 (m, 2H), 2.04 - 2.05 (m, 2H), 1.38 - 1.48 (s, 8H). MS (ESI) m/z [M + H]⁺ = 231.9.

### Step 2: Synthesis of G4-2

A mixture of compound **G1-1** (900 mg, 3.46 mmol), compound **G4-1** (880 mg, 3.80 mmol) and 4A molecular sieve (1.60 g) was added with dichloromethane (16.0 mL), cooled to 0°C, added with trimethylsilyl trifluoromethanesulfonate (1.54 g, 6.92 mmol) and stirred for 2 h. After quenched by adding aqueous solution of saturated sodium bicarbonate (20.0 mL), the reaction solution was extracted with dichloromethane (20.0 mL × 3). The organic phase was dried over anhydrous sodium sulfate and concentrated under reduced pressure to remove the solvent to obtain a crude product, which was purified by column chromatography (PE/EA) to obtain a colorless oil **G4-2** (617.7 mg, 1.43 mmol) with a yield of 41.4%.¹HNMR (400 MHz, CDCl₃) *δ* ppm 5.69 - 5.54 (m, 1H), 5.30 - 5.17 (m, 1H), 4.31 - 4.04 (m, 3H), 3.75 - 3.62 (m, 4H), 3.43 - 3.34 (m, 1H), 3.31 - 3.19 (m, 2H), 2.41 - 2.29 (m, 3H), 2.21 - 2.16 (m, 3H), 2.13 - 2.00 (m, 5H), 1.72 - 1.51 (m, 10H). MS (ESI) m/z [M + Na]⁺ = 454.0.

### Step 3: Synthesis of G4-3

**G4-2** (160 mg, 371 µmol) was dissolved in tetrahydrofuran (2.2 mL), added with 1.0 mL of aqueous solution of lithium hydroxide monohydrate (49.8 mg, 1.19 mmol), and stirred at 20°C for 16 h. The reaction solution was concentrated under reduced pressure to remove the solvent to obtain a white solid. The crude product **G4-3** (123 mg) was used directly in the next step.

### Step 4: Synthesis of G4-4

Dried **G4-3** (123 mg, 369 µmol) and 4,4'-dimethoxytriphenylmethane chloride (463 mg, 1.37 mmol) were dissolved in pyridine (1.50 mL) and stirred at 25°C for 48 h under nitrogen protection. Methanol (1.00 mL) was added to quench the reaction, and the solvent was removed by concentration under reduced pressure to obtain a crude product, which was purified by preparative chromatography (H₂O (10mM TEAB)-ACN) to obtain a white solid **G4-4** (148 mg, 233 µmol) with a yield of 62.8%. ¹HNMR (400 MHz, CDCl₃) *δ* ppm 7.19 - 7.41 (m, 9H), 6.73 - 6.77 (m, 4H), 5.81 - 5.65 (m, 2H), 5.07 - 5.08 (s, 1H), 4.31 - 4.34 (m, 1H), 3.91 - 3.92 (m, 1H), 3.54 - 3.71 (m, 6H), 3.42 - 3.54 (m, 1H), 3.16 - 3.17 (m, 1H), 3.10 - 3.14 (m, 1H), 3.06 - 3.09 (m, 4H), 2.22 - 2.25 (m, 2H), 1.92 - 2.10 (m, 4H), 1.54 - 1.57 (m, 4H), 1.12 - 1.16 (m, 4H). MS (ESI) m/z [M - H]⁻ = 634.4.

### Step 5: Synthesis of G4-5

**G4-4** (30.0 mg, 47.2 µmol), diisopropylethylamine (12.2 mg, 94.4 µmol) and O-benzotriazole-tetramethyluronium hexafluorophosphate (26.8 mg, 70.8 µmol) were dissolved in N,N-dimethylformamide (1.0 mL), added with GalNAc-NH₂.TFA (45.0 mg, 23.6 µmol, TFA), and stirred at 25°C for 2 h under nitrogen protection. The reaction solution was directly used in the next step. MS (ESI) m/z [M - 2H]²⁻ = 1204.8.

### Step 6: Synthesis of G4-6

**G4-5** (113 mg, 46.9 µmol), 4-dimethylaminopyridine (23.1 mg, 187 µmol), diisopropylethylamine (66.7 mg, 515 µmol), and succinic anhydride (42.2 mg, 421 µmol) were dissolved in N,N-dimethylformamide (1.0 mL) and stirred at 30°C for 48 h under nitrogen protection. Purification was performed by preparative chromatography (H₂O (10 mM TEAB)-ACN) to obtain a white solid **G4-6** (55.3 mg, 22 µmol) with a yield of 47.0%. MS (ESI) m/z [M - 2H]²⁻ = 1254.1.

### Step 7: Synthesis of YK-GAL-304

**G4-6** (20 mg, 7.57 µmol), 4-dimethylaminopyridine (1.00 mg, 8.20 µmol), diisopropylethylamine (7.82 mg, 60.5 µmol), and O-benzotriazole-tetramethyluronium hexafluorophosphate (14.4 mg, 37.8 µmol) were dissolved in N,N-dimethylformamide (1.5 mL), then added with CPG-NH₂ (131 mg) and stirred at 40°C for 16 h. The reaction solution was filtered, and the filtrate was washed with methanol followed by dichloromethane and dried in vacuum. The filtrate was then added to 1.20 mL of acetic anhydride/pyridine (1:5) solution and stirred at 40°C for 0.5 h. The mixture was filtered, washed with dichloromethane followed by methanol, and the filtrate was dried under vacuum for 12 h to obtain a white solid compound **YK-GAL-304** (100 mg, loading 35.57 µmol/g).

### 5. Synthesis of YK-GAL-305

The synthetic route is as follows:

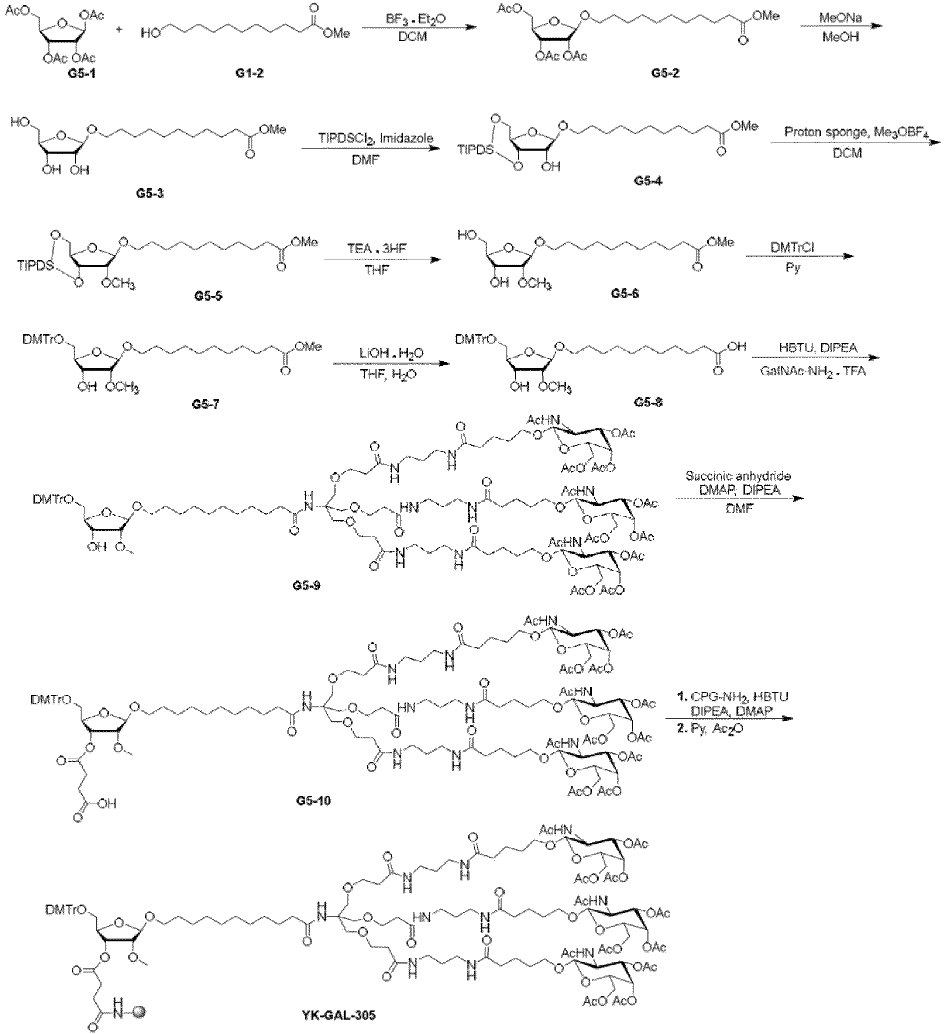

### Step 1: Synthesis of G5-2

A mixture of the dried compound **G1-1** (3.53 g, 11.1 mmol), the dried compound **G1-2** (2.0 g, 9.25 mmol) and 3A molecular sieve (10.0 g) was added with dichloromethane (100.0 mL), cooled to 0°C, added with boron trifluoride etherate (2.62 g, 18.5 mmol), and then stirred for 2 h. After quenched by adding triethylamine (2.7 mL), the reaction solution was washed with aqueous solution of saturated sodium bicarbonate (50 mL). The organic phase was dried over anhydrous sodium sulfate and concentrated under reduced pressure to remove the solvent to obtain a crude product, which was purified by column chromatography (PE/EA) to obtain a colorless oil G5-2 (2.11 g, 4.46 mmol) with a yield of 48.2%. ¹HNMR (400 MHz, CDCl₃) *δ* ppm 5.32 (dd, *J* = 6.7, 4.8 Hz, 1H), 5.25 - 5.18 (m, 1H), 4.97 (s, 1H), 4.39 - 4.22 (m, 2H), 4.14 - 4.00 (m, 1H), 3.73 - 3.60 (m, 4H), 3.36 (dt, *J* = 9.3, 6.7 Hz, 1H), 2.29 (t, *J* = 7.5 Hz, 2H), 2.11 - 2.04 (m, 8H), 1.64 - 1.52 (m, 5H), 1.30 - 1.24 (m, 12H). MS (ESI) m/z [M + Na]⁺ = 497.1.

### Step 2: Synthesis of G5-3

**G5-2** (1.80 g, 3.79 mmol) was dissolved in methanol (10.0 mL). Sodium methoxide (68.3 mg, 379 µmol) was added and stirred at 15°C for 2 h. Hydrogen ion exchange resin was added to quench the reaction, the reaction mixture was filtered and the filtrate was concentrated under reduced pressure to remove the solvent to obtain a yellow solid. The crude product G5-3 (1.32 g) was used directly in the next step. ¹HNMR (400 MHz, CDCl₃) *δ* ppm 4.91 (s, 1H), 4.32 (t, *J =* 5.5 Hz, 1H), 4.08 - 3.97 (m, 2H), 3.76 (dd, *J* = 11.8, 3.3 Hz, 1H), 3.71 - 3.57 (m, 5H), 3.41 (dt, *J =* 9.3, 6.5 Hz, 1H), 2.74 (s, 2H), 2.27 (t, *J* = 7.5 Hz, 2H), 1.55 (dq, *J =* 20.6, 7.0 Hz, 5H), 1.28 - 1.25 (m, 12H). MS (ESI) m/z [M + Na]⁺ = 371.2.

### Step 3: Synthesis of G5-4

**G5-3** (1.32 g, 3.79 mmol) was dissolved in N,N-dimethylformamide (10.0 mL), added with 1,3-dichloro-1,1,3,3-tetraisopropyldisiloxane (1.31 g, 4.17 mmol) and imidazole (645 mg, 9.47 mmol) and stirred at 15°C for 2 h under nitrogen protection. Aqueous solution of saturated sodium bicarbonate (10.0 mL) was added to quench the reaction, and the mixture was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a colorless oil **G5-4** (1.20 g, 2.03 mmol) with a yield of 53.6%. ¹HNMR (400 MHz, CDCl₃) *δ* ppm 4.91 (s, 1H), 4.51 (t, *J* = 5.3 Hz, 1H), 4.06 - 3.97 (m, 3H), 3.76 (dd, *J* = 10.4, 8.7 Hz, 1H), 3.66 (s, 3H), 3.61 (dt, *J* = 9.6, 6.8 Hz, 1H), 3.34 (dt, *J* = 9.5, 6.4 Hz, 1H), 2.97 (s, 1H), 2.30 (t, *J* = 7.5 Hz, 2H), 1.61 (t, *J* = 7.4 Hz, 3H), 1.51 (q, *J* = 6.8 Hz, 3H), 1.27 - 1.32 (m, 12H), 1.11 - 1.02 (m, 26H). MS (ESI) m/z [M + Na]⁺ = 613.4.

### Step 4: Synthesis of G5-5

**G5-4** (1.20 g, 2.03 mmol) was dissolved in dichloromethane (12.0 mL), added with 1,8-bis(dimethylamino) naphthalene (1.18 g, 5.48 mmol) and trimethyloxonium tetrafluoroborate (751 mg, 5.08 mmol) and stirred at 15°C for 16 h under nitrogen protection. Aqueous ammonium chloride solution (10.0 mL) was added to quench the reaction, and the mixture was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a colorless oil **G5-5** (1.05 g, 1.74 mmol) with a yield of 85.5%. ¹HNMR (400 MHz, CDCl₃) *δ* ppm 4.84 (s, 1H), 4.49 (dd, *J* = 7.6, 4.3 Hz, 1H), 3.98 (dq, *J* = 9.0, 3.1 Hz, 2H), 3.86 (dd, *J* = 12.6, 6.6 Hz, 1H), 3.69 - 3.54 (m, 8H), 3.40 - 3.27 (m, 1H), 2.30 (t, *J* = 7.5 Hz, 2H), 1.65 - 1.57 (m, 2H), 1.53 - 1.48 (m, 2H), 1.33 - 1.24 (m, 12H), 1.11 - 0.97 (m, 28H). MS (ESI) m/z [M + Na]⁺ = 627.4.

### Step 5: Synthesis of G5-6

**G5-5** (1.05 g, 1.74 mmol) was dissolved in tetrahydrofuran (10.0 mL), added with triethylamine trihydrofluoride (839 mg, 5.20 mmol) and stirred at 15°C for 16 h. Aqueous solution of saturated sodium bicarbonate (10.0 mL) was added to quench the reaction, and the mixture was extracted with dichloromethane (10 mL × 2). The organic phase was dried over anhydrous sodium sulfate, and the solvent was removed by concentration under reduced pressure to obtain a crude product **G5-6** (0.63 g), which was used directly in the next step. ¹HNMR (400 MHz, CDCl₃) *δ* ppm 4.93 (d, *J* = 1.3 Hz, 1H), 4.25 (t, *J* = 5.2 Hz, 1H), 4.02 - 3.94 (m, 1H), 3.75 - 3.54 (m, 6H), 3.48 - 3.31 (m, 4H), 2.55 (brs, 1H), 2.23 (t, *J* = 7.5 Hz, 2H), 1.53 (dp, *J* = 14.1, 7.0 Hz, 4H), 1.22 (d, *J =* 5.8 Hz, 12H), 0.99 (dq, *J =* 5.8, 3.1, 2.6 Hz, 2H). MS (ESI) m/z [M + Na]⁺ = 385.2.

### Step 6: Synthesis of G5-7

Dried **G5-6** (0.63 g, 1.74 mmol) and 4,4'-dimethoxytriphenylmethane chloride (618 mg, 1.83 mmol) were dissolved in pyridine (10.0 mL) and stirred at 15°C for 16 h under nitrogen protection. Aqueous solution of saturated sodium bicarbonate (10.00 mL) was added to quench the reaction, and the mixture was extracted with dichloromethane (15.0 mL × 2). The organic phase was dried over anhydrous sodium sulfate and concentrated under reduced pressure to remove the solvent to obtain a crude product, which was purified by column chromatography (PE/EA) to obtain a white solid **G5-7** (1.01 g, 1.52 mmol) with a yield of 87.4%. ¹HNMR (400 MHz, CDCl₃) *δ* ppm 7.52 - 7.46 (m, 2H), 7.40 - 7.34 (m, 4H), 7.29 - 7.24 (m, 2H), 7.22 - 7.16 (m, 1H), 6.83 - 6.78 (m, 4H), 5.02 (d, *J* = 1.4 Hz, 1H), 4.19 (dt, *J* = 8.3, 5.5 Hz, 1H), 4.04 (td, *J* = 5.8, 3.8 Hz, 1H), 3.78 (brs, 6H), 3.76 - 3.63 (m, 6H), 3.50 (s, 3H), 3.38 (dt, *J* = 9.4, 6.8 Hz, 1H), 3.27 (dd, *J =* 9.9, 3.8 Hz, 1H), 3.14 (dd, *J* = 9.9, 5.6 Hz, 1H), 2.48 (d, *J* = 8.4 Hz, 1H), 2.29 (t, *J* = 7.6 Hz, 2H), 1.60 (d, *J* = 14.7 Hz, 2H), 1.28 - 1.23 (m, 11H). MS (ESI) m/z [M - H]⁻ = 663.6.

### Step 7: Synthesis of G5-8

**G5-7** (1.00 g, 1.50 mmol) was dissolved in tetrahydrofuran (12.0 mL), added with 6.0 mL of aqueous solution of lithium hydroxide monohydrate (94.7 mg, 2.26 mmol) and stirred at 15°C for 12 h. The reaction solution was concentrated under reduced pressure to remove the solvent to obtain a yellow solid. The crude product **G5-8** (0.98 g) was used directly in the next step. ¹HNMR (400 MHz, CDCl₃) *δ* ppm 7.53 - 7.45 (m, 2H), 7.35 (d, *J* = 8.50 Hz, 4H), 7.23 (t, *J* = 7.19 Hz, 2H), 7.19 - 7.14 (m, 1H), 6.84 - 6.73 (m, 4H), 5.04 (s, 1H), 4.26 - 4.00 (m, 2H), 3.80 - 3.63 (m, 8H), 3.45 (s, 3H), 3.39 (s, 1H), 3.30 - 3.20 (m, 1H), 3.12 (br s, 1H), 2.12 - 2.05 (m, 1H), 2.04 - 1.98 (m, 3H), 1.58 -1.41 (m, 4H), 1.19 (br s, 12H),. MS (ESI) m/z [M - H]⁻ = 649.6.

### Step 8: Synthesis of G5-9

**G5-8** (0.97 g, 1.49 mmol), diisopropylethylamine (385 mg, 2.98 mmol) and O-benzotriazole-tetramethyluronium hexafluorophosphate (848 mg, 2.24 mmol) were dissolved in N,N-dimethylformamide (35.0 mL), added with GalNAc-NH₂.TFA (2.84 g, 1.49 mmol, TFA) and stirred at 15°C for 1 h under nitrogen protection. The reaction solution was used directly in the next step. MS (ESI) m/z [M - 2H]²⁻ = 1212.4.

### Step 9: Synthesis of G5-10

**G5-9** (3.62 g, 1.49 mmol), 4-dimethylaminopyridine (364 mg, 2.98 mmol), diisopropylethylamine (771 mg, 5.97 mmol), and succinic anhydride (896 mg, 8.95 mmol) were dissolved in N,N-dimethylformamide (35.0 mL) and stirred at 30°C for 48 h under nitrogen protection. Purification was performed by preparative chromatography (H₂O (50 mM TEAB)-ACN) to obtain a white solid **G5-10** (2.10 g, 937 µmol) with a yield of 62.8%. ¹HNMR (400 MHz, CDCl₃) *δ* ppm 7.48 - 7.41 (m, 2H), 7.33 (d, *J =* 8.6 Hz, 3H), 7.28 - 7.21 (m, 5H), 7.17 (t, *J* = 7.2 Hz, 1H), 6.99 (t, *J* = 6.3 Hz, 2H), 6.87 - 6.77 (m, 5H), 6.49 (d, *J* = 6.0 Hz, 1H), 5.34 (d, *J* = 3.3 Hz, 3H), 5.21 - 5.13 (m, 3H), 4.98 (d, *J* = 2.3 Hz, 1H), 4.61 (d, *J* = 8.5 Hz, 2H), 4.23 (q, *J =* 4.9 Hz, 1H), 4.19 - 4.01 (m, 8H), 3.91 (dt, *J* = 13.1, 5.6 Hz, 6H), 3.77 (s, 5H), 3.68 (d, *J* = 7.6 Hz, 11H), 3.55 - 3.44 (m, 3H), 3.39 - 3.19 (m, 14H), 3.11 (dd, *J* = 9.9, 5.0 Hz, 1H), 2.80 - 2.59 (m, 13H), 2.49 (t, *J* = 7.4 Hz, 2H), 2.42 (t, *J* = 5.7 Hz, 5H), 2.30 - 2.06 (m, 16H), 2.06 - 1.91 (m, 22H), 1.81 - 1.44 (m, 19H), 1.31 - 1.19 (m, 10H), 1.11 (t, *J =* 7.2 Hz, 16H). MS (ESI) m/z [M - 2H]²⁻ = 1262.5.

### Step 10: Synthesis of YK-GAL-305

**G5-10** (1.00 g, 380 µmol), 4-dimethylaminopyridine (46.5 mg, 381 µmol), diisopropylethylamine (393 mg, 3.04 mmol), and O-benzotriazole-tetramethyluronium hexafluorophosphate (722 mg, 1.90 mmol) were dissolved in N,N-dimethylformamide (65.0 mL), then added with CPG-NH₂ (6.60 g) and stirred at 40°C for 16 h. The reaction solution was filtered, and the filtrate was washed with methanol followed by dichloromethane and dried in vacuum. The filtrate was then added to 65 mL of acetic anhydride/pyridine (1:5) solution and stirred at 40°C for 0.5 h. The mixture was filtered, washed with dichloromethane followed by methanol, and the filtrate was dried in vacuum for 12 h to obtain a white solid compound **YK-GAL-305** (7.05 g, loading 34.4 µmol/g).

### 6. Synthesis of YK-GAL-306

The synthetic route is as follows:

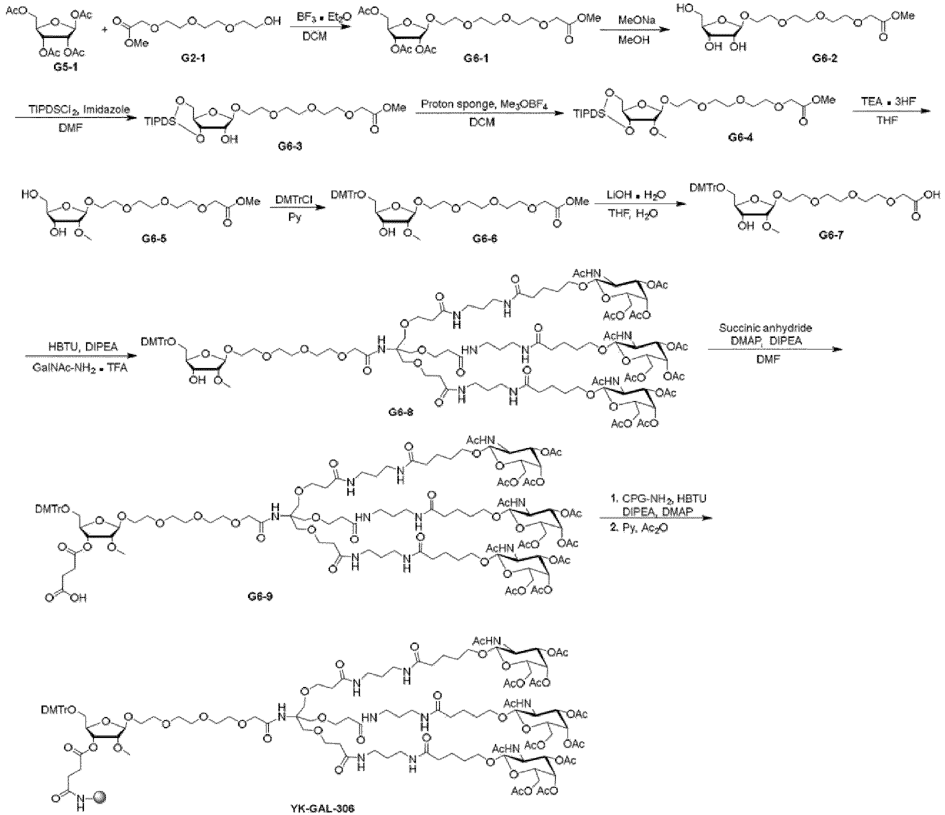

### Step 1: Synthesis of G6-1

A mixture of the dried compound **G5-1** (1.58 g, 4.95 mmol), the dried compound **G2-1** (1.00 g, 4.50 mmol) and 3A molecular sieve (1.50 g, 4.50 mmol) was added with dichloromethane (15.0 mL), cooled to 0°C, added with boron trifluoride etherate (1.28 g, 9.00 mmol), stirred for 2 h, heated to 15°C and then stirred for 2 h. After quenched by adding triethylamine (1.32 mL), the reaction solution was washed with aqueous solution of saturated sodium bicarbonate (30.0 mL × 2). The organic phase was dried over anhydrous sodium sulfate and concentrated under reduced pressure to remove the solvent to obtain a crude product, which was purified by column chromatography (PE/EA) to obtain a yellow oil **G6-1** (1.60 g, 3.33 mmol) with a yield of 74.0%. ¹HNMR (400 MHz, CDCl₃) *δ* ppm 5.34 (dd, *J* = 6.7, 4.8 Hz, 1H), 5.26 (d, *J =* 4.8 Hz, 1H), 4.37 - 4.25 (m, 2H), 4.12 (dd, *J* = 11.3, 5.9 Hz, 2H), 3.89 - 3.77 (m, 1H), 3.75 - 3.56 (m, 16H), 2.10 (s, 3H), 2.08 (s, 3H), 2.04 (s, 3H). MS (ESI) m/z [M + Na]⁺ = 503.0.

### Step 2: Synthesis of G6-2

**G6-1** (1.60 g, 3.33 mmol) was dissolved in methanol (16.0 mL), added with sodium methoxide (60.0 mg, 333 µmol) and stirred at 15°C for 2 h. Hydrogen ion exchange resin was added to quench the reaction, the reaction mixture was filtered and filtrate was concentrated under reduced pressure to remove the solvent to obtain a white solid. The crude product G6-2 (1.18 g) was used directly in the next step. ¹H NMR (400 MHz, MeOH-*d*₄) δ 4.89 (s, 1H), 4.13 (dd, *J* = 7.1, 4.7 Hz, 1H), 3.99 - 3.89 (m, 2H), 3.88 - 3.80 (m, 1H), 3.78 - 3.53 (m, 18H). MS (ESI) m/z [M + Na]⁺ = 376.9.

### Step 3: Synthesis of G6-3

**G6-2** (1.18 g, 3.33 mmol) was dissolved in N,N-dimethylformamide (12.0 mL), added with 1,3-dichloro-1,1,3,3-tetraisopropyldisiloxane (1.16 g, 3.66 mmol) and imidazole (567 mg, 8.33 mmol) and stirred at 15°C for 2 h under nitrogen protection. Aqueous solution of saturated sodium bicarbonate (10.0 mL) was added to quench the reaction, and the mixture was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a colorless oil G6-3 (1.38 g, 2.27 mmol) with a yield of 68.0%. ¹HNMR (400 MHz, MeOH-*d*₄) *δ* ppm 4.52 (t, *J* = 5.4 Hz, 1H), 4.09 - 3.97 (m, 3H), 3.80 - 3.54 (m, 20H), 1.09 - 1.02 (m, 28H).

### Step 4: Synthesis of G6-4

**G6-3** (1.25 g, 2.09 mmol) was dissolved in dichloromethane (17.0 mL), added with 1,8-bis(dimethylamino) naphthalene (1.48 g, 6.91 mmol) and trimethyloxonium tetrafluoroborate (929 mg, 6.28 mmol) and stirred at 15°C for 6 h under nitrogen protection. Aqueous solution of ammonium chloride (30.0 mL) was added to quench the reaction, and the mixture was extracted with (15 mL × 2). The organic phase was dried over anhydrous sodium sulfate and concentrated under reduced pressure to remove the solvent to obtain a crude product, which was purified by column chromatography (PE/EA) to obtain a colorless oil **G6-4** (1.11 g, 1.82 mmol) with a yield of 86%. ¹HNMR (400 MHz, MeOH-*d*₄) *δ* ppm 4.43 (dd, *J* = 8.1, 4.3 Hz, 1H), 3.95 - 3.44 (m, 26H), 1.03 - 0.95 (m, 28H).

### Step 5: Synthesis of G6-5

**G6-4** (1.11 g, 1.82 mmol) was dissolved in tetrahydrofuran (20.0 mL), added with triethylamine trihydrofluoride (1.46 g, 9.09 mmol), and stirred at 20°C for 2 h. Aqueous solution of saturated sodium bicarbonate (10.0 mL) was added to quench the reaction, and the mixture was extracted with dichloromethane (25 mL × 2). The organic phase was dried over anhydrous sodium sulfate and concentrated under reduced pressure to remove the solvent to obtain a colorless oil. The crude product **G6-5** (0.67 g) was used directly in the next step.

### Step 6: Synthesis of G6-6

Dried **G6-5** (0.67 g, 1.82 mmol) and 4,4'-dimethoxytriphenylmethane chloride (616 mg, 1.82 mmol) were dissolved in pyridine (10.0 mL) and stirred at 25°C for 2 h under nitrogen protection. Methanol (1.00 mL) was added to quench the reaction, and the solvent was removed by concentration under reduced pressure to obtain a crude product, which was purified by preparative chromatography (H₂O (10 mM NH₄HCO₃)-ACN) to obtain a white solid **G6-6** (1.05 g, 1.57 mmol) with a yield of 86.0%. ¹HNMR (400 MHz, CD₃CN) *δ* ppm 7.46 - 7.36 (m, 2H), 7.31 - 7.25 (m, 4H), 7.22 - 7.16 (m, 2H), 7.15 - 7.06 (m, 1H), 6.79 - 6.71 (m, 4H), 4.15 (s, 1H), 4.08 (s, 2H), 3.97 (td, *J* = 6.1, 3.8 Hz, 1H), 3.77 - 3.42 (m, 27H), 3.21 (dd, *J* = 9.9, 3.7 Hz, 1H), 3.08 (dd, *J* = 9.9, 5.8 Hz, 1H). MS (ESI) m/z [M - H]⁻ = 669.4.

### Step 7: Synthesis of G6-7

**G6-6** (195 mg, 291 µmol) was dissolved in tetrahydrofuran (1.0 mL), added with 1.0 mL of aqueous solution of lithium hydroxide monohydrate (60.0 mg, 333 µmol), and stirred at 25°C for 12 h. The reaction solution was concentrated under reduced pressure to remove the solvent to obtain a white solid. The crude product **G6-7** (190 mg) was directly used in the next step. ¹HNMR (400 MHz, MeOH-*d*₄) *δ* ppm 7.52 - 7.45 (m, 2H), 7.39 - 7.31 (m, 4H), 7.31 - 7.24 (m, 2H), 7.24 - 7.15 (m, 1H), 6.92 - 6.80 (m, 4H), 4.22 (dd, *J* = 7.2, 4.7 Hz, 1H), 4.07 - 4.03 (m, 1H), 3.86 - 3.68 (m, 10H), 3.65 - 3.51 (m, 12H), 3.49 (s, 3H), 3.27 (dd, *J =* 10.0, 3.1 Hz, 1H), 3.12 (dd, *J* = 10.0, 5.8 Hz, 1H). MS (ESI) m/z [M - H]⁻ = 655.4.

### Step 8: Synthesis of G6-8

**G6-7** (190 mg, 293 µmol), diisopropylethylamine (112 mg, 868 µmol) and O-benzotriazole-tetramethyluronium hexafluorophosphate (165 mg, 433 µmol) were dissolved in N,N-dimethylformamide (5.0 mL), added with GalNAc-NH₂.TFA (442 mg, 232 µmol, 0.8 eq, TFA) and stirred at 15°C for 2 h under nitrogen protection. The reaction solution was used directly in the next step. MS (ESI) m/z [M - 2H]²⁻ = 1215.0.

### Step 9: Synthesis of G6-9

**G6-8** (703 mg, 289 µmol), 4-dimethylaminopyridine (70.6 mg, 578 µmol), diisopropylethylamine (261 mg, 2.02 mmol), and succinic anhydride (173 mg, 1.73 mmol) were dissolved in N,N-dimethylformamide (1.0 mL) and stirred at 30°C for 48 h under nitrogen protection. Purification was performed by preparative chromatography (H₂O (50 mM TEAB)-ACN) to obtain a white solid **G6-9** (300 mg, 118 mol) with a yield of 50.0%. MS (ESI) m/z [M - 2H]²⁻ = 1265.3.

### Step 10: Synthesis of YK-GAL-306

**G6-9** (300 mg, 110 µmol), 4-dimethylaminopyridine (13.4 mg, 109 µmol), diisopropylethylamine (113 mg, 877 µmol), and O-benzotriazole-tetramethyluronium hexafluorophosphate (208 mg, 548 µmol) were dissolved in N,N-dimethylformamide (16.0 mL), then added with CPG-NH₂ (1.9 g) and stirred at 40°C for 12 h. The reaction solution was filtered, and the filtrate was washed with methanol followed by dichloromethane and dried in vacuum. The filtrate was then added to 20 mL of acetic anhydride/pyridine (1:5) solution and stirred at 40°C for 0.5 h. The mixture was filtered, washed with dichloromethane followed by methanol, and the filtrate was dried in vacuum for 12 h to obtain a white solid compound **YK-GAL-306** (1.94 g, loading 37.43 µmol/g).

### 7. Synthesis of YK-GAL-307

The synthetic route is as follows:

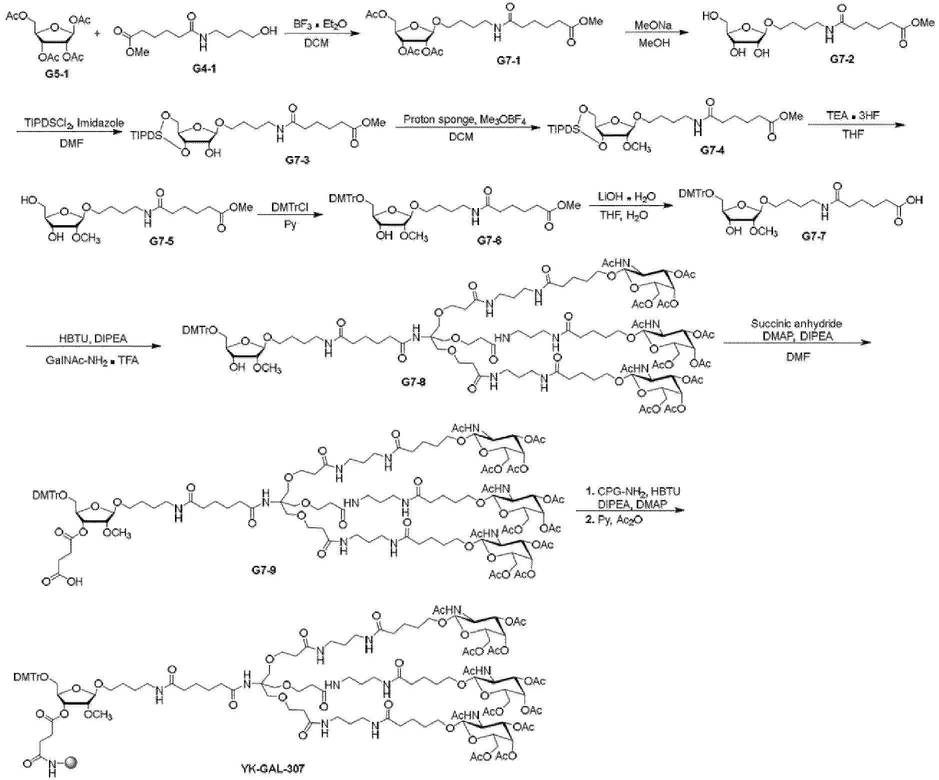

### Step 1: Synthesis of G7-1

A mixture of compound **G5-1** (5.00 g, 15.7 mmol), the dried compound **G4-1** (3.50 g, 15.1 mmol) and 4A molecular sieve (1.0 g) was added with dichloromethane (70.0 mL), cooled to 0°C, added with boron trifluoride etherate (4.30 g, 30.2 mmol) and stirred for 2 h. After quenched by adding triethylamine (3.06 g, 30.2 mmol), the reaction solution was washed with aqueous solution of saturated sodium bicarbonate (20 mL). The organic phase was dried over anhydrous sodium sulfate and concentrated under reduced pressure to remove the solvent to obtain a crude product, which was purified by column chromatography (PE/EA) to obtain a colorless oil **G7-1** (3.70 g, 7.56 mmol) with a yield of 50.1%. ¹HNMR (400 MHz, CDCl₃) *δ* ppm 5.74 (d, *J* = 31.1 Hz, 1H), 5.33 - 5.14 (m, 1H), 4.98 (d, *J* = 13.6 Hz, 1H), 4.30 - 4.26 (m, 1H), 4.19 - 4.03 (m, 2H), 3.87 - 3.61 (m, 4H), 3.43 - 3.37 (m 1H), 3.33 - 3.18 (m, 2H), 2.35 - 2.31 (m, 2H), 2.23 - 1.98 (m, 11H), 1.71 - 1.50 (m, 9H). MS (ESI) m/z [M + Na]⁺ = 512.1.

### Step 2: Synthesis of G7-2

**G7-1** (3.10 g, 6.33 mmol) was dissolved in methanol (30.0 mL), added with sodium methoxide (114 mg, 633 µmol) and stirred at 25°C for 2 h. Hydrogen ion exchange resin was added to quench the reaction, and the filtrate was filtered and concentrated under reduced pressure to remove the solvent to obtain a yellow oil. The crude product **G7-2** (2.20 g) was used directly in the next step. MS (ESI) m/z [M - H]⁻ = 362.2.

### Step 3: Synthesis of G7-3

**G7-2** (2.20 g, 6.05 mmol) was dissolved in N,N-dimethylformamide (20.0 mL), added with 1,3-dichloro-1,1,3,3-tetraisopropyldisiloxane (2.10 g, 6.66 mmol) and imidazole (1.03 g, 15.1 mmol) and stirred at 25°C for 2 h under nitrogen protection. Aqueous solution of saturated sodium bicarbonate (10.0 mL) was added to quench the reaction, and the mixture was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a yellow oil **G7-3** (2.15 g, 3.56 mmol) with a yield of 58.8%. ¹HNMR (400 MHz, CDCl₃) *δ* ppm 5.59 (s, 1H), 4.90 (s, 1H), 4.49 (t, *J =* 5.4 Hz, 1H), 4.08 - 3.91 (m, 3H), 3.80 - 3.60 (m, 6H), 3.40 - 3.35 (m, 1H), 3.26 (q, *J =* 6.4 Hz, 2H), 2.35 - 2.32 (m, 2H), 2.22 - 2.15 (m, 2H), 1.68 - 1.64 (p, *J =* 3.7 Hz, 4H), 1.59 - 1.54 (m, 4H), 1.10 - 1.02 (m, 28H). MS (ESI) m/z [M + H]⁺= 606.4.

### Step 4: Synthesis of G7-4

**G7-3** (1.0 g, 1.65 mmol) was dissolved in dichloromethane (15.0 mL), added with 1,8-bis(dimethylamino) naphthalene (955 mg, 4.46 mmol) and trimethyloxonium tetrafluoroborate (610 mg, 4.13 mmol) and stirred at 15°C for 6 h under nitrogen protection. Aqueous solution of ammonium chloride (10.0 mL) was added to quench the reaction, and the mixture was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a colorless oil **G7-4** (890.2 mg, 1.43 mmol) with a yield of 87.1%. ¹HNMR (400 MHz, CDCl₃) *δ* ppm 5.59 (d, *J =* 6.1 Hz, 1H), 4.53 - 4.42 (m, 1H), 4.08 - 3.85 (m, 3H), 3.80 - 3.60 (m, 5H), 3.58 (d, *J =* 7.2 Hz, 2H), 3.47 - 3.31 (m, 3H), 3.27 - 3.20 (m, 2H), 2.38 - 2.29 (m, 2H), 2.18 (q, *J =* 6.2, 5.0 Hz, 2H), 1.66 (h, *J =* 3.8 Hz, 4H), 1.60 - 1.51 (m, 4H), 1.17 - 0.89 (m, 28H). MS (ESI) m/z [M + H]⁺= 620.5.

### Step 5: Synthesis of G7-5

**G7-4** (300 mg, 484 µmol) was dissolved in tetrahydrofuran (10.0 mL), added with triethylamine trihydrofluoride (390 mg, 2.42 mmol) and stirred at 25°C for 3 h. Aqueous solution of saturated sodium bicarbonate (10.0 mL) was added to quench the reaction, and the mixture was extracted with dichloromethane (20 mL × 3). The organic phase was dried over anhydrous sodium sulfate and concentrated under reduced pressure to remove the solvent to obtain a yellow oil. The crude product **G7-6** (182 mg) was used directly in the next step. MS (ESI) m/z [M + H]⁺ = 378.2.

### Step 6: Synthesis of G7-6

Dried **G7-5** (180 mg, 477 µmol) and 4,4'-dimethoxytriphenylmethane chloride (161 mg, 477 µmol) were dissolved in pyridine (2.0 mL) and stirred at 25°C for 3 h under nitrogen protection. Methanol (2.00 mL) was added to quench the reaction, and the solvent was removed by concentration under reduced pressure to obtain a crude product, which was purified by preparative chromatography (H₂O (10 mM NH₄HCO₃)-ACN) to obtain a white solid G7-6 (262 mg, 385.7 µmol) with a yield of 80.85%. ¹HNMR (400 MHz, CDCl₃) *δ* ppm 7.52 - 7.45 (m, 2H), 7.40 - 7.32 (m, 4H), 7.30 - 7.26 (m, 2H), 7.19 (dd, *J =* 14.1, 8.0 Hz, 1H), 6.87 - 6.78 (m, 4H), 5.38 (s, 1H), 5.01 (d, *J =* 1.5 Hz, 1H), 4.26 - 4.17 (m, 1H), 4.07 - 4.03 (m, 1H), 3.82 - 3.71 (m, 7H), 3.66 (d, *J =* 6.2 Hz, 4H), 3.51 (d, *J =* 2.2 Hz, 3H), 3.41 (dd, *J =* 9.7, 5.9 Hz, 1H), 3.30 (dd, *J* = 10.0, 3.7 Hz, 1H), 3.22 - 3.07 (m, 3H), 2.48 (d, *J =* 8.3 Hz, 1H), 2.34 - 2.29 (m, 2H), 2.07 - 1.98 (m, 2H), 1.60 - 1.47 (m, 8H). MS (ESI) m/z [M - H]⁻= 678.5.

### Step 7: Synthesis of G7-7

**G7-6** (100 mg, 147 µmol) was dissolved in tetrahydrofuran (2.5 mL), added with 2.5 mL of aqueous solution of lithium hydroxide monohydrate (9.26 mg, 220 µmol), and stirred at 15°C for 16 h. The reaction solution was concentrated under reduced pressure to remove the solvent to obtain a yellow solid. The crude product **G7-7** (0.95 g) was used directly in the next step. MS (ESI) m/z [M - H]⁻= 664.5.

### Step 8: Synthesis of G7-8

**G7-7** (90.0 mg, 135 µmol), diisopropylethylamine (34.9 mg, 270 µmol) and O-benzotriazole-tetramethyluronium hexafluorophosphate (76.9 mg, 202 µmol) were dissolved in N,N-dimethylformamide (2.0 mL), added with GalNAc-NH₂.TFA (206 mg, 108 µmol, TFA) and stirred at 15°C for 4 h under nitrogen protection. The reaction solution was used directly in the next step. MS (ESI) m/z [M - 2H]²⁻ = 1219.9.

### Step 9: Synthesis of G7-9

**G7-8** (280 mg, 114 µmol), 4-dimethylaminopyridine (28.0 mg, 229 µmol), diisopropylethylamine (44.4 mg, 344 µmol), and succinic anhydride (91.8 mg, 917 µmol) were dissolved in N,N-dimethylformamide (2.0 mL) and stirred at 30°C for 48 h under nitrogen protection. Purification was performed by preparative chromatography (H₂O (50 mM TEAB)-ACN) to obtain a yellow solid **G7-9** (172 mg, 68.1 µmol) with a yield of 59.7%. ¹HNMR (400 MHz, CDCl₃) *δ* ppm 7.54 - 7.45 (m, 4H), 7.38 - 7.24 (m, 7H), 7.23 - 7.20 (m, 1H), 7.15 - 7.07 (m, 5H), 6.83 (dd, *J =* 8.8, 3.8 Hz, 4H), 5.37 (d, *J =* 3.8 Hz, 2H), 5.24 - 5.16 (m, 3H), 5.01 (t, *J =* 3.1 Hz, 1H), 4.65 (dd, *J =* 8.4, 3.9 Hz, 2H), 4.26 (t, *J =* 4.7 Hz, 1H), 4.21 - 4.07 (m, 8H), 3.99 - 3.91 (m, 6H), 3.81 - 3.75 (m, 8H), 3.70 - 3.65 (m, 11H), 3.55 - 3.50 (m, 2H), 3.40 (d, *J* = 3.9 Hz, 3H), 3.29 - 3.25 (m, 12H), 2.81 - 2.64 (m, 16H), 2.52 (dd, *J =* 7.8, 4.0 Hz, 2H), 2.46 - 2.42 (m, 5H), 2.26 - 2.16 (m, 16H), 2.09 - 1.94 (m, 26H), 1.75 - 1.54 (m, 14H), 1.16 - 1.11 (m, 18H). MS (ESI) m/z [M - 2H]²⁻= 1269.9.

### Step 10: Synthesis of YK-GAL-307

**G7-9** (120 mg, 47.2 µmol), 4-dimethylaminopyridine (5.77 mg, 47.2 µmol), diisopropylethylamine (48.8 mg, 377 µmol), and O-benzotriazole-tetramethyluronium hexafluorophosphate (89.5 mg, 236 µmol) were dissolved in N,N-dimethylformamide (10.0 mL), added with CPG-NH₂ (790 mg) and stirred at 40°C for 16 h. The reaction solution was filtered, and the filtrate was washed with methanol followed by dichloromethane and dried in vacuum. The filtrate was then added to 12 mL of acetic anhydride/pyridine (1:5) solution and stirred at 40°C for 0.5 h. The mixture was filtered, washed with dichloromethane followed by methanol, and the filtrate was dried in vacuum for 12 h to obtain a white solid compound **YK-GAL-307** (810 mg, loading 38.9 µmol/g).

### 8. Synthesis of YK-GAL-308

The synthetic route is as follows:

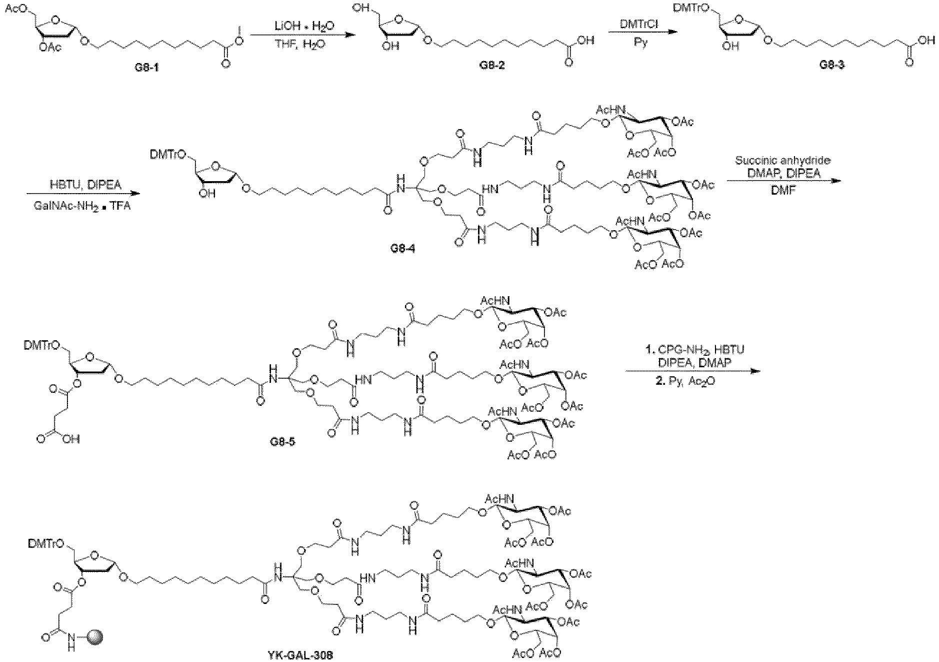

### Step 1: Synthesis of G8-2

**G8-1** (0.34 g, 816 µmol) was dissolved in tetrahydrofuran (8.0 mL), added with 4.0 mL of aqueous solution of lithium hydroxide monohydrate (109 mg, 2.61 mmol), and stirred at 15°C for 12 h. The reaction solution was concentrated under reduced pressure to remove the solvent to obtain a yellow solid. The crude product **G8-2** (0.26 g) was used directly in the next step. MS (ESI) m/z [M + Na]⁺ = 341.2.

### Step 2: Synthesis of G8-3

The crude product **G8-2** (0.26 g, 816 µmol) and anhydrous pyridine (20 mL × 3) were co-rotary evaporated to remove water. The dried **G8-2** and 4,4'-dimethoxytriphenylmethane chloride (580 mg, 1714 µmol) were dissolved in pyridine (6.0 mL) and stirred at 15°C for 36 h under nitrogen protection. Methanol (20 mL) was added to quench the reaction, and the solvent was removed by concentration under reduced pressure to obtain a crude product, which was purified by preparative chromatography (H₂O (10 mM NH₄HCO₃)-ACN) to obtain a white solid **G8-3** (401.9 mg, 647.9 µmol) with a yield of 79.4%. MS (ESI) m/z [M + Na]⁺ = 643.4.

### Step 3: Synthesis of G8-4

**G8-3** (90 mg, 145 µmol), diisopropylethylamine (37.5 mg, 290 µmol) and O-benzotriazole-tetramethyluronium hexafluorophosphate (82.5 mg, 217 µmol) were dissolved in N,N-dimethylformamide (3.0 mL), added with GalNAc-NH₂.TFA (331.3 mg, 174 µmol, 1.2 eq, TFA) and stirred at 15°C for 2 h under nitrogen protection. The reaction solution was used directly in the next step. MS (ESI) m/z [M - 2H]²⁻ = 1197.3.

### Step 4: Synthesis of G8-5

**G8-4,** 4-dimethylaminopyridine (35.4 mg, 289 µmol), diisopropylethylamine (149.8 mg, 1158 µmol), and succinic anhydride (159.4 mg, 1592 µmol) were dissolved in N,N-dimethylformamide (3.0 mL) and stirred at 30°C for 48 h under nitrogen protection. Purification was performed by preparative chromatography (H₂O (10 mM TEAB)-ACN) to obtain a white solid **G8-5** (190 mg, 85.9 µmol) with a yield of 59.4%. ¹HNMR (400 MHz, CDCl₃) *δ* ppm 7.44 - 7.37 (m, 2H), 7.34 - 7.26 (m, 8H), 7.20 (d, *J* = 7.1 Hz, 2H), 6.99 (d, *J* = 5.7 Hz, 4H), 6.81 (d, *J* = 8.7 Hz, 4H), 6.72 (d, *J* = 8.9 Hz, 2H), 6.57 (s, 1H), 5.35 (d, *J* = 3.3 Hz, 2H), 5.26 (d, *J =* 5.1 Hz, 1H), 5.23 - 5.12 (m, 4H), 4.61 (d, *J* = 8.4 Hz, 3H), 4.23 - 4.04 (m, 10H), 3.95 - 3.89 (m, 6H), 3.78 (s, 6H), 3.73 - 3.62 (m, 14H), 3.53 - 3.47 (m, 4H), 3.43 - 3.35 (m, 2H), 3.30 - 3.24 (m, 12H), 3.17 (dd, *J* = 9.9, 4.0 Hz, 2H), 2.62 - 2.57 (m, 4H), 2.43 (d, *J* = 5.7 Hz, 6H), 2.28 - 2.15 (m, 13H), 2.10 - 1.92 (m, 30H), 1.74 - 1.51 (m, 22H), 1.238 - 1.26 (m, 14H). MS (ESI) m/z [M - 2H]²⁻ = 1247.4.

### Step 5: Synthesis of YK-GAL-308

**G8-5** (190 mg, 76.1 µmol), 4-dimethylaminopyridine (9.30 mg, 76.1 µmol), diisopropylethylamine (78.7 mg, 609 µmol), and O-benzotriazole-tetramethyluronium hexafluorophosphate (144 mg, 381 µmol) were dissolved in N,N-dimethylformamide (12 mL), added with CPG-NH₂ (1.27 g) and stirred at 40°C for 16 h. The reaction solution was filtered, and the filtrate was washed with methanol followed by dichloromethane and dried in vacuum. The filtrate was then added to 12 mL of acetic anhydride/pyridine (1:5) solution and stirred at 40°C for 0.5 h. The mixture was filtered, washed with dichloromethane followed by methanol, and the filtrate was dried in vacuum for 12 h to obtain a white solid compound YK-GAL-308 (1.24 g, loading 29.7 µmol/g).

### 9. Synthesis of YK-GAL-309

The synthetic route is as follows:

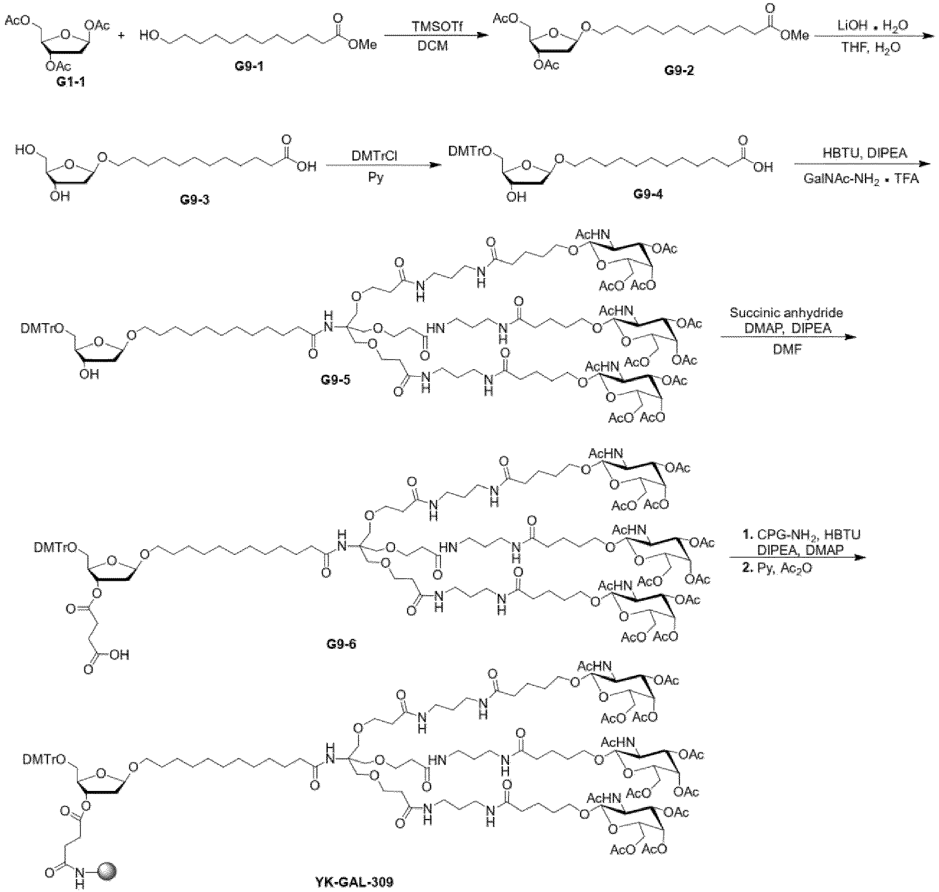

### Step 1: Synthesis of G9-2

A mixture of the dried compound **G1-1** (734 mg, 2.82 mmol), the dried compound **G9-1** (0.50 g, 2.17 mmol) and 3A molecular sieve (2.0 g) was added with dichloromethane (20 mL), cooled to 0°C, added with boron trifluoride etherate (616 mg, 4.34 mmol), and stirred for 2 h. After quenched by adding aqueous solution of saturated sodium bicarbonate (20 mL), the reaction solution was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a yellow oil **G9-2** (589 mg, 1.37 mmol) with a yield of 48.5%. MS (ESI) m/z [M + Na]⁺= 453.1.

### Step 2: Synthesis of G9-3

**G9-2** (0.30 g, 696 µmol) was dissolved in tetrahydrofuran (4.0 mL), added with 2.0 mL of aqueous solution of lithium hydroxide monohydrate (93.6 mg, 2.23 mmol), and stirred at 15°C for 12 h. The reaction solution was concentrated under reduced pressure to remove the solvent to obtain a yellow solid. The crude product **G9-3** (0.23 g) was used directly in the next step. MS (ESI) m/z [M + Na]⁺ = 355.0.

### Step 3: Synthesis of G9-4

The crude product **G9-3** (0.23 g, 692 µmol) and anhydrous pyridine (20 mL x 3) were co-rotary evaporated to remove water. The dried **G9-3** and 4,4'-dimethoxytriphenylmethane chloride (534.58 mg, 1579.8 µmol) were dissolved in pyridine (5.0 mL) and stirred at 15°C for 28 h under nitrogen protection. Methanol (10 mL) was added to quench the reaction, and the solvent was removed by concentration under reduced pressure to obtain a crude product, which was purified by preparative chromatography (H₂O (10 mM NH₄HCO₃)-ACN) to obtain a white solid G9-4 (337.3 mg, 531.7 µmol) with a yield of 76.84%. MS (ESI) m/z [M - H]⁻ = 633.5.

### Step 4: Synthesis of G9-5

**G9-4** (30 mg, 47.3 µmol), diisopropylethylamine (12.2 mg, 94.5 µmol) and O-benzotriazole-tetramethyluronium hexafluorophosphate (26.9 mg, 70.9 µmol) were dissolved in N,N-dimethylformamide (0.5 mL), added with GalNAc-NH₂.TFA (90.2 mg, 47.3 µmol, TFA) and stirred at 15°C for 1 h under nitrogen protection. The reaction solution was used directly in the next step. MS (ESI) m/z [M - 2H]²⁻= 1204.3.

### Step 5: Synthesis of G9-6

**G9-5** (114 mg, 47.3 µmol), 4-dimethylaminopyridine (11.55 mg, 94.58 µmol), diisopropylethylamine (55.1 mg, 425.45 µmol), and succinic anhydride (52.1 mg, 519 µmol) were dissolved in N,N-dimethylformamide (2.0 mL) and stirred at 30°C for 36 h under nitrogen protection. Purification was performed by preparative chromatography (H₂O (10 mM TEAB)-ACN) to obtain a white solid **G9-6** (56.7 mg, 22.6 µmol) with a yield of 47.8%. ¹HNMR (400 MHz, CDCl₃) *δ* ppm 7.44 - 7.38 (m, 2H), 7.33 - 7.26 (m, 6H), 7.24 - 7.17 (m, 1H), 6.86 - 6.79 (m, 4H), 5.24 (d, *J* = 4.3 Hz, 1H), 4.27 - 4.21 (m, 1H), 4.19 (d, *J* = 5.6 Hz, 1H), 3.79 (s, 6H), 3.74 (dd, *J =* 9.6, 6.8 Hz, 1H), 3.40 (dt, *J =* 9.6, 6.5 Hz, 2H), 3.27 (s, 1H), 3.16 - 3.07 (m, 3H), 2.33 (t, *J =* 7.4 Hz, 2H), 2.21 - 2.15 (m, 1H), 2.02 (d, *J* = 13.3 Hz, 1H), 1.67 - 1.52 (m, 4H), 1.23 - 1.30 (d, *J =* 13.9 Hz, 13H). MS (ESI) m/z [M - 2H]²⁻ = 1254.4.

### Step 6: Synthesis of YK-GAL-309

**G9-6** (32 mg, 12.7 µmol), 4-dimethylaminopyridine (1.56 mg, 12.7 µmol), diisopropylethylamine (13.2 mg, 102 µmol), and O-benzotriazole-tetramethyluronium hexafluorophosphate (24.2 mg, 63.7 µmol) were dissolved in N,N-dimethylformamide (4.0 mL), added with CPG-NH₂ (221 mg) and stirred at 40°C for 12 h. The reaction solution was filtered, and the filtrate was washed with methanol followed by dichloromethane and dried in vacuum. The filtrate was then added to 4 mL of acetic anhydride/pyridine (1:5) solution and stirred at 40°C for 0.5 h. The mixture was filtered, washed with dichloromethane followed by methanol, and the filtrate was dried in vacuum for 12 h to obtain a white solid compound **YK-GAL-309** (103 mg, loading 24.5 µmol/g).

### 10. Synthesis of YK-GAL-310

The synthetic route is as follows:

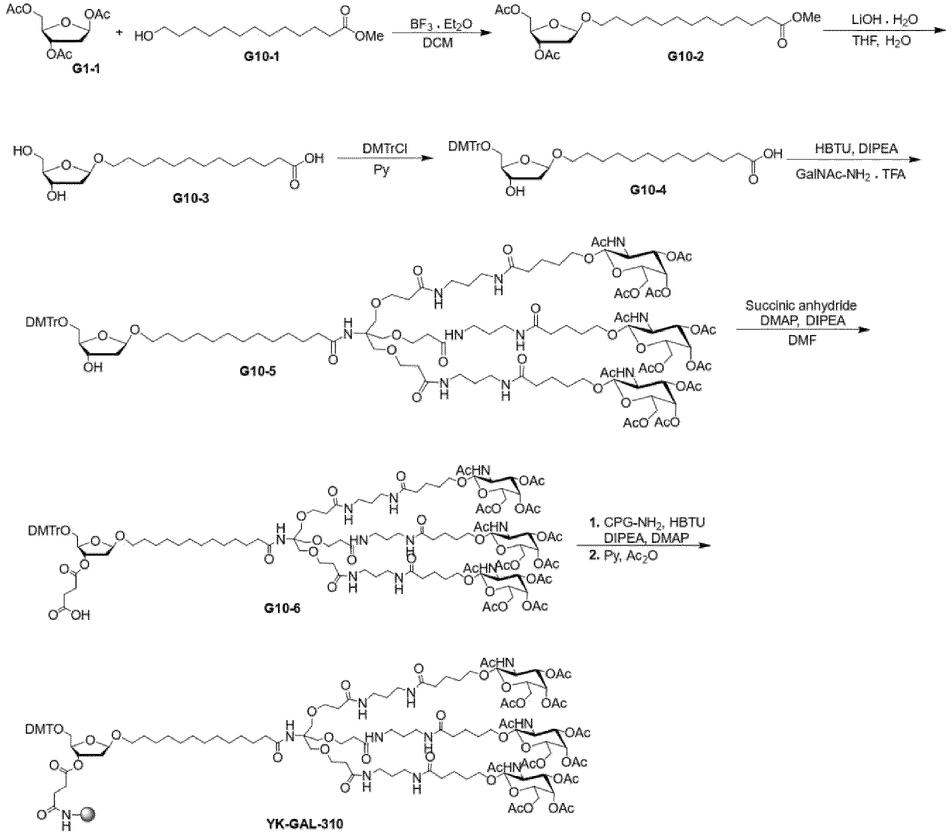

### Step 1: Synthesis of G10-2

A mixture of the dried compound **G1-1** (692 mg, 2.66 mmol), the dried compound **G10-1** (500 mg, 2.05 mmol) and 3A molecular sieve (2.0 g) was added with dichloromethane (20 mL), cooled to 0°C, added with boron trifluoride etherate (581 mg, 4.09 mmol), heated to 25°C and stirred for 2 h. After quenched by adding aqueous solution of saturated sodium bicarbonate (20 mL), the reaction solution was extracted with dichloromethane (10 mL × 2). The organic phase was dried over anhydrous sodium sulfate and concentrated under reduced pressure to remove the solvent to obtain a crude product, which was purified by column chromatography (PE/EA) to obtain a colorless oil **G10-2** (576.6 mg, 1.30 mmol) with a yield of 48.8%. MS (ESI) m/z [M + Na]⁺= 467.1.

### Step 2: Synthesis of G10-3

**G10-2** (69 mg, 155 µmol) was dissolved in tetrahydrofuran (2.0 mL), added with 1.0 mL of aqueous solution of lithium hydroxide monohydrate (20.8 mg, 497 µmol), and stirred at room temperature for 16 h. The reaction solution was concentrated under reduced pressure to remove the solvent to obtain a yellow oil. The crude product **G10-3** (53.8 mg) was used directly in the next step. MS (ESI) m/z [M + Na]⁺= 369.0.

### Step 3: Synthesis of G10-4

The crude product **G10-3** (53.8 mg, 155 µmol) and anhydrous pyridine (20 mL × 3) were co-rotary evaporated to remove water. The dried **G10-3** and 4,4'-dimethoxytriphenylmethane chloride (189 mg, 559 µmol) were dissolved in pyridine (3.0 mL) and stirred at room temperature for 16 h under nitrogen protection. Methanol (10 mL) was added to quench the reaction, and the solvent was removed by concentration under reduced pressure to obtain a crude product, which was purified by column chromatography (DCM/MeOH) to obtain a light yellow solid **G10-4** (79.1 mg, 122 µmol) with a yield of 78.7%. MS (ESI) m/z [M - H]⁻ = 647.5.

### Step 4: Synthesis of G10-5

**G10-4** (39 mg, 60.1 µmol), diisopropylethylamine (15.5 mg, 120 µmol) and O-benzotriazole-tetramethyluronium hexafluorophosphate (34.2 mg, 90.2 µmol) were dissolved in N,N-dimethylformamide (1.5 mL), added with GalNAc-NH₂.TFA (115 mg, 60.1 µmol) and stirred at room temperature for 1 h under nitrogen protection. The reaction solution was directly used in the next step. MS (ESI) m/z [M - 2H]²⁻ = 1211.3.

### Step 5: Synthesis of G10-6

**G10-5** (146 mg, 60.1 µmol), 4-dimethylaminopyridine (7.34 mg, 60.1 µmol), diisopropylethylamine (31.1 mg, 240 µmol), and succinic anhydride (30.1 mg, 301 µmol) were dissolved in N,N-dimethylformamide (1.5 mL) and stirred at 30°C for 16 h under nitrogen protection. Purification was performed by preparative chromatography (H₂O (10 mM TEAB)-ACN) to obtain a white solid **G10-6** (74.2 mg, 49.4 µmol) with a yield of 48.9%. ¹HNMR (400 MHz, CDCl₃) *δ* ppm 7.49 - 7.42 (m, 2H), 7.36 - 7.31 (m, 4H), 7.28 - 7.23 (m, 8H), 7.21 - 7.16 (m, 1H), 6.99 (t, *J =* 6.0 Hz, 3H), 6.79 (dd, *J =* 11.8, 8.8 Hz, 6H), 6.47 (d, *J =* 9.6 Hz, 1H), 5.35 (d, *J =* 3.3 Hz, 2H), 5.23 - 5.17 (dt, *J =* 11.2, 3.8 Hz, 4H), 4.61 (d, *J =* 8.5 Hz, 2H), 4.19 - 4.04 (m, 9H), 3.99 - 3.86 (m, 6H), 3.78 (d, *J =* 2.1 Hz, 6H), 3.69 - 3.60 (m, 11H), 3.53 - 3.48 (m, 3H), 3.35 - 3.14 (m, 15H), 2.60 - 2.50 (m, 4H), 2.43 (t, *J =* 5.7 Hz, 7H), 2.29 - 2.13 (m, 24H), 2.10 (d, *J =* 5.0 Hz, 3H), 2.04 (s, 9H), 1.99 (s, 9H), 1.94 (d, *J =* 2.5 Hz, 8H), 1.75 - 1.55 (m, 18H), 1.42 (s, 2H), 1.26 - 1.21 (d, *J =* 3.6 Hz, 15H). MS (ESI) m/z [M - 2H]²⁻ = 1261.1.

### Step 6: Synthesis of YK-GAL-310

**G10-6** (59 mg, 22.1 µmol), 4-dimethylaminopyridine (2.70 mg, 22.13 µmol), diisopropylethylamine (22.88 mg, 177.01 µmol), and O-benzotriazole-tetramethyluronium hexafluorophosphate (41.96 mg, 110.63 µmol) were dissolved in N,N-dimethylformamide (4.0 mL), then added with CPG-NH₂ (383 mg) and stirred at 40°C for 16 h. The reaction solution was filtered, and the filtrate was washed with methanol followed by dichloromethane and dried in vacuum. The filtrate was then added to 4 mL of acetic anhydride/pyridine (1:4) solution and stirred at 40°C for 0.5 h. The mixture was filtered, washed with dichloromethane followed by methanol, and the filtrate was dried under vacuum for 12 h to obtain a white solid compound **YK-GAL-310** (362 mg, loading 30.2 µmol/g).

### 11. Synthesis of YK-GAL-311

The synthetic route is as follows:

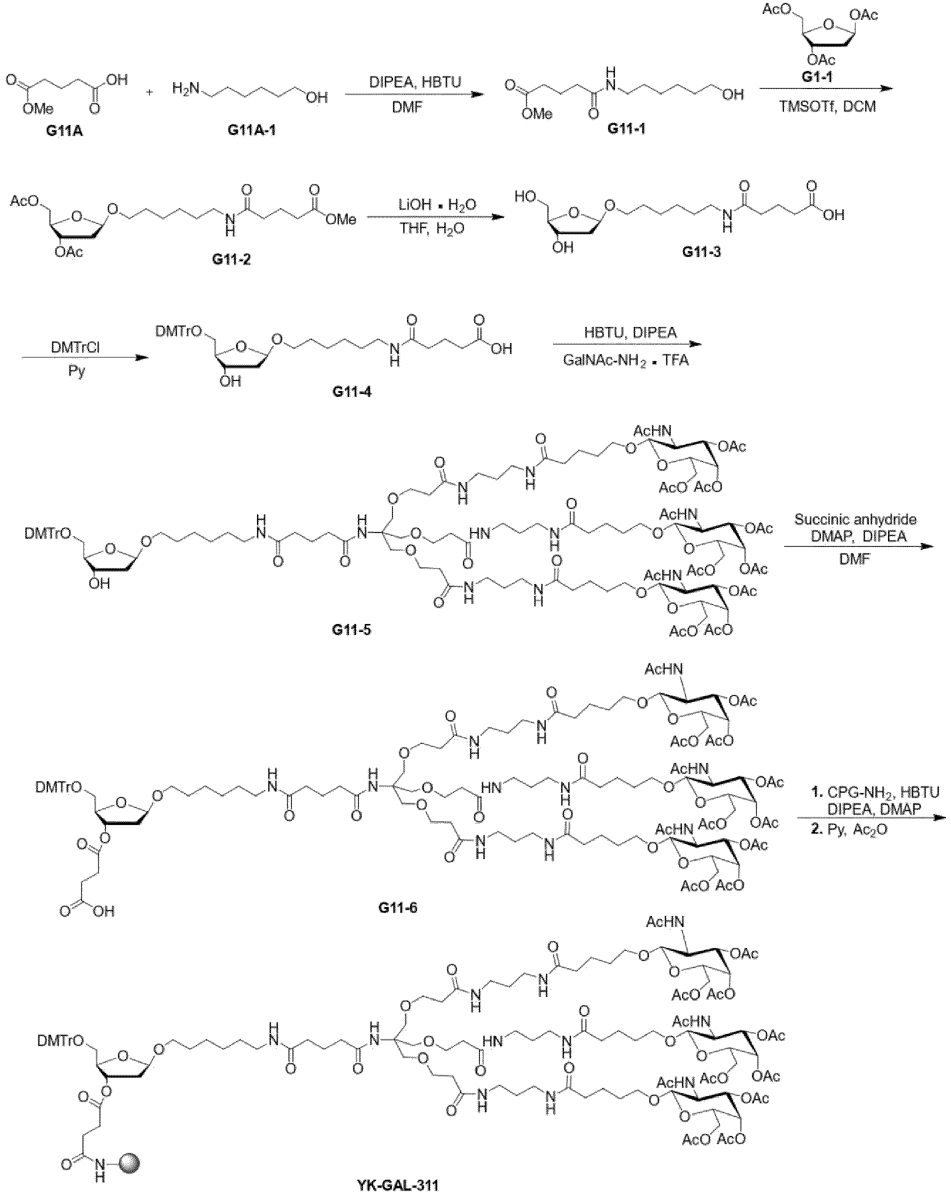

### Step 1: Synthesis of G11-1

**G11A** (2.00 g, 13.7 mmol, 1.72 mL), diisopropylethylamine (3.54 g, 27.3 mmol) and O-benzotriazole-tetramethyluronium hexafluorophosphate (5.71 g, 15.0 mmol) were dissolved in N,N-dimethylformamide (20.0 mL), added with **G11A-1** (1.92 g, 16.4 mmol), and stirred at 15°C for 2 h. Purification was performed by preparative chromatography (H₂O (0.1% TFA) - ACN) to obtain a white solid **G11-1** (2.00 g, 8.15 mmol) with a yield of 59.5%. ¹HNMR (400 MHz, DMSO*-d₆*)*δ* ppm 7.76 - 7.73 (m, 1H), 4.38 - 4.30 (m, 1H), 3.57 (s, 3H), 3.36 (t, *J =* 6.4 Hz, 2H), 3.02 - 2.97 (m, 2H), 2.28 (t, *J =* 7.6 Hz, 2H), 2.69 (t, *J =* 7.2 Hz, 2H), 1.76 - 1.68 (m, 2H), 1.42 - 1.18 (m, 8H). MS (ESI) m/z [M + H]⁺= 245.9.

### Step 2: Synthesis of G11-2

A mixture of compound G1-1 (500 mg, 1.92 mmol), compound **G11-1** (565 mg, 2.31 mmol) and 4A molecular sieve (1.0 g) was added with dichloromethane (10.0 mL), cooled to 0°C, added with trimethylsilyl trifluoromethanesulfonate (854 mg, 3.84 mmol), and stirred for 2 h. After quenched by adding aqueous solution of saturated sodium bicarbonate (30.0 mL), the reaction solution was extracted with dichloromethane (15.0 mL × 3). The organic phase was dried over anhydrous sodium sulfate and concentrated under reduced pressure to remove the solvent to obtain a crude product, which was purified by column chromatography (PE/EA) to obtain a colorless oil **G11-2** (400 mg, 899 µmol) with a yield of 46.8%. ¹HNMR (400 MHz, CDCl₃) *δ* ppm 5.61 - 5.54 (m, 1H), 5.24 - 5.20 (m, 1H), 4.32 - 4.00 (m, 3H), 3.67 (d, *J =* 1.0 Hz, 4H), 3.34 (dt, *J* = 9.4, 6.6 Hz, 1H), 3.27 - 3.19 (m, 2H), 2.42 - 2.30 (m, 3H), 2.27 - 2.02 (m, 9H), 2.02 - 1.90 (m, 3H), 1.57 - 1.45 (m, 4H), 1.37 - 1.31 (m, 4H). MS (ESI) m/z [M + H]⁺= 446.0.

### Step 3: Synthesis of G11-3

**G11-2** (180 mg, 404 µmol, 1.0 eq) was dissolved in tetrahydrofuran (1.8 mL), added with 0.9 mL of aqueous solution of lithium hydroxide monohydrate (67.8 mg, 1.62 mmol, 4.0 eq), and stirred at 35°C for 16 h. The reaction solution was concentrated under reduced pressure to remove the solvent to obtain a brown oil. The crude product **G11-3** (140.36 mg) was used directly in the next step.

### Step 4: Synthesis of G11-4

Dried **G11-3** (40.0 mg, 115 µmol) and 4,4'-dimethoxytriphenylmethane chloride (65.0 mg, 191 µmol) were dissolved in pyridine (1.00 mL) and stirred at 15°C for 8 h under nitrogen protection. Methanol (2.00 mL) was added to quench the reaction, and the solvent was removed by concentration under reduced pressure to obtain a crude product, which was purified by preparative chromatography (H₂O (10 mM TEAB)-ACN) to obtain a white solid **G11-4** (54.7 mg, 84.3 µmol) with a yield of 73.3%. ¹HNMR (400 MHz, CDCl₃) *δ* ppm 7.38 (d, *J =* 7.7 Hz, 2H), 7.29 - 7.20 (m, 6H), 7.13 (t, *J =* 7.3 Hz, 1H), 6.75 (d, *J =* 8.6 Hz, 4H), 5.60 (s, 1H), 5.08 (dd, *J =* 5.5, 2.3 Hz, 1H), 4.34 (q, *J =* 6.2 Hz, 1H), 3.89 (q, *J =* 5.6 Hz, 1H), 3.72 (s, 6H), 3.56 - 3.50 (m, 1H), 3.29 - 3.17 (m, 3H), 3.16 - 3.06 (m, 3H), 2.33 (d, *J =* 7.0 Hz, 3H), 2.18 (t, *J =* 7.2 Hz, 3H), 2.01 - 1.95 (m, 1H), 1.88 (t, *J =* 7.0 Hz, 2H), 1.40 - 1.31 (m, 4H), 1.21 - 1.15 (m, 4H). MS (ESI) m/z [M - H]⁻ = 648.2.

### Step 5: Synthesis of G11-5

**G11-4** (17.0 mg, 26.1 µmol), diisopropylethylamine (6.76 mg, 52.3 µmol) and O-benzotriazole-tetramethyluronium hexafluorophosphate (14.8 mg, 39.2 µmol) were dissolved in N,N-dimethylformamide (0.3 mL), added with GalNAc-NH₂.TFA (62.4 mg, 27.4 µmol, TFA) and stirred at 15°C for 6 h under nitrogen protection. The reaction solution was used directly in the next step. MS (ESI) m/z [M - 2H]²⁻= 1211.6.

### Step 6: Synthesis of G11-6

**G11-5** (63.0 mg, 25.9 µmol), 4-dimethylaminopyridine (6.35 mg, 51.9 µmol), diisopropylethylamine (6.71 mg, 51.9 µmol), and succinic anhydride (13.0 mg, 129 µmol) were dissolved in N,N-dimethylformamide (0.5 mL) and stirred at 30°C for 16 h under nitrogen protection. Purification was performed by preparative chromatography (H₂O (10 mM TEAB)-ACN) to obtain a white solid **G11-6** (35.0 mg, 13.8 µmol) with a yield of 53.3%. ¹HNMR (400 MHz, CDCl₃) *δ* ppm 7.49 - 7.44 (m, 5H), 7.35 - 7.31 (m, 4H), 7.28 (s, 1H), 7.24 (s, 1H), 7.17 (d, *J =* 7.5 Hz, 1H), 7.01 - 7.00 (m, 3H), 6.91 (d, *J* = 9.0 Hz, 2H), 6.83 - 6.72 (m, 6H), 5.35 (d, *J =* 3.3 Hz, 2H), 5.19 (dd, *J* = 10.9, 3.3 Hz, 5H), 4.62 (d, *J =* 8.3 Hz, 3H), 4.18 - 4.05 (m, 10H), 3.95 - 3.89 (m, 6H), 3.78 (s, 7H), 3.67 - 3.61 (m, 12H), 3.50 (s, 4H), 3.31 - 3.15 (m, 17H), 2.57 (d, *J =* 6.3 Hz, 2H), 2.51 (d, *J =* 6.7 Hz, 2H), 2.41 (t, *J =* 5.8 Hz, 6H), 2.28 - 2.12 (m, 21H), 2.12 - 1.84 (m, 34H), 1.75 - 1.61 (m, 20H), 1.25 (s, 4H). MS (ESI) m/z [M - 2H]²⁻ = 1261.9.

### Step 7: Synthesis of YK-GAL-311

**G11-7** (35.0 mg, 13.8 µmol), 4-dimethylaminopyridine (1.69 mg, 13.8 µmol), diisopropylethylamine (14.3 mg, 110 µmol), and O-benzotriazole-tetramethyluronium hexafluorophosphate (26.2 mg, 69.3 µmol) were dissolved in N,N-dimethylformamide (2.0 mL), then added with CPG-NH₂ (220 mg) and stirred at 40°C for 16 h. The reaction solution was filtered, and the filtrate was washed with methanol followed by dichloromethane and dried in vacuum. The filtrate was then added to 12.0 mL of acetic anhydride/pyridine (1:5) solution and stirred at 40°C for 0.5 h. The mixture was filtered, washed with dichloromethane followed by methanol, and the filtrate was dried under vacuum for 12 h to obtain a light yellow solid compound **YK-GAL-311** (205 mg, loading 32.4 µmol/g).

### 12. Synthesis of YK-GAL-312

The synthetic route is as follows:

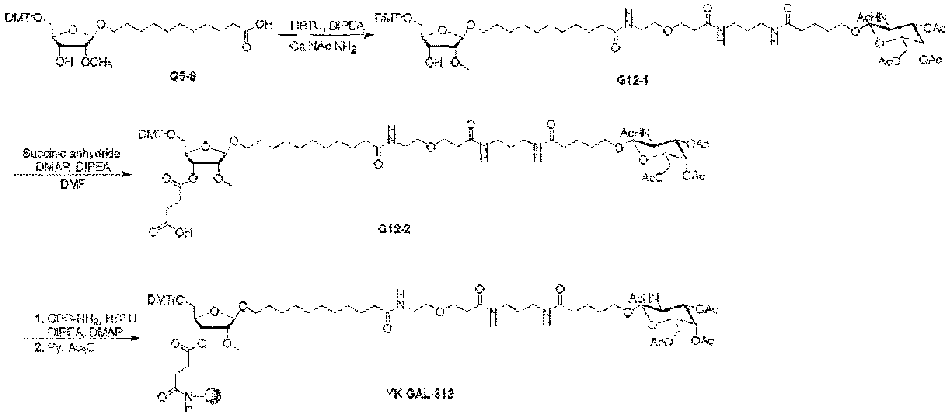

### Step 1: Synthesis of G12-1

**G5-8** (1.3 g, 2.0 mmol), diisopropylethylamine (517 mg, 4.0 mmol) and O-benzotriazole-tetramethyluronium hexafluorophosphate (1.14 g, 3.0 mmol) were dissolved in N,N-dimethylformamide (100.0 mL), added with GalNAc-NH₂ (the compound was prepared according to the synthetic method of patent application CN115315263A, with a total yield of 20.18%) (1.24 g, 2.0 mmol) and stirred at 15°C for 1 h under nitrogen protection. The reaction solution was directly used in the next step. MS (ESI) m/z [M - H]⁻ = 1249.9.

### Step 2: Synthesis of G12-2

**G12-1** (2.0 mmol), 4-dimethylaminopyridine (488.7 mg, 4.0 mmol), diisopropylethylamine (1.03 g, 8.0 mmol), and succinic anhydride (1.2 g, 12.0 mmol) were dissolved in N,N-dimethylformamide (100.0 mL) and stirred at 30°C for 48 h under nitrogen protection. Purification was performed by preparative chromatography (H₂O (50 mM TEAB)-ACN) to obtain a white solid **G12-2** (1.9 g, 1.31 mmol) with a yield of 65.5%. MS (ESI) m/z [M - H]⁻ = 1350.1.

### Step 3: Synthesis of YK-GAL-312

**G12-2** (1.9 g, 1.31 mmol), 4-dimethylaminopyridine (160 mg, 1.31 mmol), diisopropylethylamine (1.35 g, 10.48 mmol), and O-benzotriazole-tetramethyluronium hexafluorophosphate (2.48 g, 6.55 mmol) were dissolved in N,N-dimethylformamide (210 mL), added with CPG-NH₂ (13.6 g) and stirred at 40°C for 16 h. The reaction solution was filtered, and the filtrate was washed with methanol followed by dichloromethane and dried under vacuum. The filtrate was then added to 124 mL of acetic anhydride/pyridine (1:5) solution and stirred at 40°C for 0.5 h. The mixture was filtered, washed with dichloromethane followed by methanol, and the filtrate was dried under vacuum for 12 h to obtain a white solid compound **YK-GAL-312** (24.3 g, loading 36.2 µmol/g).

### 13. Synthesis of YK-GAL-313

The synthetic route is as follows:

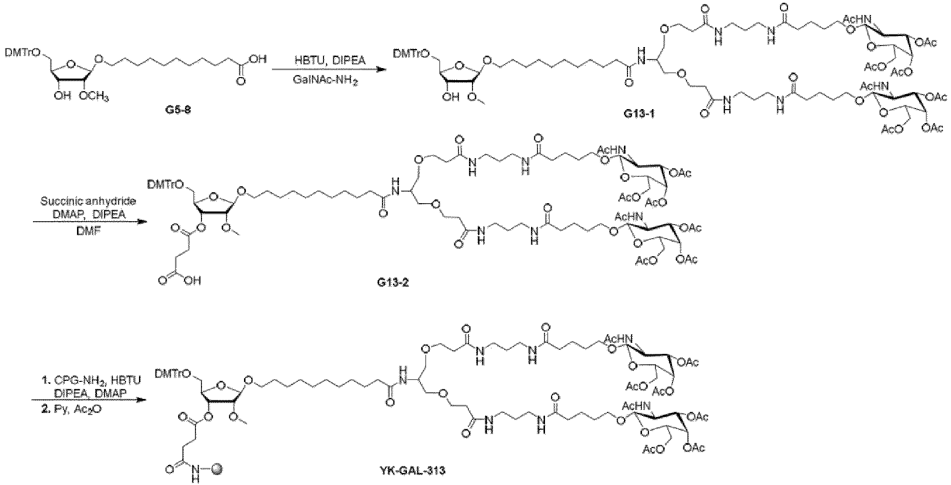

### Step 1: Synthesis of G13-1

G5-8 (1.3 g, 2.0 mmol), diisopropylethylamine (517 mg, 4.0 mmol) and O-benzotriazole-tetramethyluronium hexafluorophosphate (1.14 g, 3.0 mmol) were dissolved in N,N-dimethylformamide (100.0 mL), added with GalNAc-NH₂ (the compound was prepared according to the synthesis method of patent WO 2023288033A1, with a total yield of 2.1%) (2.41 g, 2.0 mmol), and stirred at 15°C for 1 h under nitrogen protection. The reaction solution was directly used in the next step. MS (ESI) m/z [M - H]⁻ = 1837.5.

### Step 2: Synthesis of G13-2

**G13-1** (2.0 mmol), 4-dimethylaminopyridine (488.7 mg, 4.0 mmol), diisopropylethylamine (1.03 g, 8.0 mmol), and succinic anhydride (1.2 g, 12.0 mmol) were dissolved in N,N-dimethylformamide (100.0 mL) and stirred at 30°C for 48 h under nitrogen protection. Purification was performed by preparative chromatography (H₂O (50 mM TEAB)-ACN) to obtain a white solid **G13-2** (2.66 g, 1.29 mmol) with a yield of 64.5%. MS (ESI) m/z [M - H]⁻= 1937.2.

### Step 3: Synthesis of YK-GAL-313

**G13-2** (2.66 g, 1.29 mmol), 4-dimethylaminopyridine (157.6 mg, 1.29 mmol), diisopropylethylamine (1.33 g, 10.32 mmol), and O-benzotriazole-tetramethyluronium hexafluorophosphate (2.45 g, 6.45 mmol) were dissolved in N,N-dimethylformamide (210.0 mL), then added with CPG-NH₂ (18.9 g) and stirred at 40°C for 16 h. The reaction solution was filtered, and the filtrate was washed with methanol followed by dichloromethane and dried in vacuum. The filtrate was then added to 173 mL of acetic anhydride/pyridine (1:5) solution and stirred at 40°C for 0.5 h. The mixture was filtered, washed with dichloromethane followed by methanol, and the filtrate was dried under vacuum for 12 h to obtain a white solid compound **YK-GAL-313** (26.3 g, loading 37.1 µmol/g).

### 14. Synthesis of NAG0052

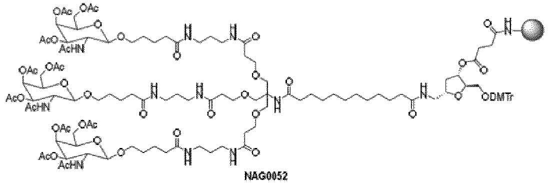

According to the synthesis method of NAG0052 on page 100 of WO2023109938A1, 288 mg of product was obtained with a total yield of 1.26% and a loading of 31.5 µmol/g.

### Example 2: Synthesis of GalNAc phosphoramidite compounds

### 1. Synthesis of YK-GAL-318

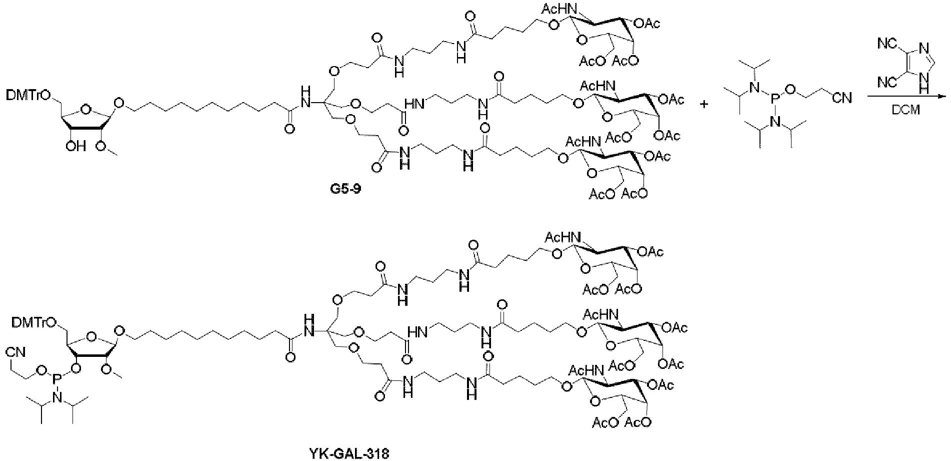

Compound **G5-9** (2.43 g, 1.0 mmol) and DCM (25 mL) were added to a reaction flask, stirred until dissolved, added with 3-bis(diisopropylamino) phosphinoyloxypropionitrile (0.75 g, 2.5 mmol) and 4,5-dicyanoimidazole (0.24 g, 2.0 mmol) in sequence, and stirred at 10°C for 2 h. The reaction solution was diluted with DCM (100 mL) and washed twice with NaHCO₃ (100 mL). The organic phase was dried over anhydrous Na₂SO₄ and concentrated to remove the solvent to obtain a residue. The residue was dissolved in DCM (30 mL) and slurried with n-heptane/methyl tert-butyl ether (5/1) at 10-15°C for 10 min, 3 times in total, to obtain 2.39 g of a white solid with a yield of 91%. MS (ESI) m/z [M + H]⁺= 2626.8.

### 2. Synthesis of other GalNAc phosphoramidite compounds

Using **G1-6, G2-5, G3-5, G4-5, G6-8, G7-8, G8-4, G9-5, G10-5, G11-5, G12-1** and **G13-1** as starting materials, other GalNAc phosphoramidite compounds listed in Table 1 were synthesized according to the method for synthesizing YK-GAL-318.

**Table 1: GalNAc phosphoramidite compounds**

| Name | Compound structure | yield (%) | **MS (ESI) m/z** [M + H]⁺ |
|---|---|---|---|
| **YK-GAL -314** | 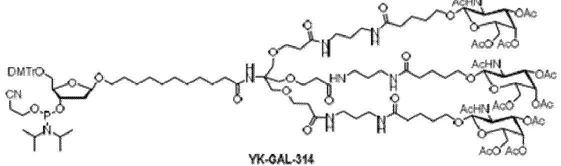 | 90 | 2596.8 |
| **YK-GAL -315** | 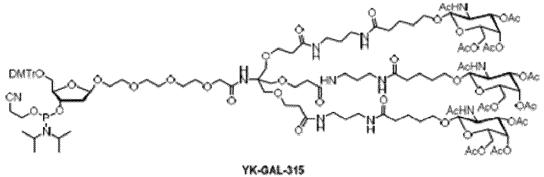 | 88 | 2602.9 |
| **YK-GAL -316** | 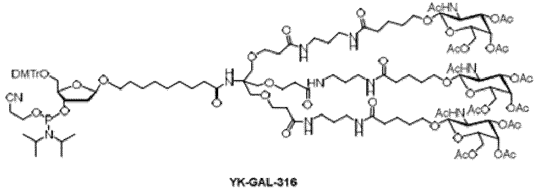 | 91 | 2569.1 |
| **YK-GAL -317** | 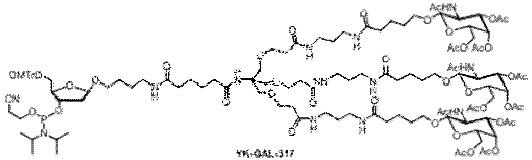 | 85 | 2612.1 |
| **YK-GAL -318** | 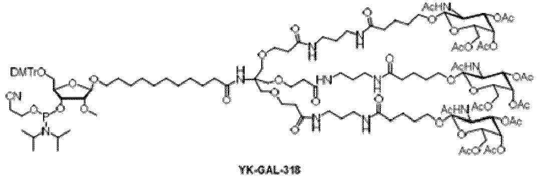 | 91 | 2626.8 |
| **YK-GAL -319** | 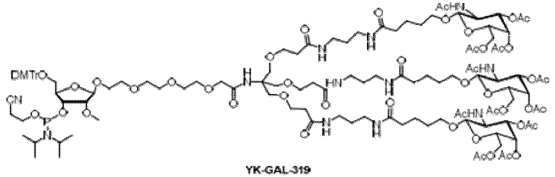 | 87 | 2632.9 |
| **YK-GAL -320** | 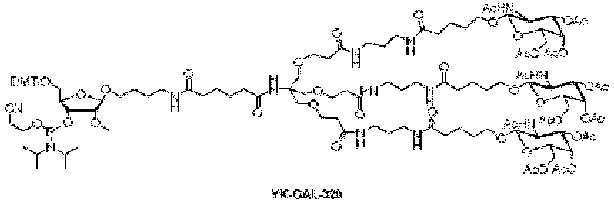 | 85 | 2642.4 |
| **YK-GAL -321** | 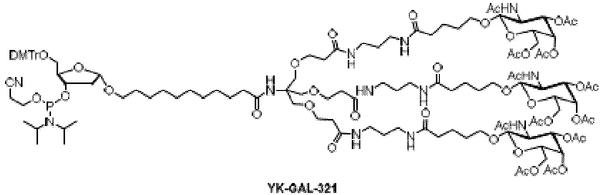 | 81 | 2597.0 |
| **YK-GAL -322** | 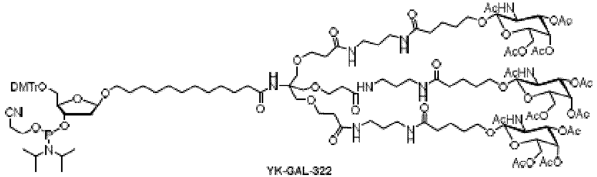 | 88 | 2611.2 |
| **YK-GAL -323** | 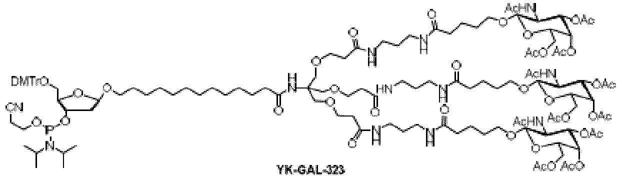 | 85 | 2624.9 |
| **YK-GAL -324** | 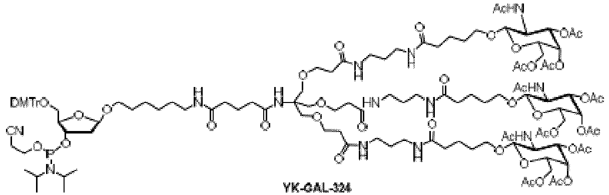 | 82 | 2625.8 |
| **YK-GAL -325** | 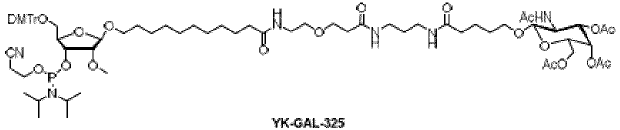 | 90 | 1452.2 |
| **YK-GAL -326** | 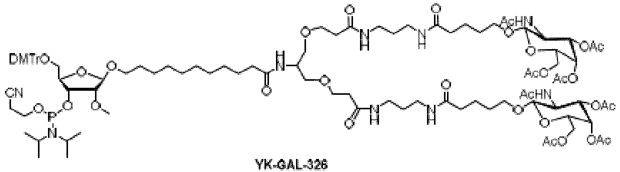 | 86 | 2039.8 |

### 3. Synthesis of GalNAc 1b

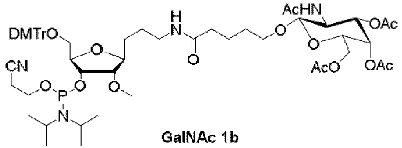

According to the synthesis method of GalNAc 1b on page 145 of WO2023014938A1, 377.2 mg of product was obtained with a total yield of 0.58%. MS (ESI) m/z [M + H]⁺= 1137.9.

### 4. Synthesis of GalNAc 2

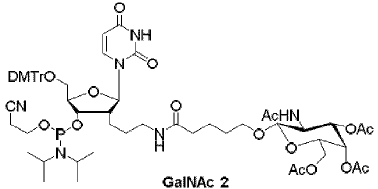

According to the synthesis method of GalNAc 2 on page 216 of WO2023049258A1, 468.5 mg of product was obtained with a total yield of 1.75%. MS (ESI) m/z [M + H]⁺= 1217.7.

### Example 3: Coupling of GalNAc compounds to oligonucleotides

In this example, the same siRNA sequence was used for synthesis, and the conjugated siRNA sequence was a sequence numbered as inc. The inc sequence is shown as follows:
Sense strand (inc-SS):
   5'-Cms-Ums-Am-Gm-Am-Cm-Cf-Um-Gf-Um-dT-Um-Um-Gm-Cm-Um-Um-Um-Um-Gm-Um-3' (SEQ ID NO:1),
Antisense strand (inc-AS):
   5'-Ams-Cfs-Am-Af-Af-Af-Gm-Cf-Am-Af-Am-Af-Cm-Af-Gm-Gf-Um-Cf-Um-Am-Gms-Ams-A m-3' (SEQ ID NO:2).

Wherein, A, U, C, and G represent the base composition of the nucleotide; dT represents deoxythymidine nucleotide; m represents that the nucleotide adjacent to the left of m is 2'-OMe modified; f represents that the nucleotide adjacent to the left of m is 2'-F modified; s represents that the two nucleotides adjacent to the left and right of s are connected by thiophosphate groups.

The sequence is publicly available and is the oligonucleotide sequence of the siRNA drug inclisiran that has been marketed overseas.

The inclisiran sequence is a synthetic, chemically modified double-stranded small interfering ribonucleic acid (siRNA) that targets and binds to the mRNA encoding the PCSK9 protein, inhibits the production of the PCSK9 protein through the RNA interference mechanism, and regulates the recycling and reuse of the LDL receptor and enhancing its binding with LDL, thereby achieving the purpose of lowering LDL in the blood.

### 1. Preparation of GalNAc-conjugated siRNA sense strand

Oligonucleotide GalNAc conjugates were synthesized on a solid phase support according to phosphoramidite chemical method.

Conjugates 1-11 and 18 (sequence numbers: inc-G1, inc-G2, inc-G3, inc-G4, inc-G5, inc-G6, inc-G7, inc-G8, inc-G9, inc-G10, inc-G11 and inc-NAG0052) were synthesized using the GalNAc-CPG compounds synthesized in Example 1 (including **YK-GAL-301, YK-GAL-302, YK-GAL-303, YK-GAL-304, YK-GAL-305, YK-GAL-306, YK-GAL-307, YK-GAL-308, YK-GAL-309, YK-GAL-310, YK-GAL-311 and NAG0052)** as solid phase supports. Conjugate 17 (sequence number inc-L96) was synthesized using CPG-L96 (purchased from Tianjin WuXi AppTec New Drug Development Co., Ltd., L96 is disclosed in claim 10, US 10465194B2) as a solid phase support, whose synthesis scale was 1 µmol. All of these GalNAc compounds were conjugated to the 3' end of the oligonucleotide.

Conjugates 12-16, 19 and 20 (sequence numbers: inc-G12, inc-G13, G18-inc, inc-G25, G26-inc, inc-GalNAc 1b and inc-GalNAc 2) were synthesized using a universal CPG solid phase support, and the GalNAc phosphoramidite compounds synthesized in Example 2 **(YK-GAL-318, YK-GAL-325, YK-GAL-326, inc-GalNAc 1b and inc-GalNAc 2**) were used as the first monomer or the last monomer for solid phase synthesis, wherein the solid phase support synthesis scale was 1 µmol. In conjugates 12 (sequence number inc-G12), 13 (sequence number inc-G13), 15 (sequence number inc-G25), 19 (sequence number inc-GalNAc 1b) and 20 (sequence number inc-GalNAc 2), GalNAc ligand compounds were conjugated to the 3' end of the oligonucleotide, and in conjugates 14 (sequence number G18-inc) and 16 (sequence number G26-inc), GalNAc ligand compounds were conjugated to the 5' end of the oligonucleotide.

### (1) Preparation of reagents and monomers

The monomer solution in acetonitrile (1/20, w/v) and 0.25 M 5-benzylthiotetrazolyl solution in acetonitrile were used as an activating agent. 0.2 M hydrogenated xanthanum solution in acetonitrile/pyridine (1/4, v/v) was used as a thiolation agent. 0.05M iodine solution in water/pyridine (1/9, v/v) was used as an oxidizing agent. 20% acetic anhydride in acetonitrile (v/v) was used as a capping agent A. 20/30/50 (1-methylimidazole/pyridine/acetonitrile, v/v/v) was used as a capping agent B. 20% diethylamine in acetonitrile (v/v) was used as a decyanoethylation agent. 3% dichloroacetic acid in toluene (v/v) was used as a de-DMT agent. The reagents were loaded into the designated reagent position of the 192 P DNA/RNA automatic synthesizer.

### (2) Crude product synthesis

The designated oligonucleotide sequence was input and the synthesis program was set. After a check, the cyclic oligonucleotide synthesis was started. The monomer coupling time was about 1 minute, of which the oxygenation time was about 30-45 seconds, and the thiolation time was about 2 minutes. Once the cycle was completed, the solid phase synthesis of the oligonucleotide was completed.

### (3) Deprotection

After the synthesis was completed, the solid phase support was transferred to a reactor, subjected to concentrated ammonia (25-28%) at 50-60°C for 16-24 h to cleave the oligonucleotide from the solid phase support, cooled to room temperature, filtered, and rinsed with a mixed solution of purified water and ethanol. The filtrate was combined and concentrated at low temperature to obtain a crude residue.

### (4) Purification

The crude residue after deprotection was dissolved in purified water and purified by HPLC. The product peak solution was collected. The content was measured by an enzyme marker, and the molecular weight was confirmed by ESI MS.

In this step, the GalNAc-CPG compounds synthesized in Example 1 and the GalNAc phosphoramidite compounds synthesized in Example 2 were conjugated to the 3' or 5' end of the sense strand of the siRNA.

Conjugate 12 was synthesized using **YK-GAL-325** as the phosphoramidite monomer. It was repeated three times during the sequence synthesis since **YK-GAL-325** was monoantennary, so that the synthesized conjugate 12 had three GalNAc modification groups.

Conjugate 13 was synthesized using **YK-GAL-326** as the phosphoramidite monomer. It was repeated twice during the sequence synthesis since **YK-GAL-326** was biantennary, so that the synthesized conjugate 13 had four GalNAc modification groups.

### 2. Preparation of siRNA antisense strand without GalNAc conjugation

The siRNA antisense strand was synthesized according to the method for synthesizing the siRNA sense strand, wherein a universal CPG solid phase carrier was used, and the synthesis each antisense strand complementary to the sense strand was at 1 µmol.

### 3. Preparation of conjugated double-stranded siRNA

The siRNA sense strand and the complementary antisense strand were mixed in a ratio of 1:1 according to the ultraviolet absorption content, heated to 95°C, and cooled to room temperature after 3 min to form a double strand. The obtained double-stranded siRNA solution was characterized by HPLC and the content was determined by an ELISA instrument after the product purity was qualified, and lyophilized to obtain a solid powder for storage. The obtained GalNAc-conjugated siRNA double-stranded sequence and molecular weight are shown in Table 2.

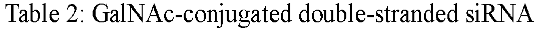

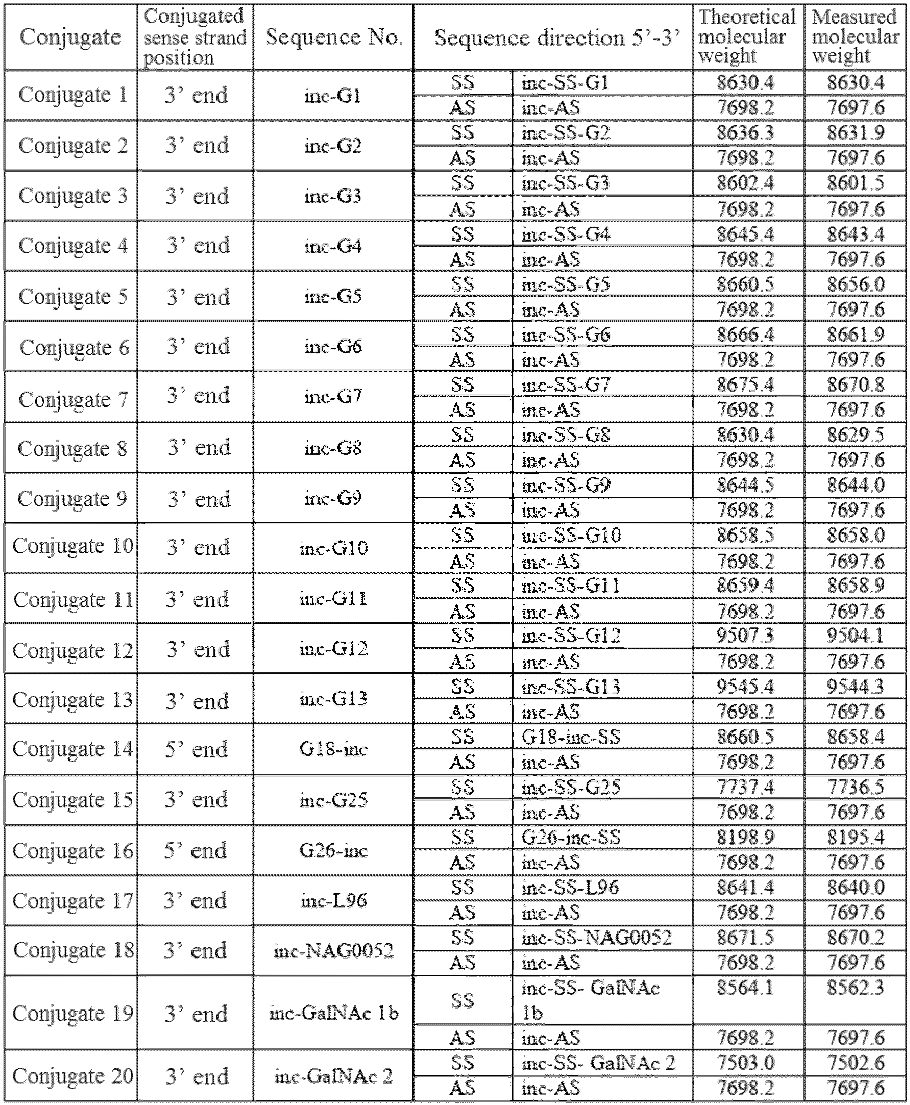

wherein: SS represents sense strand, AS represents antisense strand, and the resulting conjugated siRNA structure is as follows: 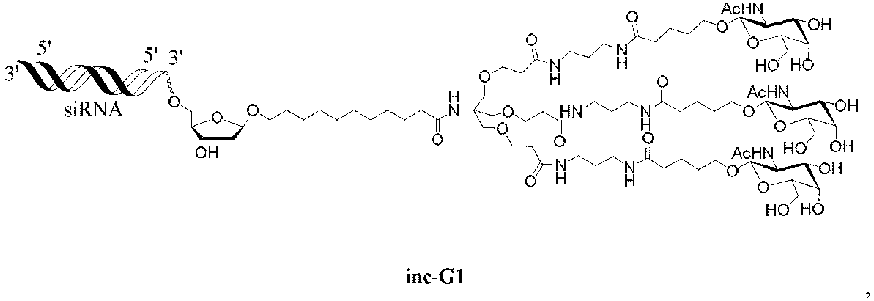 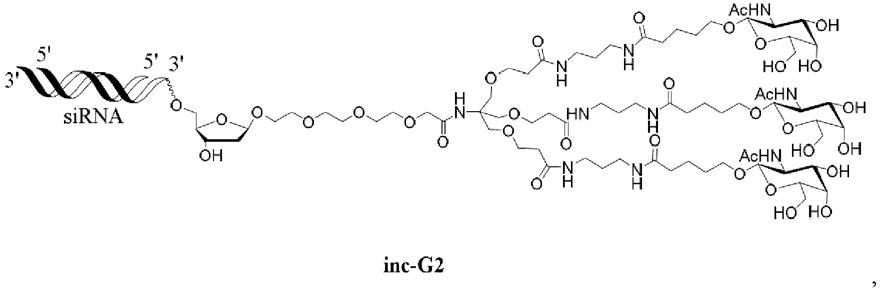 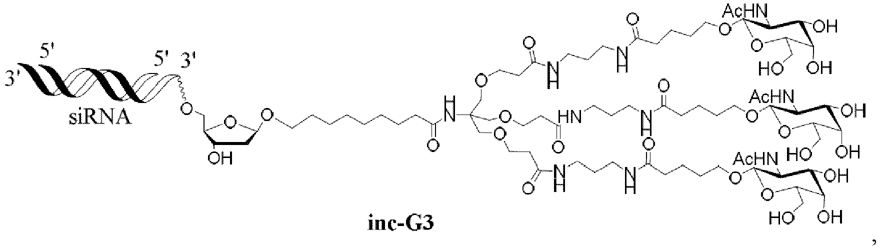 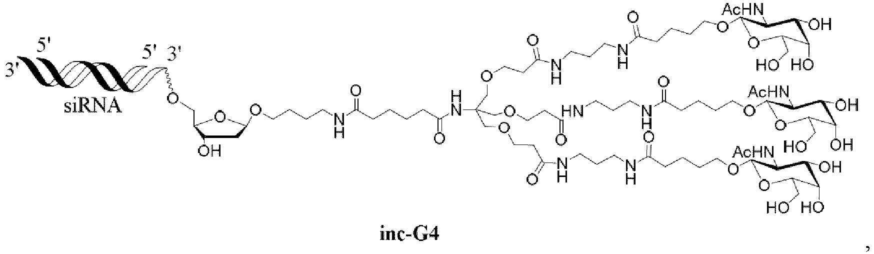 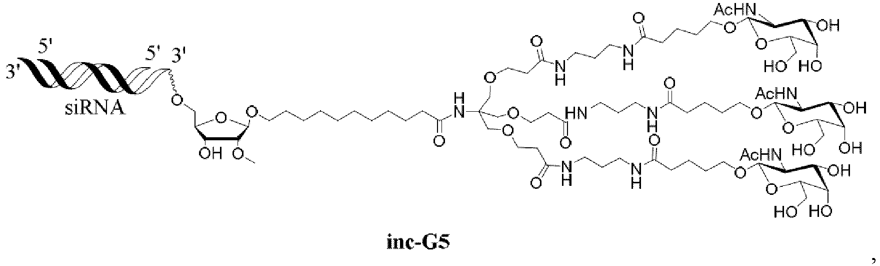 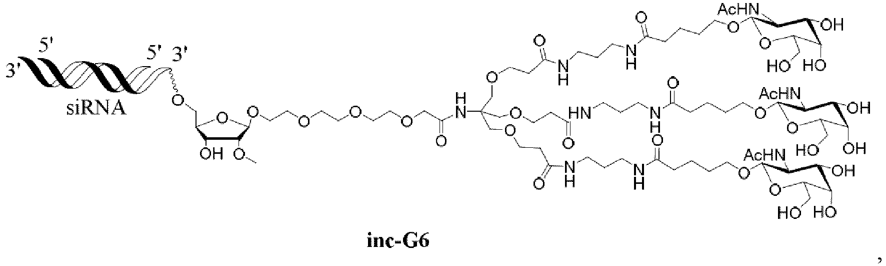 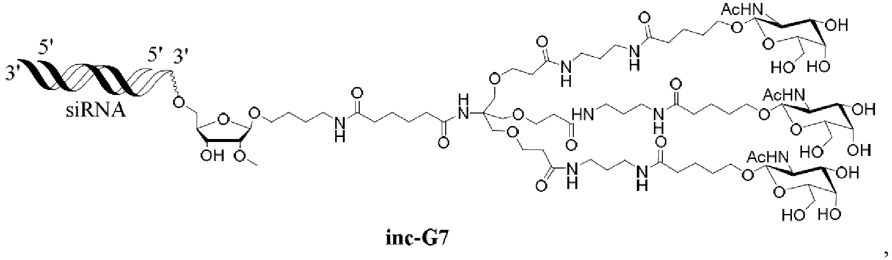 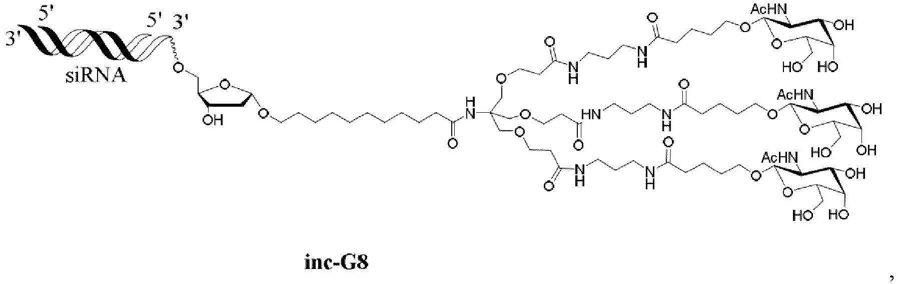 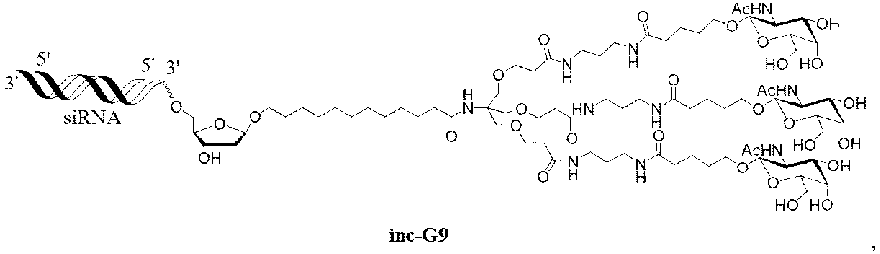 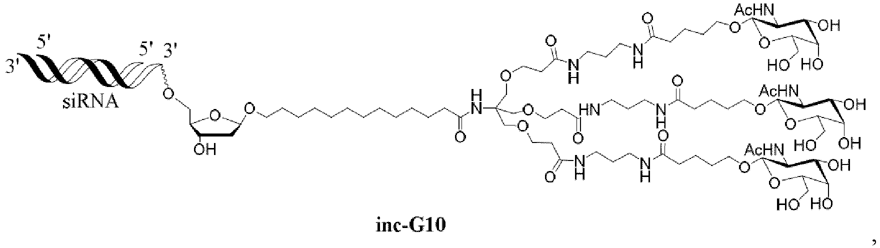 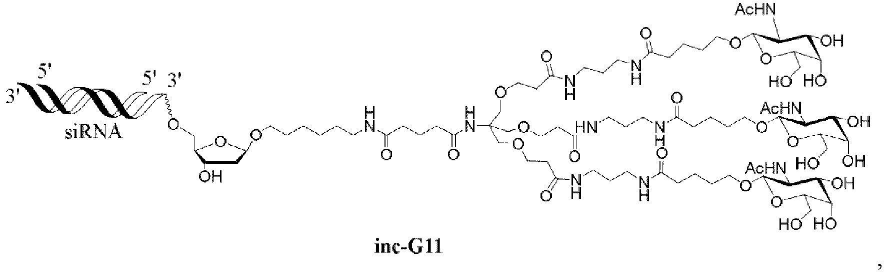 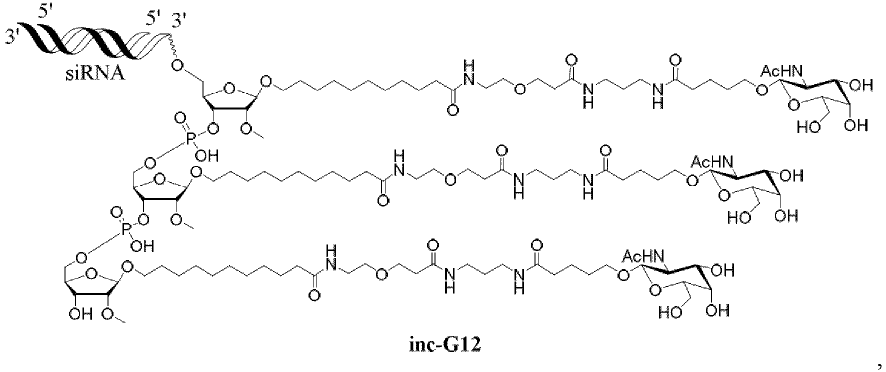 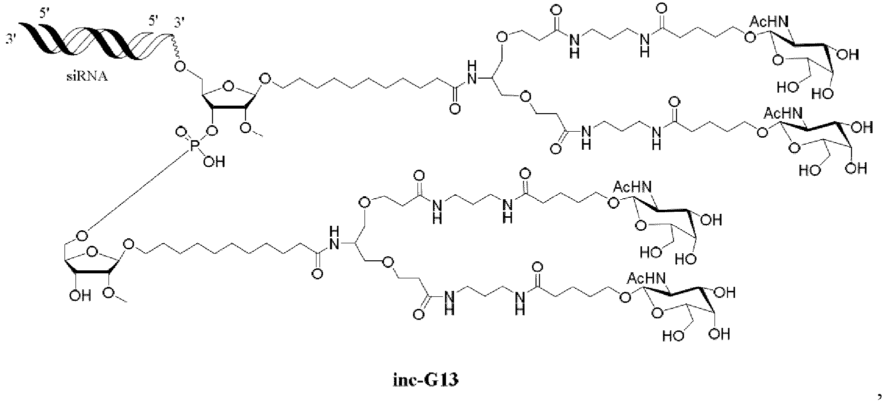 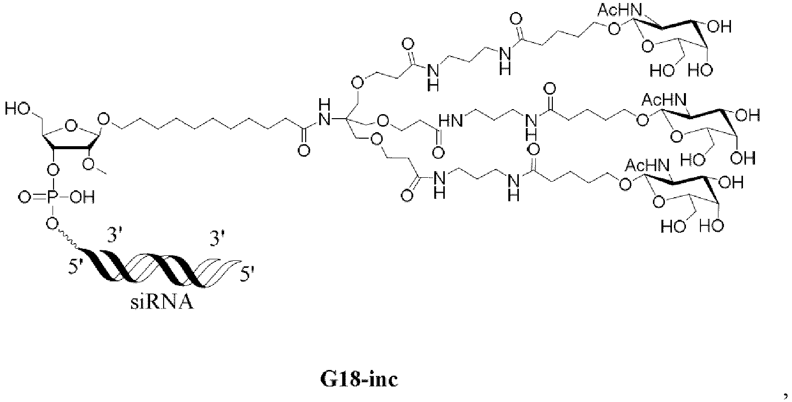 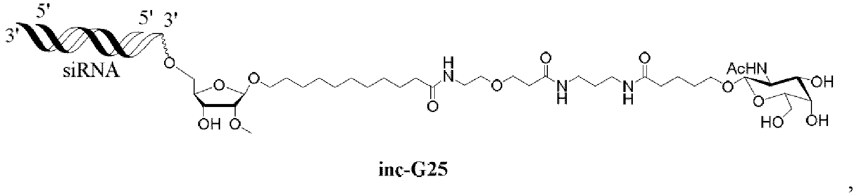 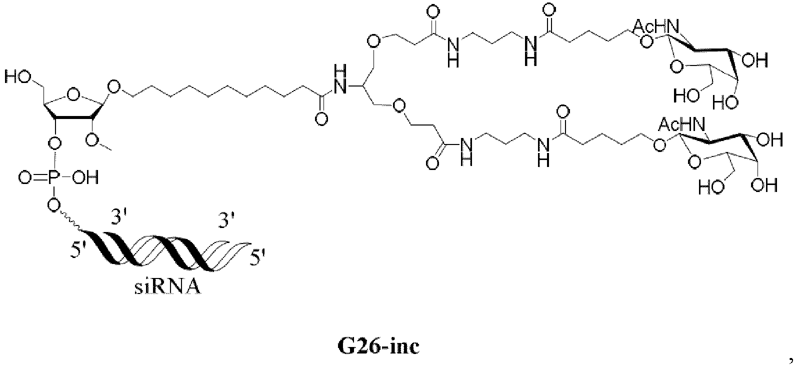 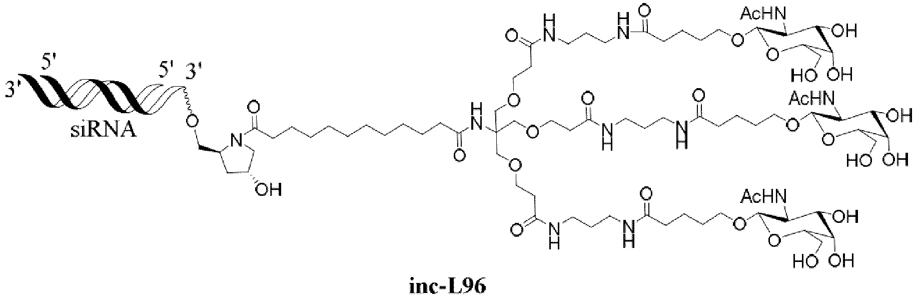 (GalNAc compound is L96, the compound of claim 10 disclosed in US10465194B2), 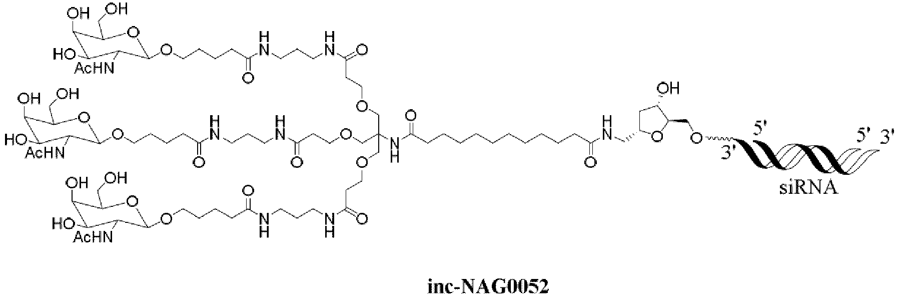 (GalNAc compound is NAG0052, page 100 of WO2023109938A1), 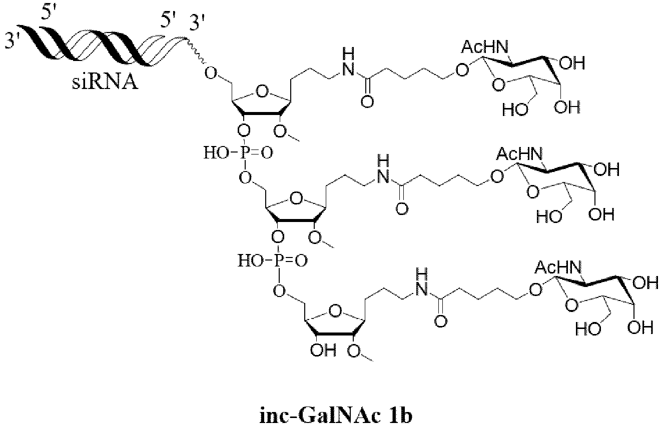 (GalNAc compound is GalNAc 1b, page 145 of WO2023014938A1), 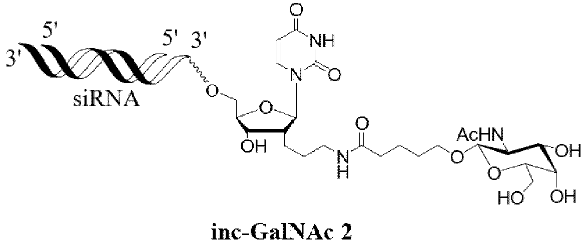 (GalNAc compound is GalNAc 2, page 216 of WO2023049258A1).

### Example 4: Effects of GalNAc-conjugated siRNA on inhibiting PCSK9 expression in mouse serum and liver and on LDL-C level

This example investigated the inhibitory rate of the siRNA conjugates in Table 2 on PCSK9 expression in mouse serum and liver and their effect on LDL-C levels. After the GalNAc-conjugated inclisiran siRNA sequence enters the blood, it first specifically binds to the asialoglycoprotein receptor (ASGPR) on the liver cell membrane through GaINAc, thereby entering the liver cells. After the inclisiran siRNA sequence enters the liver cells, it binds to the RNA-induced silencing complex (RISC), and then binds to the mRNA encoding the PCSK9 protein under the mediation of the antisense strand, to inhibit the production of the PCSK9 protein. The reduction of PCSK9 protein in the liver promotes the circulation of LDL-R, thereby increasing the number of LDL-R receptors on the surface of liver cells, increasing the uptake and degradation of plasma LDL-C, and further reducing the plasma LDL-C level. Therefore, the higher the amount of inclisiran siRNA sequence delivered to the liver, that is, the higher the GalNAc delivery efficiency, the lower the amount of PCSK9 protein in the liver and serum, and the lower the level of LDL-C in the liver.

Results show that compared to the GalNAc oligonucleotide conjugates in the prior art, in the inc-G4, inc-G5, inc-G6, inc-G7, inc-G11, inc-G12, inc-G13 and G18-inc experimental groups, the PCSK9 expression inhibition rate and LDL-C reduction level in mouse serum and liver were significantly increased. For example, compared to inc-L96, inc-NAG0052, inc-GalNAc 1b and inc-GalNAc 2, the inhibition rate of PCSK9 in serum by inc-G5 increased by 14.2%, 13.1%, 14.5% and 51.2% respectively on Day 7, and by 10.2%, 10.7%, 10.4% and 43.0% respectively on Day 14; the PCSK9 inhibition rate in the mouse liver increased by 11.1%, 13.2%, 11.0% and 44.0% respectively; and the LDL-C reduction level by inc-G5 increased by 20.4%, 17.1%, 15.8% and 29.8% respectively on Day 7, and by 23.5%, 20.5%, 20.0% and 33.3% respectively on Day 14.

### Animal preparation:

First, hPCSK9 transgenic mice (SPF grade, purchased from Jiangsu GemPharmatech Co., Ltd) were adaptively bred and then randomly divided into a negative control group (no medication) and siRNA test drug groups (inc-G1 to inc-G13, G18-inc, inc-G25, G26-inc, inc-L96, inc-NAG0052, inc-GalNAc 1b and inc-GalNAc 2 in Table 2) according to the PSCK9 protein content in serum, with 5 mice in each group, all of which were male.

### Route and dosage of administration:

The drug was administered by a single subcutaneous injection at a dose of 6 mg/kg, a volume of 1 mL/kg, and a concentration of 6 mg/mL. The day of administration was designated as day 0.

### 1. Inhibitory efficiency of PCSK9 in hPCSK9 mouse serum by different GalNA c-conjugated siRNAs

The results of the inhibition efficiency of PCSK9 in the serum of hPCSK9 mice show that the oligonucleotide conjugates prepared by the GalNAc compounds of the present disclosure significantly improved the inhibition rate of PCSK9 protein expression in the serum of mice compared with GalNAc of the prior art. For example, compared with inc-L96, inc-NAG0052, inc-GalNAc 1b and inc-GalNAc 2, the inhibition rate of inc-G5 was increased by 14.2%, 13.1%, 14.5% and 51.2% respectively on Day 7, and by 10.2%, 10.7%, 10.4% and 43.0% respectively on Day 14.

### Experimental process:

Before administration (D0), 7 days after administration (D7), and 14 days after administration (D14), about 200 µL of blood (without anticoagulation) was collected from the orbital venous plexus of the experimental animals. The whole blood samples were temporarily stored in an ice box before centrifugation, and the serum was separated by centrifugation at 4000 r/min for 10 min at 4°C. The PCSK9 protein level in the serum was detected using an ELISA kit (Sino Biological Company).

### Experimental results:

The inhibition rates of PCSK9 protein in serum of the negative control group and each test drug group 7 days after administration and 14 days after administration are shown in Table 4. Data statistics and analysis were performed using software GraphPad Prism 9.

### 1) Structural differences

The structural differences of GalNAc oligonucleotide conjugates are shown in Table 3:

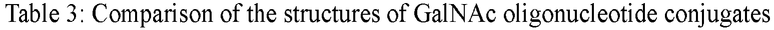

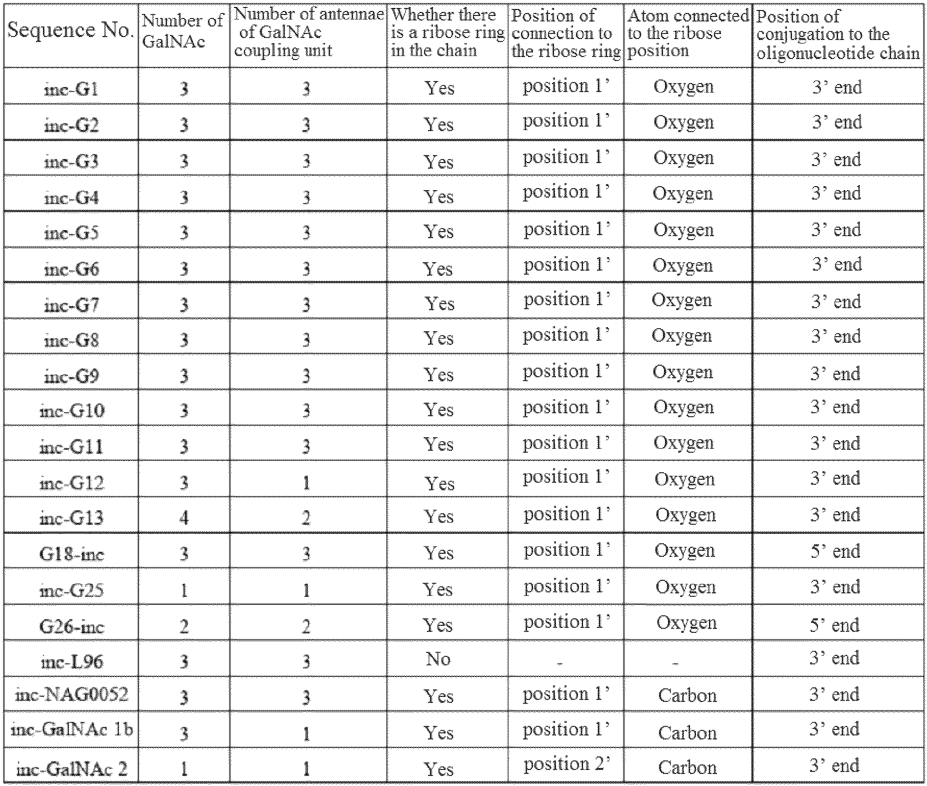

### 2) Inhibitory efficiency of PCSK9 in the serum of hPCSK9 mice

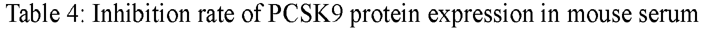

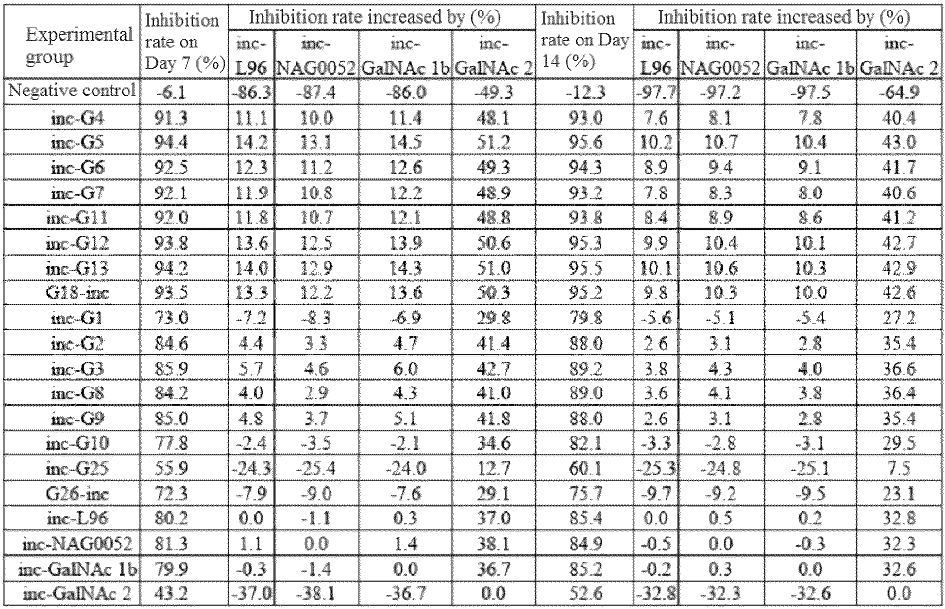

**1) inc-G4, inc-G5, inc-G6, inc-G7, inc-G11, inc-G12, inc-G13 and G18-inc had the highest inhibition rate of PCSK9 protein expression in mouse serum. For example, the inhibition rates of inc-G5 reached 94.4% and 95.6% on day 7 and day 14, respectively. It shows that a cleverly designed connection structure can significantly improve the bioavailability of drugs and achieve better efficacy.**

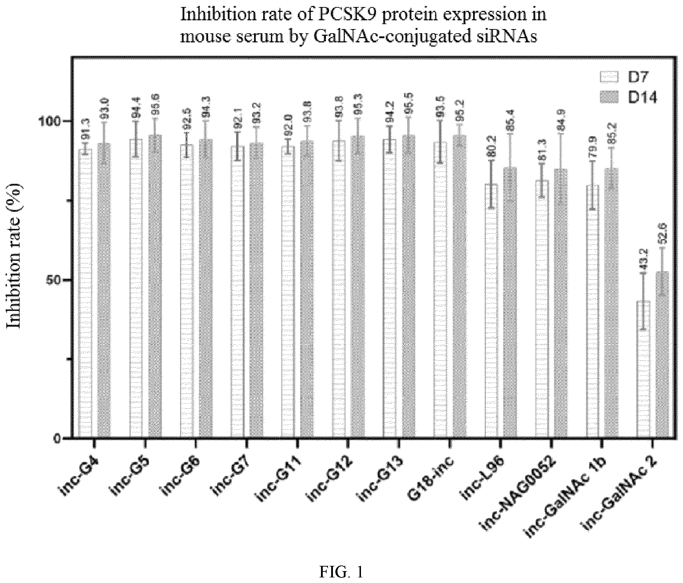

It can be seen from Table 4 that the conjugates prepared from the GalNAc compounds designed in the present disclosure had very different inhibitory effects on PCSK9 protein expression in mouse serum. The inhibition rates of inc-G4, inc-G5, inc-G6, inc-G7, inc-G11, inc-G12, inc-G13 and G18-inc were significantly higher than those of other groups, with inhibition rate exceeding 90% on days 7 and 14. The highest inhibition rate was inc-G5, reaching 94.4% and 95.6% on day 7 and 14 respectively. (FIG. 1)

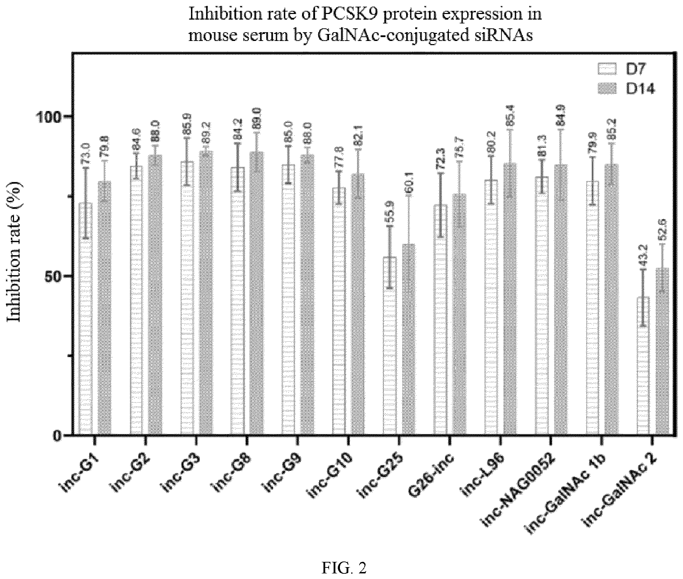

The inhibition rates of inc-G2, inc-G3, inc-G8 and inc-G9 were also high, at around 85%. The inhibition rates of inc-G1, inc-G10 and G26-inc were between 70-80%. The lowest inhibition rate was inc-G25, at 55.9%. (FIG. 2)

Inc-G5 with the highest inhibition rate increased by 38.5% and 35.5% on days 7 and 14 respectively compared with inc-G25 with the lowest inhibition rate, which was a significant difference.

**2) Conjugates with 3 or 4 GalNAc groups prepared from GalNAc compounds with 3 antennae, 1 antenna or 2 antennae designed in the present disclosure all had very high inhibition rates, and can effectively inhibit PCSK9 protein expression regardless of whether the GalNAc compound was conjugated to the 3' end or to the 5' end of the oligonucleotide.**

Among GalNAc compounds designed in the present disclosure, **YK-GAL-325** has one antenna, and **YK-GAL-326** has two antennae (see Table 1 in Example 2). inc-G12 is a conjugate with three GalNAc groups by connecting **YK-GAL-325** through two phosphodiester bonds. inc-G13 is a conjugate with four GalNAc groups by connecting **YK-GAL-326** through one phosphodiester bond. Activity test results show that inc-G12 and inc-G13 also had a significant inhibitory effect on PCSK9 protein expression in mouse serum, and the inhibition rates reached 93.8% and 94.2% respectively on Day 7 as well as 95.3% and 95.5% respectively on Day 14, which were comparable to inc-G5.

The GalNAc ligand of G18-inc was conjugated to the 5' end of the oligonucleotide, while the GalNAc ligand of inc-G5 was conjugated to the 3' end of the oligonucleotide. Activity test results show that G18-inc also had a significant inhibitory effect on PCSK9 protein expression in mouse serum, and the inhibition rates reached 93.5% and 95.2% on Day 7 and Day 14 respectively, which were comparable to inc-G5.

It can be seen that oligonucleotide conjugates with 3 or 4 GalNAc groups prepared from the GalNAc compounds with 3 antennae, 1 antenna or 2 antennae designed in the present disclosure all had very high delivery efficiency, and can efficiently deliver the oligonucleotide inclisiran siRNA sequence to the liver whether the GalNAc compound was conjugated to the 3' end or 5' end of the oligonucleotide, achieving significant inhibition of PCSK9 protein expression in mouse serum.

**3) Oligonucleotide conjugates prepared from GalNAc compounds designed in the present disclosure, compared with inc-L96, inc-NAG0052, inc-GalNAc 1b and inc-GalNAc 2 prepared from GalNAc compounds in the prior art, significantly improved the inhibition rate of PCSK9 protein expression in mouse serum. For example, the inhibition rate by inc-G5 was 14.2% higher than that of inc-L96.**

The conjugates prepared from the GalNAc compounds in the prior art, including inc-L96, inc-NAG0052, inc-GalNAc 1b and inc-GalNAc 2, had an inhibition rate of PCSK9 protein expression of 80.2%, 81.3%, 79.9% and 43.2% in mouse serum on Day 7, and 85.4%, 84.9%, 85.2% and 52.6% on Day 14, respectively.

The conjugates prepared from the GalNAc compounds of the present disclosure, including inc-G4, inc-G5, inc-G6, inc-G7, inc-G11, inc-G12, inc-G13 and G18-inc, compared with the GalNAc in the prior art, significantly improved the inhibition rate of PCSK9 protein expression in mouse serum.

For example, compared to inc-L96, inc-NAG0052, inc-GalNAc 1b and inc-GalNAc 2, the inhibition rates by inc-G5 were increased by 14.2%, 13.1%, 14.5% and 51.2% respectively on Day 7 and 10.2%, 10.7%, 10.4% and 43.0% respectively on Day 14. Both inc-G25 and inc-GalNAc 2 were oligonucleotide conjugates with one GalNAc group, while the inhibition rate by inc-G25 was 12.7% higher than that of inc-GalNAc 2, which was significantly improved.

**4) The inhibition rates of PCSK9 protein expression in mouse serum by oligonucleotide conjugates prepared from GalNAc compounds with similar structures are highly likely to be very different; while the inhibition rates of PCSK9 protein expression in mouse serum by oligonucleotide conjugates prepared from GalNAc compounds with great structural differences are also highly likely to be very similar.**

From the structural formula of the GalNAc oligonucleotide conjugates (Example 3) and the structural comparison in Table 3, it can be seen that the structures of this series of GalNAc compounds designed in the present disclosure are very similar, with only slight differences in individual groups, and are also very similar to the structures of the GalNAc compounds in the prior art, L-96, NAG0052, GalNAc 1b and GalNAc 2. However, the inhibition rates of PCSK9 protein expression in mouse serum by the oligonucleotide conjugates prepared from these structurally similar GalNAc compounds are similar in some cases, but very different in others.

For example, compared with inc-L96, inc-G5 only changes the prolinol structure 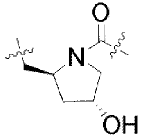 in the main chain of inc-L96 to a ribose ring 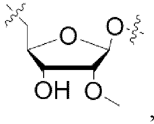 while the other structures are exactly the same. However, the inhibition rate of PCSK9 protein expression by inc-G5 could be increased by 14.2% compared with that of inc-L96, showing a significantly improved inhibitory activity.

Compared with GalNAc 1b, inc-G12 is only different in the atom connected to the 1' position of the ribose ring, which is carbon in GalNAc 1b, and oxygen in inc-G12. The connecting arm of inc-G12 is longer than that of GalNAc 1b, while the other structures are exactly the same. However, the inhibition rate by inc-G12 was 13.9% higher than that of GalNAc 1b, which was significantly improved.

Compared with inc-G1, inc-G5 is only different in the group at 2' position of the ribose ring, which is a methoxy in inc-G5, and a hydrogen in inc-G1. However, the inhibition rate by inc-G5 was 21.4% higher than that of inc-G1.

Therefore, GalNAc compounds with similar chemical structures do not necessarily have similar oligonucleotide delivery efficiencies. The inhibitory effects of the GalNAc oligonucleotide conjugates prepared therefrom on the PCSK9 gene in mouse serum are not consistent and are highly likely to have very significant differences.

### 2. Inhibitory efficiency of different GalNAc-conjugated siRNAs on PCSK9 in the liver of hPCSK9 mice

### Experimental process:

On Day 14 after administration (D14), the experimental animals were anesthetized and sacrificed, and then perfused, and then the liver was collected.

According to the ratio of tissue weight to RNA lysis solution (trizol, Ambion) = 100 mg: 1 mL, the tissue was quickly put into a 1.5 mL RNase-free EP tube containing 1 mL of RNA lysis solution (trizol), and three 3 mm steel beads (RNA-free treated) were added to the tube. The tube was put into a tissue homogenizer, which run at 50 Hz for 30 seconds, stopped for 10 seconds, and run for 3 times, to prepare tissue homogenate.

After centrifugation at 4°C and 12,000 × g for 3 minutes, 400 µL of homogenate supernatant was transferred to a 1.5 mL RNase-free EP tube and placed on ice. Each tube was added with 80 µL of chloroform, shaken vigorously for 15 seconds, and placed still at room temperature for 5 min. After centrifugation at 4°C and 12,000 × g for 15 minutes, 150 µL of supernatant was collected to a new EP tube.

An equal volume of isopropanol was added, gently mixed by inverting the tube upside down, placed still at -20°C for 10 minutes, and centrifuged at 4 °C and 12,000 × g for 15 minutes. The supernatant was then discarded.

1 mL of 75% ethanol was added to gently wash the RNA precipitate. After centrifugation at 7,500 × g and 4 °C for 5 minutes, the supernatant was removed. The rinse step was repeated once. After centrifugation at 7,500 × g and 4 °C for 5 minutes, any remaining ethanol was removed with a micropipette tip.

The residual ethanol was dried at room temperature for 10 minutes, and 150 µL of RNase-free ddH₂O was added for dissolution. The RNA concentration was detected using a micro-UV spectrophotometer. The PCSK9 gene expression was detected by qPCR, and the mRNA was measured. The results are shown in Table 5.

### Experimental results:

By counting the qPCR results (Mean±SD), plotting and data analysis were performed using software GraphPad Prism 9. The specific results are shown in Table 5.

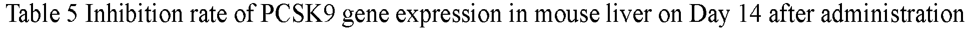

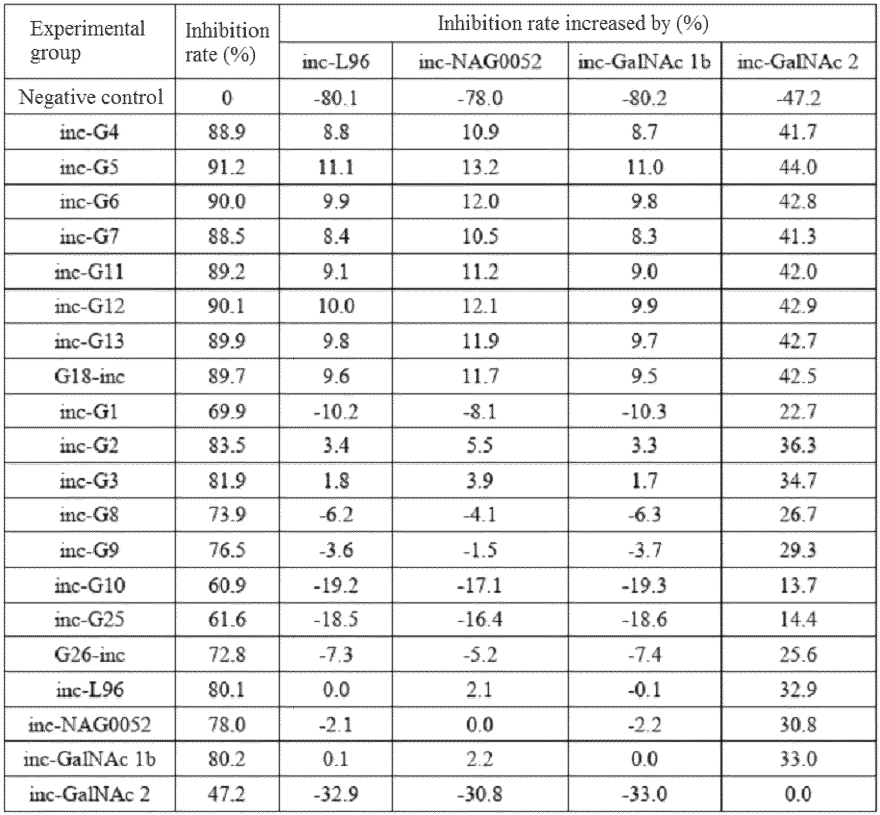

**1) inc-G4, inc-G5, inc-G6, inc-G7, inc-G11, inc-G12, inc-G13 and G18-inc had the highest inhibition rate of PCSK9 protein expression in mouse liver. For example, inc-G5, inc-G6 and inc-G12 all achieved 90% inhibition rates. It shows that a cleverly designed connection structure can significantly improve the bioavailability of drugs and achieve better efficacy.**

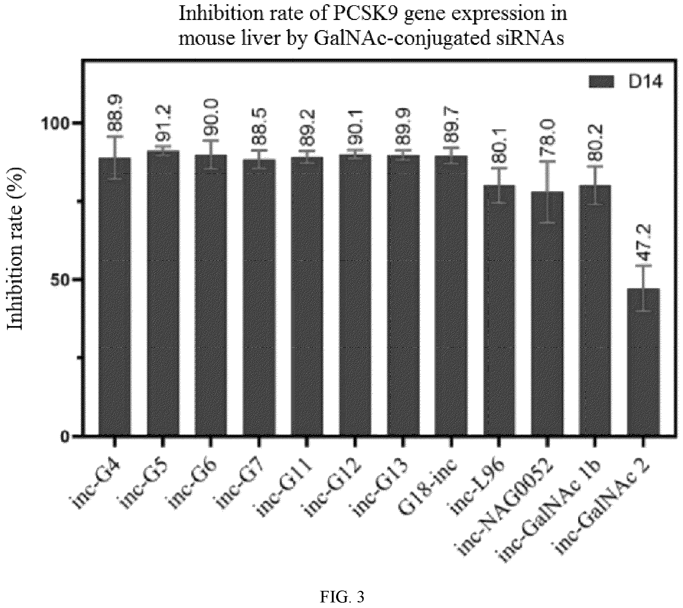

It can be seen from Table 5 that the conjugates prepared from the GalNAc compounds designed in the present disclosure had very different inhibitory effects on PCSK9 gene expression in mouse liver. The inc-G4, inc-G5, inc-G6, inc-G7, inc-G11, inc-G12, inc-G13 and G18-inc had the highest inhibition rates, all above 85%, among which inc-G5, inc-G6 and inc-G12 reached 90%. (FIG. 3)

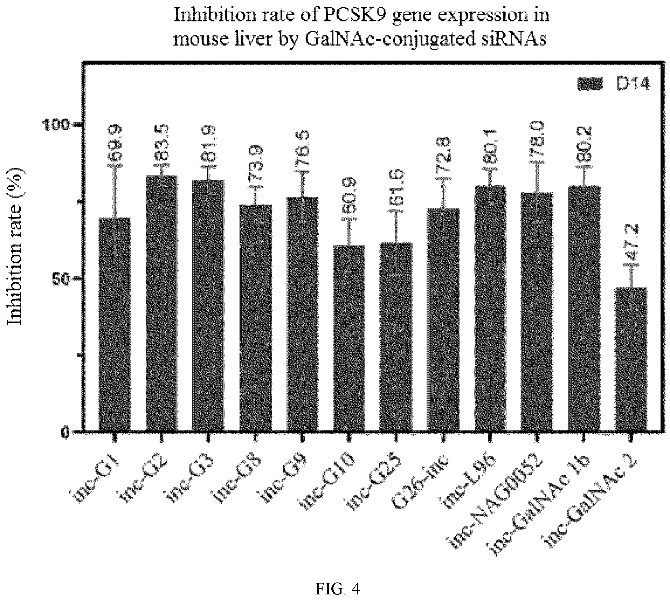

The inhibition rates by inc-G2 and inc-G3 were also high, between 80-85%. The inhibition rates by inc-G8, inc-G9 and G26-inc were between 70-80%. The inhibition rates by inc-G1, inc-G10 and inc-G25 were between 60-70%. (FIG. 4)

Inc-G5 with the highest inhibition rate increased by 30.3% compared with inc-G10 with the lowest inhibition rate, which was a significant difference.

**2) Oligonucleotide conjugates with 3 or 4 GalNAc groups prepared from GalNAc compounds with 3 antennae, 1 antenna or 2 antennae designed in the present disclosure all had very high inhibition rates, and can effectively inhibit PCSK9 gene expression regardless of whether the GalNAc compound was conjugated to the 3' end or to the 5' end of the oligonucleotide.**

Among GalNAc compounds designed in the present disclosure, **YK-GAL-325** has one antenna, and **YK-GAL-326** has two antennae (see Table 1 in Example 2). inc-G12 is a conjugate with three GalNAc groups by connecting **YK-GAL-325** through two phosphodiester bonds. inc-G13 is a conjugate with four GalNAc groups by connecting **YK-GAL-326** through one phosphodiester bond. Activity test results show that inc-G12 and inc-G13 also had a significant inhibitory effect on PCSK9 gene expression in mouse liver, with inhibition rates of 90.1% and 89.9% respectively, which were comparable to inc-G5.

The GalNAc ligand of G18-inc was conjugated to the 5' end of the oligonucleotide, while the GalNAc ligand of inc-G5 was conjugated to the 3' end of the oligonucleotide. Activity test results show that G18-inc also had a significant inhibitory effect on PCSK9 gene expression in mouse liver, with an inhibition rate of 89.7%, which was comparable to inc-G5.

It can be seen that oligonucleotide conjugates with 3 or 4 GalNAc groups prepared from the GalNAc compounds with 3 antennae, 1 antenna or 2 antennae designed in the present disclosure all had very high delivery efficiency, can efficiently deliver the oligonucleotide inclisiran siRNA sequence to the liver whether the GalNAc compound was conjugated to the 3' end or 5' end of the oligonucleotide, and had significant inhibition effect on PCSK9 gene expression in mouse liver.

**3) Oligonucleotide conjugates prepared from GalNAc compounds designed in the present disclosure, compared with inc-L96, inc-NAG0052, inc-GalNAc 1b and inc-GalNAc 2 prepared from GalNAc compounds in the prior art, significantly improved the inhibition rate. For example, the inhibition rate by inc-G5 was 11.1% higher than that of inc-L96.**

The conjugates prepared from the GalNAc compounds in the prior art, including inc-L96, inc-NAG0052, inc-GalNAc 1b and inc-GalNAc 2, inhibited PCSK9 gene expression in mouse liver by 80.1%, 78.0%, 80.2% and 47.2%, respectively.

The conjugates prepared from the GalNAc compounds of the present disclosure, including inc-G4, inc-G5, inc-G6, inc-G7, inc-G11, inc-G12, inc-G13 and G18-inc, compared with the GalNAc in the prior art, significantly improved the inhibition rate of PCSK9 gene expression in mouse liver.

For example, compared to inc-L96, inc-NAG0052, inc-GalNAc 1b and inc-GalNAc 2, the inhibition rates of inc-G5 were increased by 11.1%, 13.2%, 11.0% and 44.0%, respectively. Both inc-G25 and inc-GalNAc 2 were oligonucleotide conjugates with one GalNAc group, while the inhibition rate of inc-G25 was 14.4% higher than that of inc-GalNAc 2, which was significantly improved.

**4) The inhibition rates of PCSK9 gene expression in mouse liver by oligonucleotide conjugates prepared from GalNAc compounds with similar structures are highly likely to be very different; while the inhibition rates of PCSK9 gene expression in mouse liver by oligonucleotide conjugates prepared from GalNAc compounds with great structural differences are also highly likely to be very similar.**

From the structural formula of the GalNAc oligonucleotide conjugates (Example 3) and the structural comparison in Table 3, it can be seen that the structures of this series of GalNAc compounds designed in the present disclosure are very similar, with only slight differences in individual groups, and are also very similar to the structures of the GalNAc compounds in the prior art, L-96, NAG0052, GalNAc 1b and GalNAc 2. However, the inhibition rates of PCSK9 gene expression in mouse liver by the oligonucleotide conjugates prepared from these structurally similar GalNAc compounds are similar in some cases, but very different in others.

For example, compared with inc-L96, inc-G5 only changes the prolinol structure in the main chain of inc-L96 to a ribose ring structure, while the other structures are exactly the same. However, the inhibition rate of PCSK9 gene expression by inc-G5 could be increased by 11.1% compared with inc-L96, showing significantly improved inhibitory activity.

Compared with GalNAc 1b, inc-G12 is only different in the atom connected to the 1' position of the ribose ring, which is carbon in GalNAc 1b, and oxygen in inc-G12. The connecting arm of inc-G12 is longer than that of GalNAc 1b, while the other structures are exactly the same. However, the inhibition rate of inc-G12 was 9.9% higher than that of GalNAc 1b, which was significantly improved.

Compared with inc-G1, inc-G5 is only different in the group at 2' position of the ribose ring, which is a methoxy in inc-G5, and a hydrogen in inc-G1. However, the inhibition rate of inc-G5 was 21.3% higher than that of inc-G1.

inc-GalNAc 2 and inc-G25 were both oligonucleotide conjugates with one GalNAc group, but the inhibition rate of inc-G25 was 14.4% higher than that of inc-GalNAc 2, which was significantly improved.

Therefore, GalNAc compounds with similar chemical structures do not necessarily have similar oligonucleotide delivery efficiencies. The inhibitory effects of the GalNAc oligonucleotide conjugates prepared therefrom on the PCSK9 gene in mouse liver are not consistent and are highly likely to have very significant differences.

### 3. Effects of different GalNAC-conjugated siRNAs on LDL-C levels in hPCSK9 mice

### Experimental process:

Before administration (D0), 7 days after administration (D7), and 14 days after administration (D14), about 200 µL of blood (without anticoagulation) was collected from the orbital venous plexus of the experimental animals. The whole blood samples were temporarily stored in an ice box before centrifugation, and the serum was separated by centrifugation at 4000 r/min and 4°C for 10 minutes. The serum LDL-C level was detected.

### Experimental results:

Serum LDL-C (Mean±SD) was statistically analyzed and plotted using software GraphPad Prism 9. The specific results are shown in Table 6.

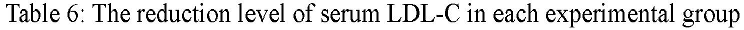

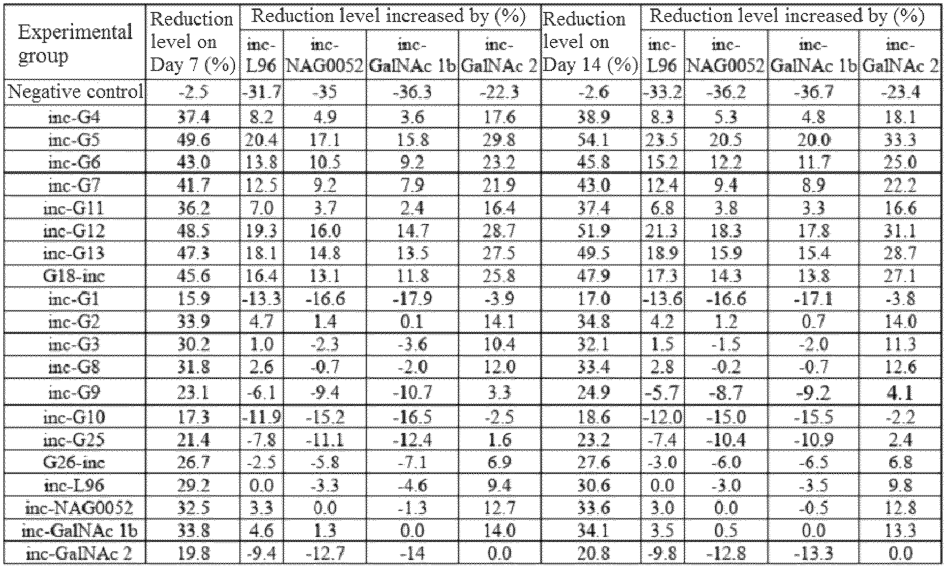

**1) inc-G4, inc-G5, inc-G6, inc-G7, inc-G11, inc-G12, inc-G13 and G18-inc had significantly higher LDL-C reduction levels than other groups. For example, inc-G5 reached 49.6% and 54.1% on days 7 and 14, respectively. It shows that a cleverly designed connection structure can significantly improve the bioavailability of drugs and achieve better efficacy.**

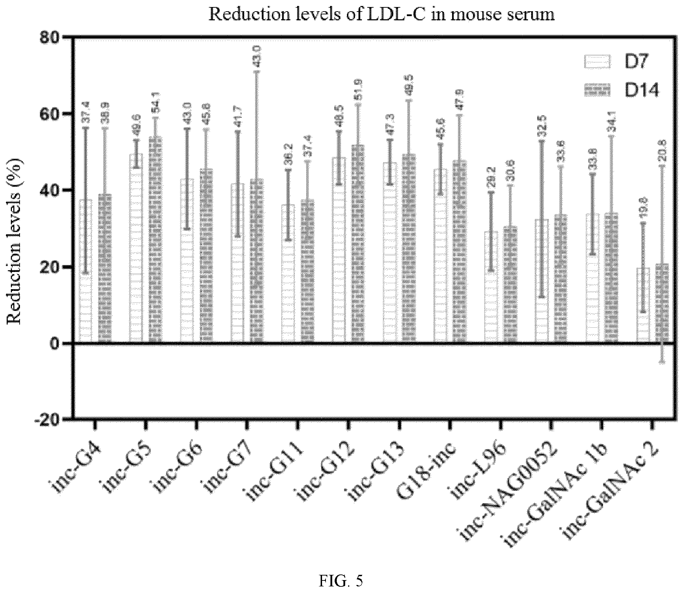

It can be seen from Table 6 that the conjugates inc-G4, inc-G5, inc-G6, inc-G7, inc-G11 , inc-G12 , inc-G13 and G18-inc which prepared from the GalNAc compoundsdesigned in the present disclosure had the highest reduction level of LDL-C in the serum of mice, exceeding 35% on days 7 and 14, which was significantly higher than that of other experimental groups. Among them, inc-G5 had the highest reduction level, reaching 49.6% and 54.1% on days 7 and 14 respectively. (FIG. 5)

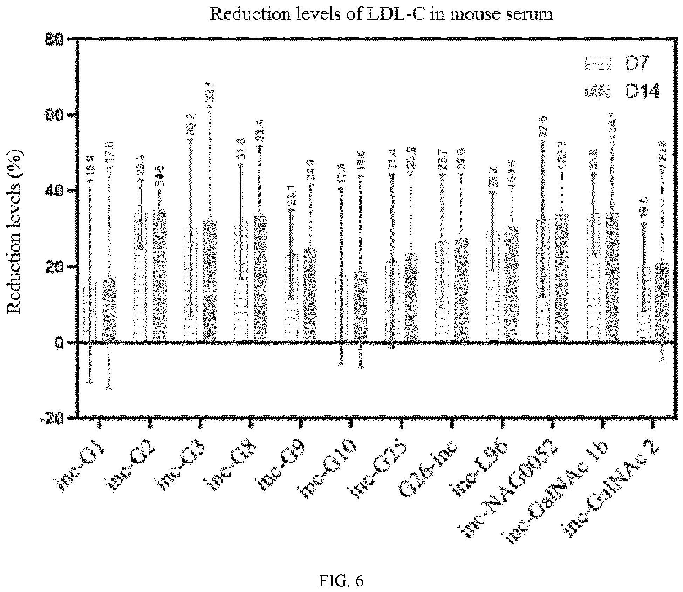

The reduction levels of inc-G2, inc-G3 and inc-G8 were between 30-35%. The reduction levels of inc-G9, inc-G25 and G26-inc were between 20-30%. The reduction levels of inc-G1 and inc-G10 were less than 20%, among which the reduction levels of inc-G1 were only 15.9% and 17.0% on Day 7 and Day 14, respectively. (FIG. 6)

Inc-G5 with the highest reduction level increased by more than 30% compared with inc-G1 with the lowest reduction level, which was a significant difference.

**2) Oligonucleotide conjugates with 3 or 4 GalNAc groups prepared from GalNAc compounds with 3 antennae, 1 antenna or 2 antennae designed in the present disclosure all had very high reduction levels, and can effectively reduce LDL-C levels regardless of whether the GalNAc compound was conjugated to the 3' end or to the 5' end of the oligonucleotide.**

Among GalNAc compounds designed in the present disclosure, **YK-GAL-325** has one antenna, and **YK-GAL-326** has two antennae (see Table 1 in Example 2). inc-G12 is a conjugate with three GalNAc groups by connecting **YK-GAL-325** through two phosphodiester bonds. inc-G13 is a conjugate with four GalNAc groups by connecting **YK-GAL-326** through one phosphodiester bond. Activity test results show that inc-G12 and inc-G13 could also significantly reduce LDL-C levels in mouse serum, with reduction levels of 48.5% and 47.3% respectively on Day 7 as well as 51.9% and 49.5 on Day 14, which were comparable to inc-G5.

The GalNAc ligand of G18-inc was conjugated to the 5' end of the oligonucleotide, while the GalNAc ligand of inc-G5 was conjugated to the 3' end of the oligonucleotide. Activity test results show that G18-inc could also significantly reduce LDL-C levels in mouse serum, with reduction levels of 45.6% and 47.9% on Day 7 and Day 14 respectively, which were comparable to inc-G5.

It can be seen that oligonucleotide conjugates with 3 or 4 GalNAc groups prepared from the GalNAc compounds with 3 antennae, 1 antenna or 2 antennae designed in the present disclosure all had significant inhibitory effect on PCSK9 gene expression, and can efficiently deliver the oligonucleotide inclisiran siRNA sequence to the liver whether the GalNAc compound was conjugated to the 3' end or 5' end of the oligonucleotide, achieving significant inhibition of PCSK9 gene expression in mouse serum, thereby reducing LDL-C levels.

**3) Oligonucleotide conjugates prepared from GalNAc compounds designed in the present disclosure, compared with inc-L96, inc-NAG0052, inc-GalNAc 1b and inc-GalNAc 2 prepared from GalNAc compounds in the prior art, significantly improved the LDL-C reduction levels in mouse serum. For example, inc-G5 increased the reduction levels by 20.4% and 23.5% on Day 7 and Day 14 respectively compared with inc-L96.**

Among the conjugates prepared from the GalNAc compounds in the prior art, the reduction levels of inc-L96, inc-NAG0052 and inc-GalNAc 1b were between 25-35%, and the reduction level of inc-GalNAc 2 was around 20%.

The conjugates prepared from the GalNAc compounds of the present disclosure, including inc-G4, inc-G5, inc-G6, inc-G7, inc-G11, inc-G12, inc-G13 and G18-inc, compared with the GalNAc in the prior art, significantly improved the reduction levels of LDL-C in mouse serum.

For example, compared to inc-L96, inc-NAG0052, inc-GalNAc 1b and inc-GalNAc 2, inc-G5 increased the reduction levels by 20.4%, 17.1%, 15.8% and 29.8% respectively on Day 7 and 23.5%, 20.5%, 20.0% and 33.3% respectively on Day 14.

**4) The reduction levels of LDL-C in mouse serum by oligonucleotide conjugates prepared from GalNAc compounds with similar structures are highly likely to be very different; while the reduction levels of LDL-C in mouse serum by oligonucleotide conjugates prepared from GalNAc compounds with great structural differences are also highly likely to be very similar.**

From the structural formula of the GalNAc oligonucleotide conjugates (Example 3) and the structural comparison in Table 3, it can be seen that the structures of this series of GalNAc compounds designed in the present disclosure are very similar, with only slight differences in individual groups, and are also very similar to the structures of the GalNAc compounds in the prior art, L-96, NAG0052, GalNAc 1b and GalNAc 2. However, the LDL-C level in mouse serum affected by the oligonucleotide conjugates prepared from these structurally similar GalNAc compounds are similar in some cases, but very different in others.

For example, compared with inc-L96, inc-G5 only changes the prolinol structure in the main chain of inc-L96 to a ribose ring structure, while the other structures are exactly the same. However, inc-G5 can increase reduction level of LDL-C in serum more than inc-L96 by 20%, which was significantly improved. Compared with GalNAc 1b, inc-G12 is only different in the atom connected to the 1' position of the ribose ring, which is carbon in GalNAc 1b, and oxygen in inc-G12. The connecting arm of inc-G12 is longer than that of GalNAc 1b, while the other structures are exactly the same. However, inc-G12 increased the reduction level higher than that of GalNAc 1b by more than 15%, which was significantly improved. Compared with inc-G1, inc-G5 is only different in the group at 2' position of the ribose ring, which is a methoxy in inc-G5, and a hydrogen in inc-G1. However, inc-G5 can reduce the level of LDL-C in mouse serum by 50%, while inc-G1 only reduced it by about 15%, showing that inc-G5 can increase the reduction level higher than that of inc-G1 by more than 30%, which was a significant difference.

Therefore, GalNAc compounds with similar chemical structures do not necessarily have similar oligonucleotide delivery efficiencies. The effects of the GalNAc oligonucleotide conjugates prepared therefrom on LDL-C levels in mouse serum are not consistent and are highly likely to have very significant differences.

### Example 5: Distribution of different GalNAc-conjugated siRNAs in different tissues and organs of mice

After entering the blood, the inclisiran siRNA sequence coupled with the GalNAc ligand first partially enters the liver cells with the help of GalNAc and inhibits the target gene. The remaining siRNA in the serum is cleared by the kidneys. Since siRNA in plasma is mainly cleared by the kidneys, the siRNA sequence is also distributed in the kidneys in addition to the liver.

### Experimental process:

6-8 week old wild-type C57BL/6 mice were used, 6 mice in each group. Each group was administered with siRNA conjugates with Cy5 fluorescent group: inc-G1, inc-G2, inc-G3, inc-G4, inc-G5, inc-G6, inc-G7, inc-G8, inc-G9, inc-G10, inc-G11, inc-G12, inc-G13, G18-inc, inc-G25, G 26-inc, inc-L 96, inc-NAG0052, inc-GalNAc 1b and inc-GalNAc 2. The negative control group was not administered with any drug. The mice were weighed and administered by subcutaneous injection at 6 mg/kg. The injection amount did not exceed 0.1-0.2 mL according to the administration volume.

After administration was completed, the hair on the mouse abdomen was shaved clean. The mice were placed in a small animal living imaging system in a supine position after anesthetized by isoflurane. The imaging of mice was observed 4 hours and 8 hours after administration under the Cy5 channel. The luminescence intensity of mice was counted using the living imaging software Living Image, and the differences between different test groups were compared.

### Experimental results:

The results of the fluorescence intensity in mouse liver and kidney (which can represent the relative content of oligonucleic acid) are shown in Table 7.

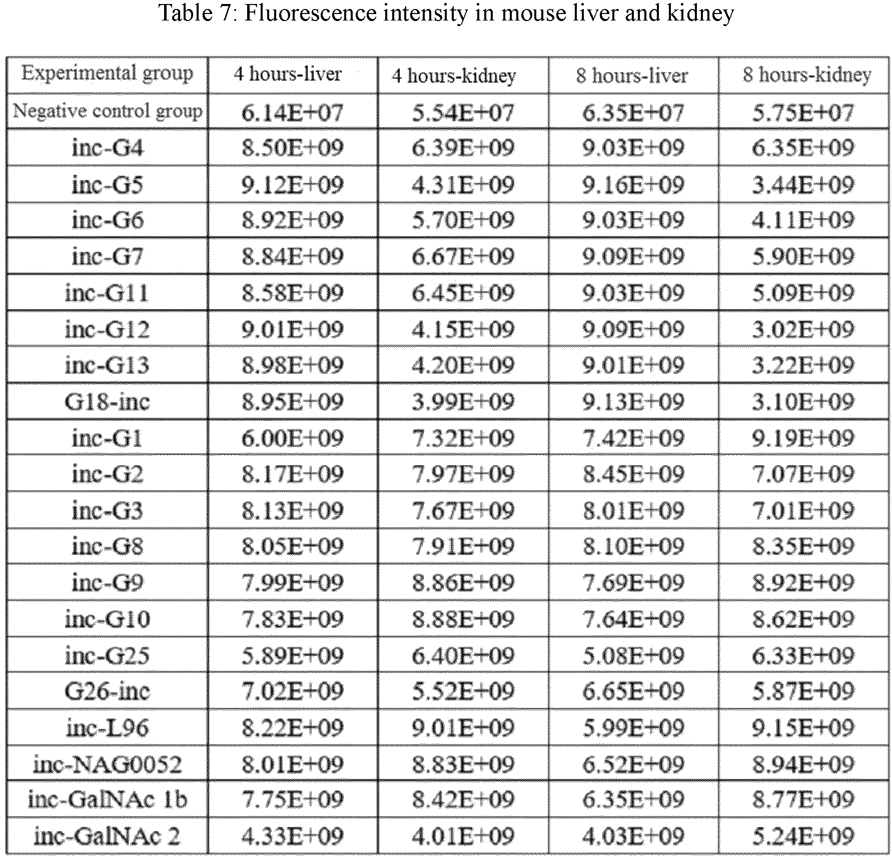

**1) inc-G4, inc-G5, inc-G6, inc-G7, inc-G11, inc-G12, inc-G13 and G18-inc had significantly higher liver fluorescence intensity than other groups. For example, inc-G5 with the highest fluorescence intensity can reach 9.12E+09 and 9.16E+09 at 4 h and 8 h respectively. It shows that a cleverly designed connection structure can significantly improve the delivery effect of oligonucleotide drugs.**

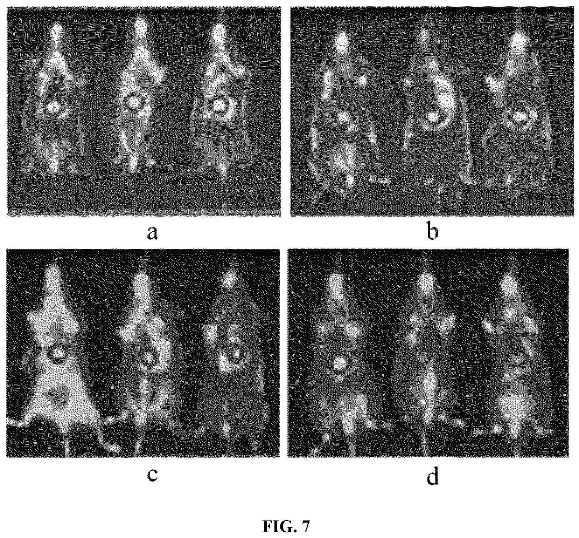

It can be seen from Table 7 that oligonucleotides labeled with Cy5 fluorescence were distributed in both mouse liver and kidney, and the fluorescence intensity of the conjugates prepared from the GalNAc compounds designed in the present disclosure varied greatly. The fluorescence intensities of inc-G4, inc-G5, inc-G6, inc-G7, inc-G12, inc-G13 and G18-inc in the liver were significantly higher than that of other groups, meaning that the relative content of oligonucleotides was significantly higher than other groups. Among them, inc-G5 had the highest fluorescence intensity, reaching 9.12E+09 at 4 h and 9.16E+09 at 8 h. (FIG. 7)

The fluorescence intensities of inc-G2, inc-G3, and inc-G8 were also high, ranging from 8.00E+09 to 8.50E+09 at 4 and 8 hours. The fluorescence intensities of inc-G9, inc-G10, and G26-inc were between 7.00E+09 and 8.00E+09 at 4 h and between 6.50E+09 and 8.00E+09 at 8 h. The fluorescence intensities of inc-G1 and inc-G25 were between 5.00E+09 and 7.50E+09.

Inc-G5 with the highest fluorescence intensity was higher than inc-G1 with the lowest fluorescence intensity by 52.0% and 23.5% at 4 h and 8 h respectively, which was a significant improvement.

**2) Oligonucleotide conjugates with 3 or 4 GalNAc groups prepared from GalNAc compounds with 3 antennae, 1 antenna or 2 antennae designed in the present disclosure all had very high fluorescence intensity in the liver, and can effectively deliver oligonucleotides to the liver regardless of whether the GalNAc compound was conjugated to the 3' end or to the 5' end of the oligonucleotide.**

Among GalNAc compounds designed in the present disclosure, **YK-GAL-325** has one antenna, and **YK-GAL-326** has two antennae (see Table 1 in Example 2). inc-G12 is a conjugate with three GalNAc groups by connecting **YK-GAL-325** through two phosphodiester bonds. inc-G13 is a conjugate with four GalNAc groups by connecting **YK-GAL-326** through one phosphodiester bond. The fluorescence intensities of inc-G12 and inc-G13 at 4 h were 9.01E+09 and 8.98E+09, respectively, and at 8 h were 9.09E+09 and 9.01E+09, respectively, which were comparable to that of inc-G5.

The GalNAc ligand of G18-inc was conjugated to the 5' end of the oligonucleotide, while the GalNAc ligand of inc-G5 was conjugated to the 3' end of the oligonucleotide. The fluorescence intensity of G18-inc at 4 h and 8 h was 8.95E+09 and 9.13E+09, respectively, which was comparable to that of inc-G5.

It can be seen that oligonucleotide conjugates with 3 or 4 GalNAc groups prepared from the GalNAc compounds with 3 antennae, 1 antenna or 2 antennae designed in the present disclosure all had very high delivery efficiency, and can efficiently deliver the oligonucleotide inclisiran siRNA sequence to the liver whether the GalNAc compound was conjugated to the 3' end or 5' end of the oligonucleotide.

**3) Oligonucleotide conjugates prepared from GalNAc compounds designed in the present disclosure, compared with inc-L96, inc-NAG0052, inc-GalNAc 1b and inc-GalNAc 2 prepared from GalNAc compounds in the prior art, significantly improved the fluorescence absorption intensity of mouse liver. For example, the fluorescence absorption intensity improved by inc-G5 was higher than inc-L96 by more than 50%.**

It can be seen from Table 7 and FIG.7 that oligonucleotides labeled with Cy5 fluorescence were distributed in mouse liver and kidney, and the fluorescence intensity of inc-G4, inc-G5, inc-G6, inc-G7, inc-G12, inc-G13 and G18-inc in the liver was significantly higher than that of inc-L96, inc-NAG0052, inc-GalNAc 1b and inc-GalNAc 2 at 4 h and 8 h. For example, the liver fluorescence intensity of inc-G5 at 4 h and 8 h was 9.12E+09 and 9.16E+09 respectively, which were 10.9% and 52.9% higher than inc-L96, 13.9% and 40.5% higher than inc-NAG0052, 17.7% and 44.3% higher than inc-GalNAc 1b, and 110.6% and 127.3% higher than inc-GalNAc 2 respectively, which was a significant improvement. The fluorescence intensity of inc-G6 at 4 h and 8 h was 8.92E+09 and 9.03E+09 respectively, which were 8.5% and 50.8% higher than inc-L96, 11.4% and 38.5% higher than inc-NAG0052, 15.1% and 42.2% higher than inc-GalNAc 1b, and 106.0% and 124.1% higher than inc-GalNAc 2 respectively, which was a significant improvement.

**4) The efficiencies in delivering oligonucleotides to the liver by oligonucleotide conjugates prepared from GalNAc compounds with similar structures are highly likely to be very different; while the efficiencies in delivering oligonucleotides to the liver by oligonucleotide conjugates prepared from GalNAc compounds with great structural differences are also highly likely to be very similar.**

From the structural formula of the GalNAc oligonucleotide conjugates (Example 3) and the structural comparison in Table 3, it can be seen that the structures of this series of GalNAc compounds designed in the present disclosure are very similar, with only slight differences in individual groups, and are also very similar to the structures of the GalNAc compounds in the prior art, L-96, NAG0052, GalNAc 1b and GalNAc 2. However, the inhibition rates of PCSK9 protein expression in mouse serum by the oligonucleotide conjugates prepared from these structurally similar GalNAc compounds are similar in some cases, but very different in others.

For example, compared with inc-L96, inc-G5 only changes the prolinol structure in the main chain of inc-L96 to a ribose ring structure, while the other structures are exactly the same. However, inc-G5 can increase the liver fluorescence intensity higher than inc-L96 by more than 50%, showing significantly improved delivery efficiency.

Compared with GalNAc 1b, inc-G12 is only different in the atom connected to the 1' position of the ribose ring, which is carbon in GalNAc 1b, and oxygen in inc-G12. The connecting arm of inc-G12 is longer than that of GalNAc 1b, while the other structures are exactly the same. However, inc-G12 increased the liver fluorescence intensity higher than that of GalNAc 1b by more than 40%.

Compared with inc-G1, inc-G5 is only different in the group at 2' position of the ribose ring, which is a methoxy in inc-G5, and a hydrogen in inc-G1. However, inc-G5 increased the liver fluorescence intensity higher than that of inc-G1by more than 50%.

Therefore, GalNAc compounds with similar chemical structures do not necessarily have similar oligonucleotide delivery efficiencies. On the contrary, they are likely to have very significant differences.

### Example 6: Pharmacokinetics of different GalNAc-conjugated siRNAs in animal liver

### Experimental process:

The experimental animals were 6-9 week old SD male rats, 30 rats in each group. Each group was administered with siRNA conjugates: inc-G1, inc-G2, inc-G3, inc-G4, inc-G5, inc-G6, inc-G7, inc-G8, inc-G9, inc-G10, inc-G11, inc-G12, inc-G13, G18-inc, inc-G25, G26-inc, inc-L96, inc-NAG0052, inc-GalNAc 1b and inc-GalNAc 2. The rats were weighed and administered at 5 mg/kg by subcutaneous injection, at a concentration of 1 mg/mL and a volume of 5 mL. Tissue samples were collected at 9 time points, including 6, 24, 72, 168, 336, 504, 672, 1008 and 1344 h after administration. After the experimental animals were sacrificed with carbon dioxide, the liver was collected and rinsed with precooled saline, then dried with filter paper, weighed and transferred to a labeled tube, and homogenized under ice-cold conditions at a ratio of 1:9 (1 g of tissue added to 9 mL of homogenate) (homogenate: 100 mM Tris, 10 mM EDTA, pH 8.0). About 800 µL of the homogenized sample was stored at -80°C, and the drug concentration in the liver was then detected by LC-MS/MS.

**Experimental results:** The half-life, peak drug concentration and area under the drug-time curve data are shown in Table 8.

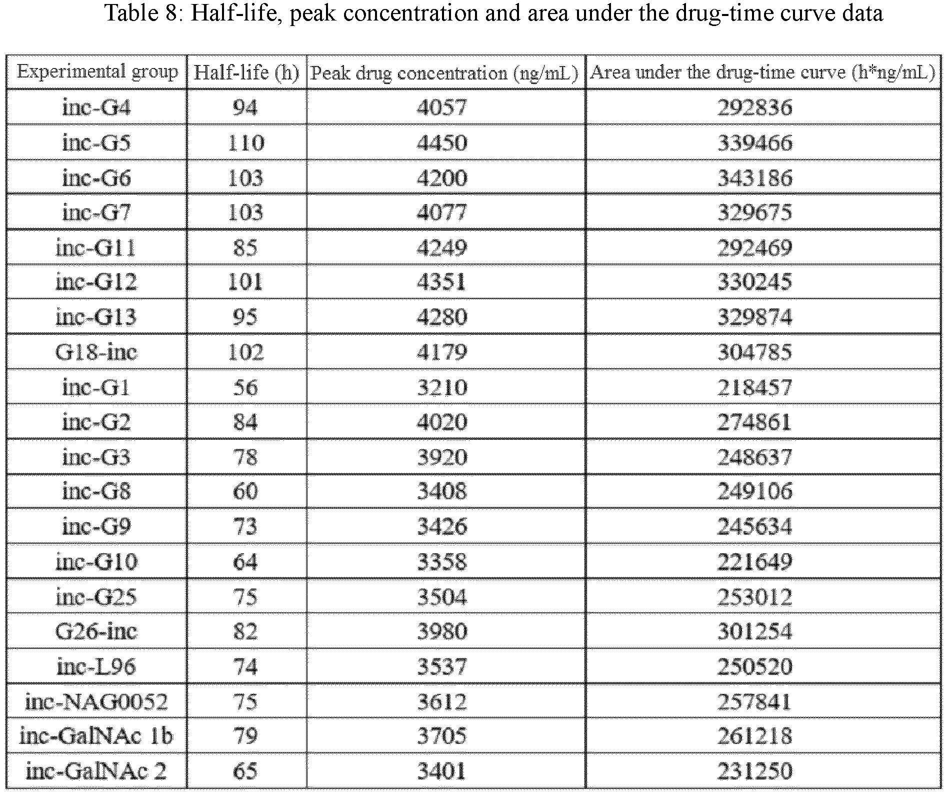

**1) inc-G4, inc-G5, inc-G6, inc-G7, inc-G11, inc-G12, inc-G13 and G18-inc had the significantly increased half-lives compared with other groups. For example, the half-lives of inc-G5, inc-G6, inc-G7, inc-G12 and G18-inc reached 110 hours, 103 hours, 103 hours, 101 hours and 102 hours respectively. It shows that a cleverly designed connection structure can significantly improve the pharmacokinetic properties of drugs in vivo.**

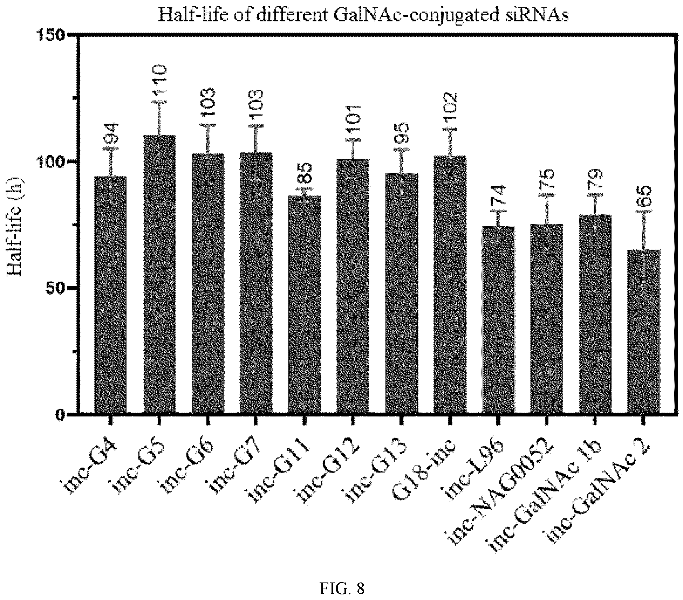

It can be seen from Table 8 that the conjugates prepared from the GalNAc compounds designed in the present disclosure had very different half-lives. The half-lives of inc-G4, inc-G5, inc-G6, inc-G7, inc-G11, inc-G12, inc-G13 and G18-inc were significantly increased compared with other groups. For example, the half-lives of inc-G5, inc-G6, inc-G7 and G18-inc reached 110 hours, 103 hours, 103 hours and 102 hours respectively, and inc-G4, inc-G11 and inc-G13 also reached 85 hours. (FIG. 8)

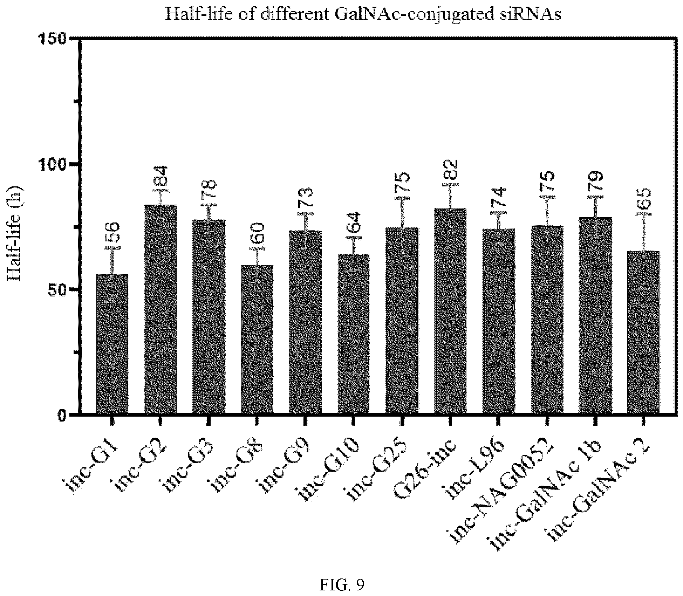

The half-lives of inc-G2, inc-G3, inc-G9, inc-G25 and G26-inc were between 70-85 hours. The half-lives of inc-G8 and inc-G10 were between 60-70 hours. Inc-G1 had the shortest half-life, which was 56 hours. (FIG. 9)

The difference between inc-G5 with the longest half-life and inc-G1 with the shortest half-life was 54 hours. The half-life of inc-G5 was twice as long as that of inc-G1, showing a very significant difference.

The increasing or decreasing trends of the peak concentration and the area under the drug-time curve of each test drug group were also consistent with the half-life.

**2) Conjugates with 3 or 4 GalNAc groups prepared from GalNAc compounds with 3 antennae, 1 antenna or 2 antennae designed in the present disclosure all had a long half-life in mice regardless of whether the GalNAc compound was conjugated to the 3' end or to the 5' end of the oligonucleotide.**

Among GalNAc compounds designed in the present disclosure, **YK-GAL-325** has one antenna, and **YK-GAL-326** has two antennae (see Table 1 in Example 2). inc-G12 is a conjugate with three GalNAc groups by connecting **YK-GAL-325** through two phosphodiester bonds. inc-G13 is a conjugate with four GalNAc groups by connecting **YK-GAL-326** through one phosphodiester bond. The half-lives of inc-G12 and inc-G13 in mice were 101 hours and 95 hours, respectively, which were comparable to that of inc-G5.

The GalNAc ligand of G18-inc was conjugated to the 5' end of the oligonucleotide, while the GalNAc ligand of inc-G5 was conjugated to the 3' end of the oligonucleotide. The half-life of G18-inc in mice reached 102 hours, which was comparable to that of inc-G5.

It can be seen that oligonucleotide conjugates with 3 or 4 GalNAc groups prepared from the GalNAc compounds with 3 antennae, 1 antenna or 2 antennae designed in the present disclosure were all very stable in mice, and had a long half-life whether the GalNAc compound was conjugated to the 3' end or 5' end of the oligonucleotide.

The increasing or decreasing trends of the peak concentration and the area under the drug-time curve of each test drug group were also consistent with the half-life.

**3) Oligonucleotide conjugates prepared from GalNAc compounds designed in the present disclosure, compared with inc-L96, inc-NAG0052, inc-GalNAc 1b and inc-GalNAc 2 prepared from GalNAc compounds in the prior art, had significantly improved half-life in mice. For example, the half-life of inc-G5 was 48.6% longer than that of inc-L96.**

The conjugates prepared from the GalNAc compounds in the prior art, including inc-L96, inc-NAG0052, inc-GalNAc 1b and inc-GalNAc 2, had half-lives in mice of 74 hours, 75 hours, 79 hours and 65 hours respectively. The conjugates prepared from the GalNAc compounds of the present disclosure, including inc-G4, inc-G5, inc-G6, inc-G7, inc-G11, inc-G12, inc-G13 and G18-inc, compared with the GalNAc in the prior art, had significantly improved half-life in mice.

For example, compared to inc-L96, inc-NAG0052, inc-GalNAc 1b and inc-GalNAc 2, the half-life of inc-G5 was improved by 48.6%, 46.7%, 39.2% and 69.2% respectively, which was significantly improved.

Both inc-G25 and inc-GalNAc 2 were oligonucleotide conjugates with one GalNAc group, while the half-life of inc-G25 was 15.4% higher than that of inc-GalNAc 2, which was significantly improved.

The increasing or decreasing trends of the peak concentration and the area under the drug-time curve of each test drug group were also consistent with the half-life.

**4) The half-lives in mice of oligonucleotide conjugates prepared from GalNAc compounds with similar structures are highly likely to be very different; while the half-lives in mice of oligonucleotide conjugates prepared from GalNAc compounds with great structural differences are also highly likely to be very similar.**

From the structural formula of the GalNAc oligonucleotide conjugates (Example 3) and the structural comparison in Table 3, it can be seen that the structures of this series of GalNAc compounds designed in the present disclosure are very similar, with only slight differences in individual groups, and are also very similar to the structures of the prior art GalNAc compounds L-96, NAG0052, GalNAc 1b and GalNAc 2. However, the half-lives in mice of the oligonucleotide conjugates prepared from these structurally similar GalNAc compounds are very different in some cases.

For example, compared with inc-L96, inc-G5 only changes the prolinol structure in the main chain of inc-L96 to a ribose ring structure, while the other structures are exactly the same. However, the half-life of inc-G5 can be 48.6% higher than that of inc-L96, which was significantly improved.

Compared with GalNAc 1b, inc-G12 is only different in the atom connected to the 1' position of the ribose ring, which is carbon in GalNAc 1b, and oxygen in inc-G12. The connecting arm of inc-G12 is longer than that of GalNAc 1b, while the other structures are exactly the same. However, the half-life of inc-G12 was 27.8% higher than that of GalNAc 1b, which was significantly improved.

Compared with inc-G1, inc-G5 is only different in the group at 2' position of the ribose ring, which is a methoxy in inc-G5, and a hydrogen in inc-G1. However, the half-life of inc-G5 was 96.4% higher than that of inc-G1, which was significantly improved.

Therefore, oligonucleotide conjugates prepared from GalNAc compounds with similar chemical structures do not necessarily have similar half-lives in mice. On the contrary, they are very likely to have very significant differences.

The increasing or decreasing trends of the peak concentration and the area under the drug-time curve of each test drug group were also consistent with the half-life.

The present disclosure designed a series of new GalNAc compounds, such as **YK-GAL-304, YK-GAL-305, YK-GAL-306, YK-GAL-307, YK-GAL-311, YK-GAL-318, YK-GAL-325 and YK-GAL-326.** The GalNAc oligonucleotide conjugates prepared therefrom can achieve efficient liver-targeted delivery and exhibit significantly improved activity and half-life compared to representative GalNAc compounds in the prior art.
1. The designed GalNAc compounds have a significantly different chemical structure compared with the prior art GalNAc compounds. The GalNAc compounds designed in the present disclosure introduce a ribose ring structure into the connecting arm and an oxygen or sulfur atom at the 1' position of the ribose ring.
2. The oligonucleotide conjugates inc-G4, inc-G5, inc-G6, inc-G7, inc-G11, inc-G12, inc-G13 and G18-inc prepared by the GalNAc compounds designed in the present disclosure have significantly improved delivery efficiency and half-life compared with other groups. It shows that a cleverly designed connection structure can significantly improve the bioavailability of drugs and improve the pharmacokinetic properties of drugs to achieve better efficacy. The conjugates with 3 or 4 GalNAc groups prepared from the GalNAc compounds with 3 antennae, 1 antenna or 2 antennae designed in the present disclosure all have high activities and long half-lives. Moreover, the activities and half-lives of oligonucleotide conjugates prepared from GalNAc compounds with similar structures are highly likely to be very different, and the activities and half-lives of oligonucleotide conjugates prepared from GalNAc compounds with large structural differences are also highly likely to be very similar.

The details are as follows:

### 1) Inhibition rate of PCSK9 protein expression in mouse serum

I. inc-G4, inc-G5, inc-G6, inc-G7, inc-G11, inc-G12, inc-G13 and G18-inc had the inhibition rate of PCSK9 protein expression in mouse serum that were significantly higher than those of other groups. For example, inc-G5 reached the inhibition rate of 94.4% and 95.6% on days 7 and 14, respectively. Moreover, compared with inc-L96, inc-NAG0052, inc-GalNAc 1b and inc-GalNAc 2 prepared from representative GalNAc compounds in the prior art, the inhibition rates of PCSK9 protein expression in mouse serum by the conjugates prepared from the GalNAc compounds of the present disclosure were significantly improved. For example, the inhibition rate of PCSK9 protein expression by inc-G5 was 14.2% better than inc-L96. It shows that a cleverly designed connection structure can significantly improve the bioavailability of drugs and achieve better efficacy.
II. Conjugates with 3 or 4 GalNAc groups prepared from GalNAc compounds with 3 antennae, 1 antenna or 2 antennae designed in the present disclosure all had very high inhibition rates, and can effectively inhibit PCSK9 protein expression regardless of whether the GalNAc compound was conjugated to the 3' end or to the 5' end of the oligonucleotide.
III. The inhibition rates of PCSK9 protein expression in mouse serum by oligonucleotide conjugates prepared from GalNAc compounds with similar structures are highly likely to be very different; while the inhibition rates of PCSK9 protein expression in mouse serum by oligonucleotide conjugates prepared from GalNAc compounds with great structural differences are also highly likely to be very similar.

### 2) Inhibition rate of PCSK9 gene expression in mouse liver

I. inc-G4, inc-G5, inc-G6, inc-G7, inc-G11, inc-G12, inc-G13 and G18-inc had the inhibition rate of PCSK9 protein expression in mouse liver that were significantly higher than that of other groups. For example, inc-G5, inc-G6 and inc-G12 all achieved 90% inhibition rates. Moreover, compared with inc-L96, inc-NAG0052, inc-GalNAc 1b and inc-GalNAc 2 prepared from the GalNAc compounds in the prior art, the inhibition rates of the conjugates mentioned above that prepared from the GalNAc compounds of the present disclosure were significantly improved. For example, the inhibition rate of inc-G5 was 11.1% higher than that of inc-L96. It shows that a cleverly designed connection structure can significantly improve the bioavailability of drugs and achieve better efficacy.
II. Oligonucleotide conjugates with 3 or 4 GalNAc groups prepared from GalNAc compounds with 3 antennae, 1 antenna or 2 antennae designed in the present disclosure all had very high inhibition rates, and can effectively inhibit PCSK9 gene expression in the liver regardless of whether the GalNAc compound was conjugated to the 3' end or to the 5' end of the oligonucleotide.
III. The inhibition rates of PCSK9 protein expression in mouse liver by oligonucleotide conjugates prepared from GalNAc compounds with similar structures are highly likely to be very different; while the inhibition rates of PCSK9 gene expression in mouse liver by oligonucleotide conjugates prepared from GalNAc compounds with great structural differences are also highly likely to be very similar.

### 3) Effects on LDL-C levels in mice

I. inc-G4, inc-G5, inc-G6, inc-G7, inc-G11, inc-G12, inc-G13 and G18-inc caused a more significant reduction level of LDL-C than other groups. For example, inc-G5 reached a reduction level of 49.6% and 54.1% on days 7 and 14, respectively. Moreover, compared with inc-L96, inc-NAG0052, inc-GalNAc 1b and inc-GalNAc 2 prepared from GalNAc compounds in the prior art, the reduction level of LDL-C in mouse serum was significantly improved by the conjugates of the present dosclosure. For example, inc-G5 increased the reduction level of LDL-C by 20.4% and 23.5% on Day 7 and Day 14 respectively compared with inc-L96. It shows that a cleverly designed connection structure can significantly improve the bioavailability of drugs and achieve better efficacy.
II. Oligonucleotide conjugates with 3 or 4 GalNAc groups prepared from GalNAc compounds with 3 antennae, 1 antenna or 2 antennae designed in the present disclosure all had very high reduction levels, and can effectively reduce LDL-C levels regardless of whether the GalNAc compound was conjugated to the 3' end or to the 5' end of the oligonucleotide.
III. The reduction levels of LDL-C in mouse serum by oligonucleotide conjugates prepared from GalNAc compounds with similar structures are highly likely to be very different; while the reduction levels of LDL-C in mouse serum by oligonucleotide conjugates prepared from GalNAc compounds with great structural differences are also highly likely to be very similar.

### 4) Distribution in mouse liver

I. inc-G4, inc-G5, inc-G6, inc-G7, inc-G11, inc-G12, inc-G13 and G18-inc had the liver fluorescence intensity significantly higher than other groups. For example, inc-G5 with the highest fluorescence intensity can reach 9.12E+09 and 9.16E+09 at 4 h and 8 h respectively. Moreover, compared with inc-L96, inc-NAG0052, inc-GalNAc 1b and inc-GalNAc 2 prepared from GalNAc compounds in the prior art, the fluorescence absorption intensity in the mouse liver of the conjugates of the present disclosure was significantly improved. For example, the fluorescence absorption intensity of inc-G5 was more than 50% higher than inc-L96. It shows that a cleverly designed connection structure can significantly improve the bioavailability of drugs and achieve better efficacy..
II. Oligonucleotide conjugates with 3 or 4 GalNAc groups prepared from GalNAc compounds with 3 antennae, 1 antenna or 2 antennae designed in the present disclosure all had very high fluorescence intensity in the liver, and can effectively deliver oligonucleotides to the liver regardless of whether the GalNAc compound was conjugated to the 3' end or to the 5' end of the oligonucleotide.
III. The efficiencies in delivering oligonucleotides to the liver by oligonucleotide conjugates prepared from GalNAc compounds with similar structures are highly likely to be very different; while the efficiencies in delivering oligonucleotides to the liver by oligonucleotide conjugates prepared from GalNAc compounds with great structural differences are also highly likely to be very similar.

### 5) Half-life in mice

I. inc-G4, inc-G5, inc-G6, inc-G7, inc-G11, inc-G12, inc-G13 and G18-inc had significantly increased half-lives compared with other groups. For example, the half-lives of inc-G5, inc-G6, inc-G7, inc-G12 and G18-inc reached 110 hours, 103 hours, 103 hours, 101 hours and 102 hours respectively. Moreover, compared with inc-L96, inc-NAG0052, inc-GalNAc 1b and inc-GalNAc 2 prepared from GalNAc compounds in the prior art, the half-lives of the conjugates mentioned above that prepared from the GalNAc compounds of the present disclosure in mice were significantly prolonged. For example, the half-life of inc-G5 was 48.6% longer than that of inc-L96. It shows that a cleverly designed connection structure can significantly improve the pharmacokinetic properties of drugs in vivo.
II. Conjugates with 3 or 4 GalNAc groups prepared from GalNAc compounds with 3 antennae, 1 antenna or 2 antennae designed in the present disclosure all had a long half-life in mice regardless of whether the GalNAc compound was conjugated to the 3' end or to the 5' end of the oligonucleotide.
III. The half-lives in mice of oligonucleotide conjugates prepared from GalNAc compounds with similar structures are highly likely to be very different; while the half-lives in mice of oligonucleotide conjugates prepared from GalNAc compounds with great structural differences are also highly likely to be very similar.

## Claims

1. A compound represented by formula (I) or a pharmaceutically acceptable salt thereof: 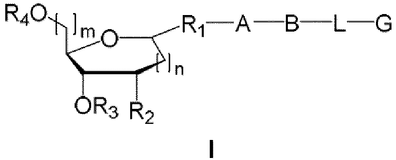 wherein,
R₁ is oxygen or sulfur;
R₂ is hydrogen, C₁₋₄ alkyl, C₁₋₄ alkoxy or halogen;
R₃ is hydrogen, a hydroxyl protecting group, a phosphorus-containing active reactive group, 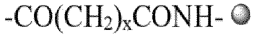 or -CO(CH₂)ₓCOOH, wherein x is an integer selected from 1 to 10, and 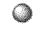 is controlled pore glass or polystyrene;
R₄ is hydrogen or a hydroxyl protecting group;
A is -(CH₂)ₐ-, -(CH₂CH₂O)_{b}-, -((CH₂)_{c}NHCO)_{d}-, or -((CH₂)_{c}CONH)_{d}-, wherein a is an integer selected from 1 to 15, b is an integer selected from 1 to 7, c is an integer selected from 1 to 7, and d is an integer selected from 1 to 5;
B is -(CH₂)ₑ-, wherein e is an integer selected from 0 to 7;
L is -CONH- or -NHCO-;
G is 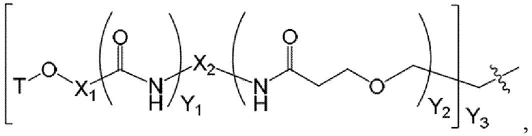
wherein,
T is N-acetyl-galactosamine with hydroxyl fully protected by acyl, galactose with hydroxyl fully protected by acyl, galactosamine with hydroxyl fully protected by acyl, N-formyl-galactosamine with hydroxyl fully protected by acyl, N-propionyl-galactosamine with hydroxyl fully protected by acyl, N-n-butyryl-galactosamine with hydroxyl fully protected by acyl, or N-isobutyryl-galactosamine with hydroxyl fully protected by acyl, wherein the acyl is acetyl or benzoyl;
X₁ is -(CH₂)_{f}- or -(CH₂CH₂O)_{f}CH₂-, wherein f is an integer selected from 1 to 5;
X₂ is -(CH₂)_{g}-, wherein g is an integer selected from 1 to 6;
Y₁ is 0 or 1;
Y₂ is 0, 1 or 2;
Y₃ is 1, 2 or 3;
m is an integer selected from 0 to 4; and
n is an integer selected from 0 to 4.

2. The compound represented by formula (I) or the pharmaceutically acceptable salt thereof according to claim 1, wherein A is -(CH₂)₁₀-, -(CH₂)₇-, -(CH₂)₈-, -(CH₂)₉-, -(CH₂)₁₁-, -(CH₂)₁₂-, -(CH₂CH₂O)₃-, -(CH₂)₄NHCO-, or -(CH₂)₆NHCO-.

3. The compound represented by formula (I) or the pharmaceutically acceptable salt thereof according to claim 1, wherein B is -(CH₂)₀-, -CH₂-, -(CH₂)₂-, -(CH₂)₄-, or -(CH₂)₃-.

4. The compound represented by formula (I) or the pharmaceutically acceptable salt thereof according to claim 1, wherein R₁ is oxygen.

5. The compound represented by formula (I) or the pharmaceutically acceptable salt thereof according to claim 1, wherein R₁ has an α configuration or a β configuration.

6. The compound represented by formula (I) or the pharmaceutically acceptable salt thereof according to claim 1, wherein R₂ is hydrogen or -OCH₃.

7. The compound represented by formula (I) or the pharmaceutically acceptable salt thereof according to any one of claims 1 to 6, wherein R₃ is a hydroxyl protecting group or a phosphorus-containing active reactive group.

8. The compound represented by formula (I) or the pharmaceutically acceptable salt thereof according to claim 7, wherein R₃ is acetyl, 1,1,3,3-tetraisopropyldisiloxanyl, tert-butyldimethylsilyl, phenyldimethylsilyl, 4,4'-dimethoxytrityl, or

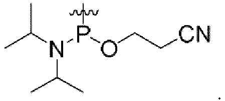

9. The compound represented by formula (I) or the pharmaceutically acceptable salt thereof according to any one of claims 1 to 6, wherein R₃ is

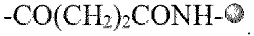

10. The compound represented by formula (I) or the pharmaceutically acceptable salt thereof according to any one of claims 1 to 6, wherein R₃ is -CO(CH₂)₂COOH.

11. The compound represented by formula (I) or the pharmaceutically acceptable salt thereof according to any one of claims 1 to 6, wherein R₄ is a hydroxyl protecting group.

12. The compound represented by formula (I) or the pharmaceutically acceptable salt thereof according to claim 11, wherein R₄ is trityl, monomethoxytrityl or 4,4'-dimethoxytrityl.

13. The compound represented by formula (I) or the pharmaceutically acceptable salt thereof according to any one of claims 1 to 6, wherein m is 1.

14. The compound represented by formula (I) or the pharmaceutically acceptable salt thereof according to any one of claims 1 to 6, wherein n is 0.

15. The compound represented by formula (I) or the pharmaceutically acceptable salt thereof according to any one of claims 1 to 6, wherein G is 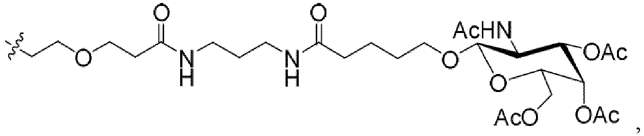 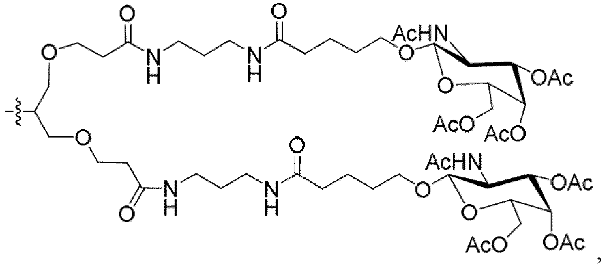 or

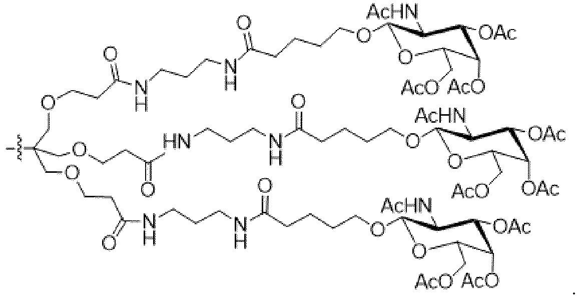

16. The compound represented by formula (I) or the pharmaceutically acceptable salt thereof according to claim 1, wherein the compound represented by formula (I) is YK-GAL-301, YK-GAL-302, YK-GAL-303, YK-GAL-304, YK-GAL-305, YK-GAL-306, YK-GAL-307, YK-GAL-308, YK-GAL-309, YK-GAL-310, YK-GAL-311, YK-GAL-312, or YK-GAL-313: 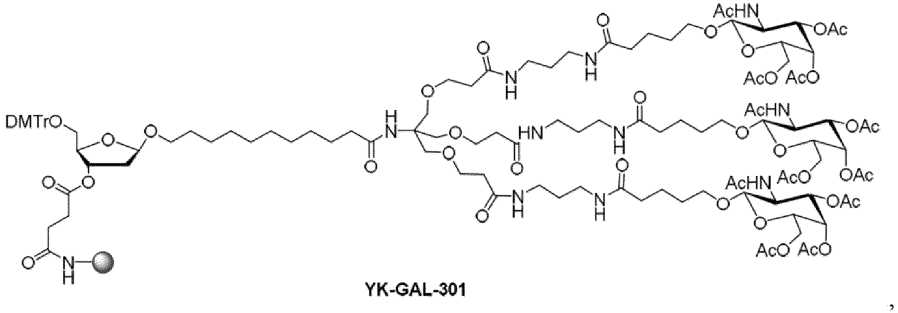 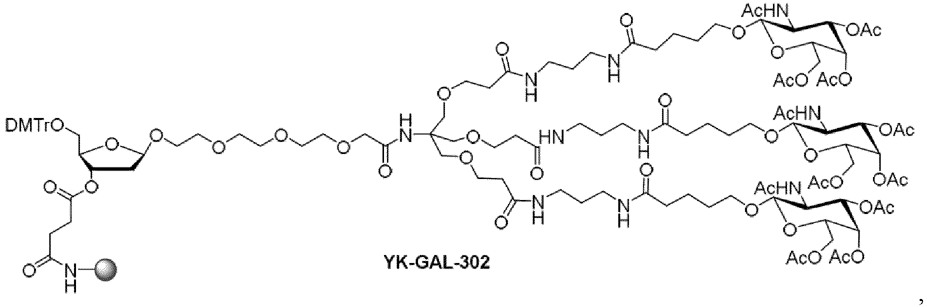 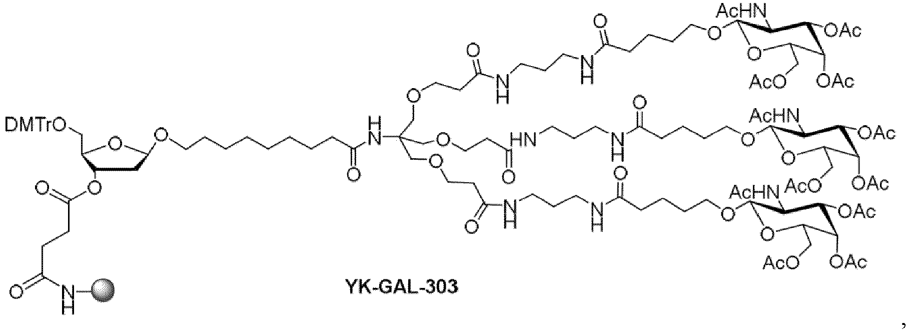 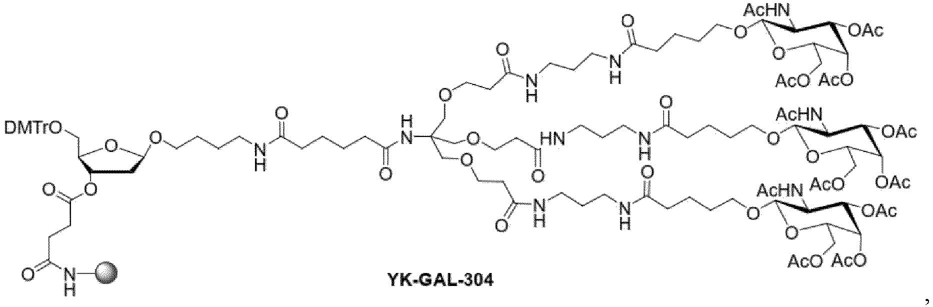 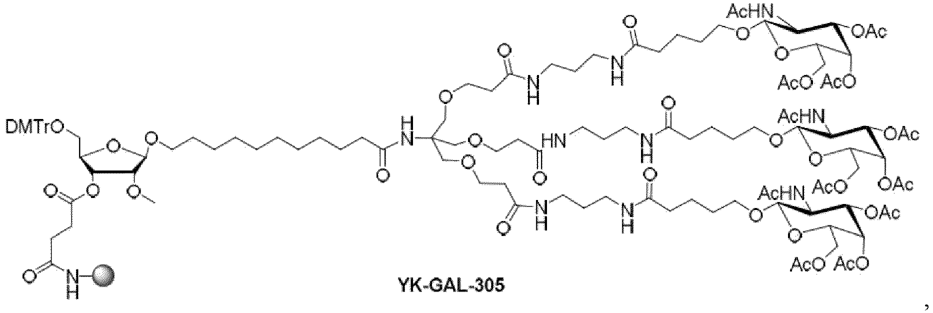 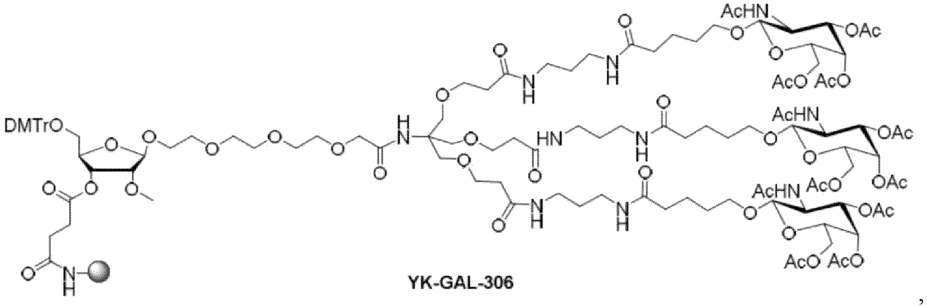 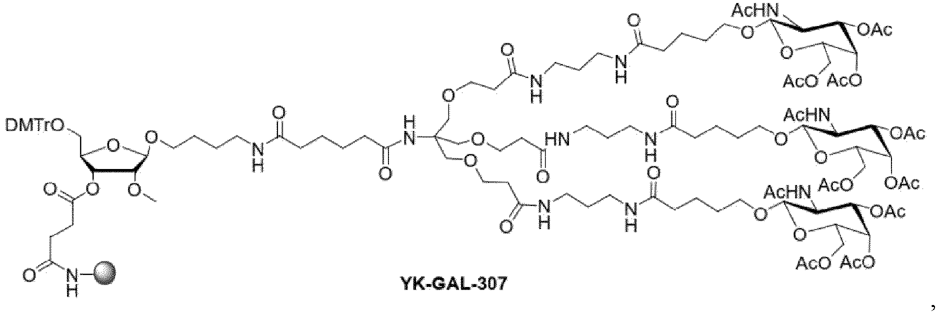 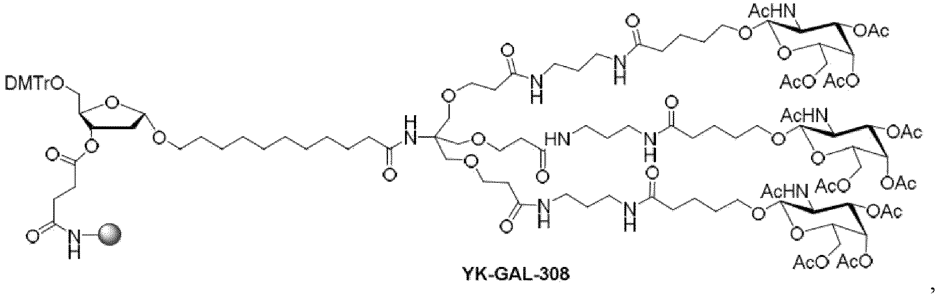 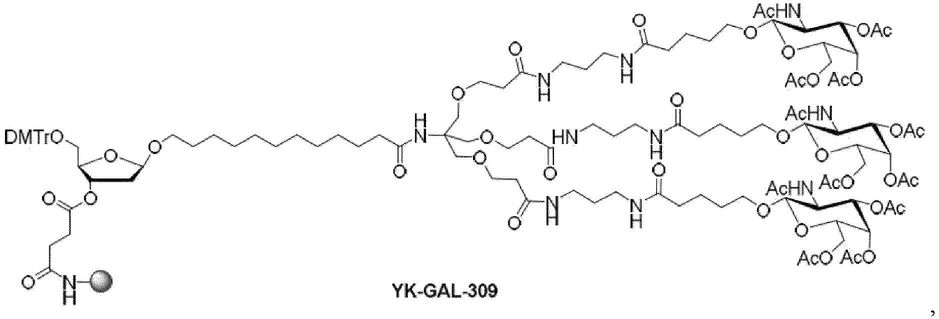 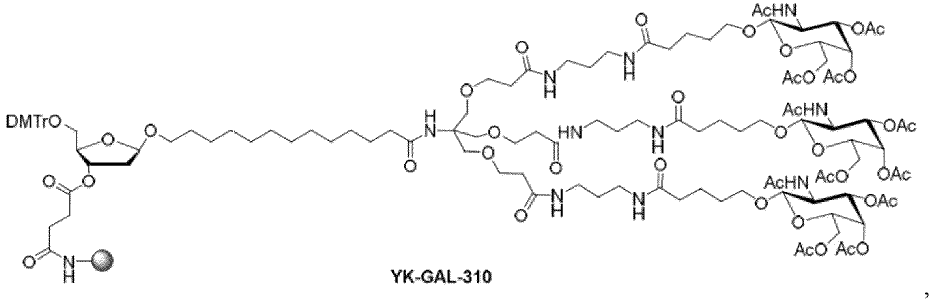 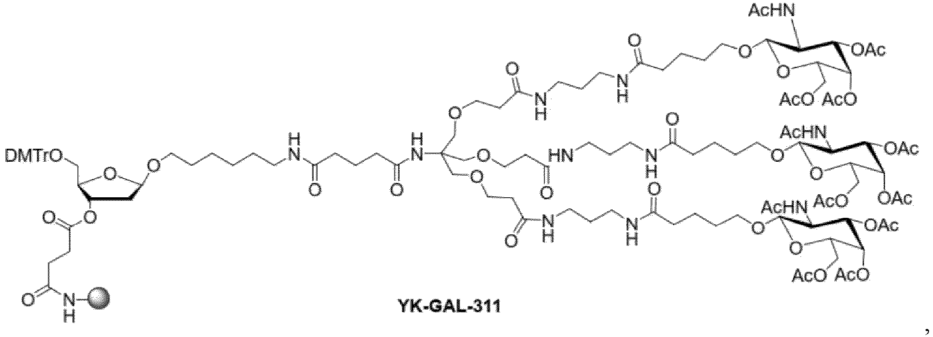 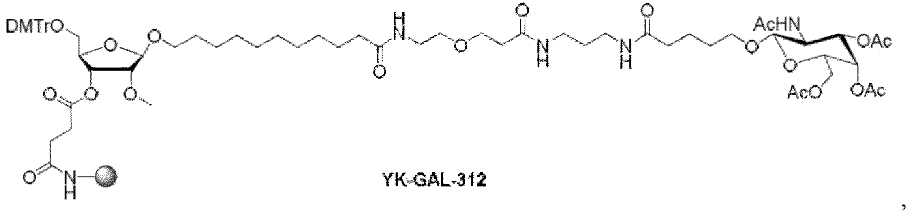 or 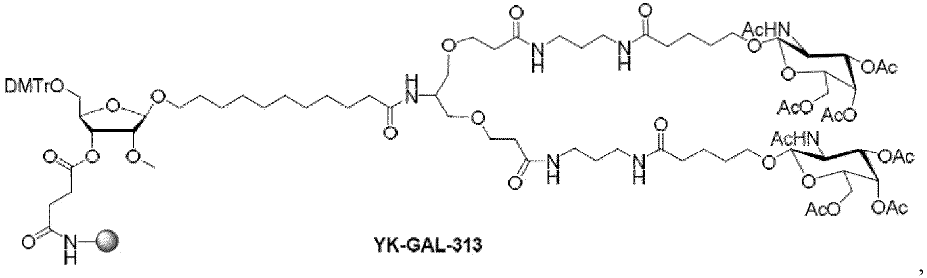 wherein 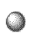 is controlled pore glass.

17. The compound represented by formula (I) or the pharmaceutically acceptable salt thereof according to claim 1, wherein the compound represented by formula (I) is YK-GAL-314, YK-GAL-315, YK-GAL-316, YK-GAL-317, YK-GAL-318, YK-GAL-319, YK-GAL-320, YK-GAL-321, YK-GAL-322, YK-GAL-323, YK-GAL-324, YK-GAL-325, or YK-GAL-326: 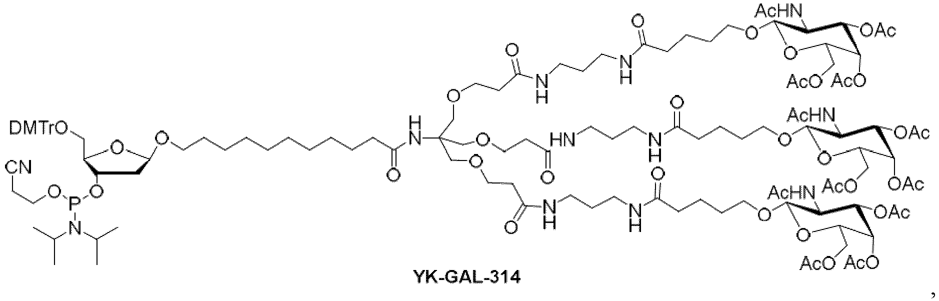 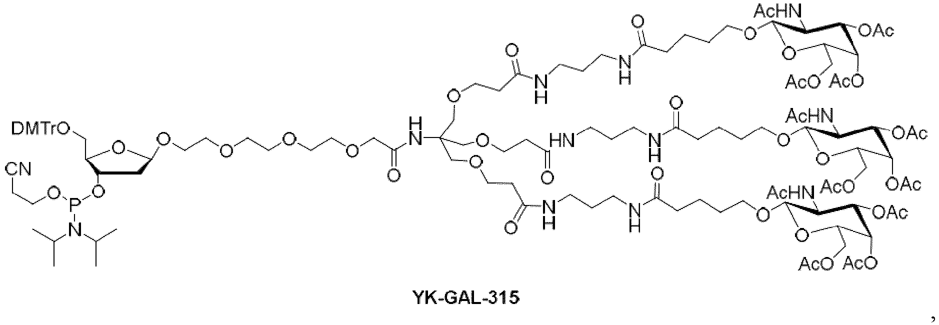 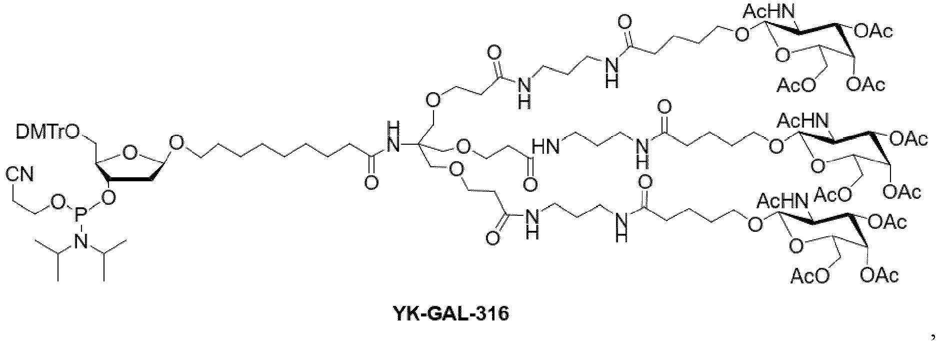 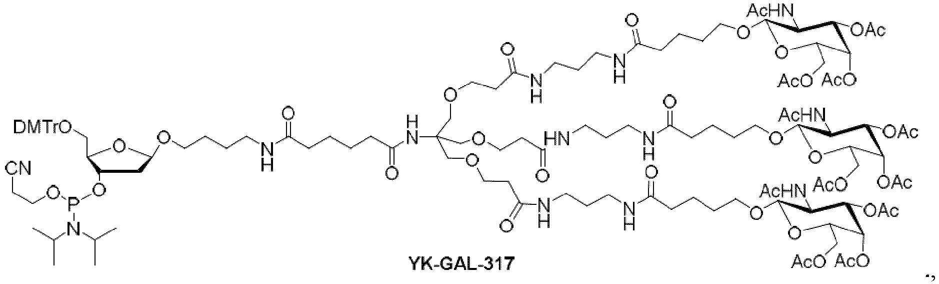 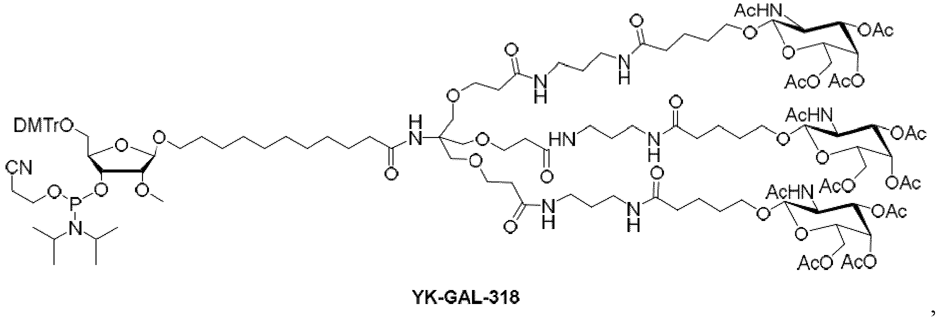 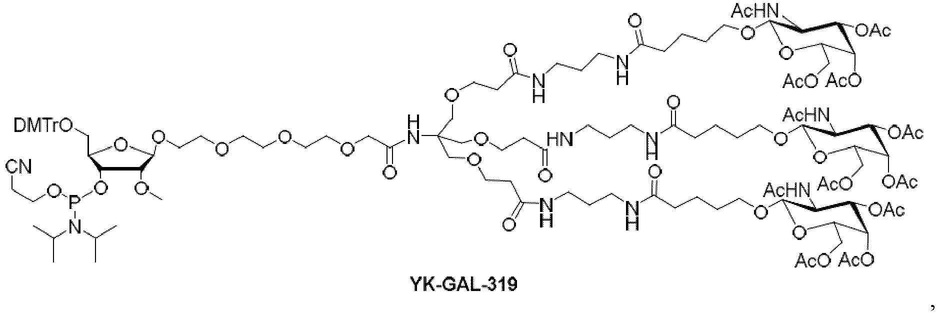 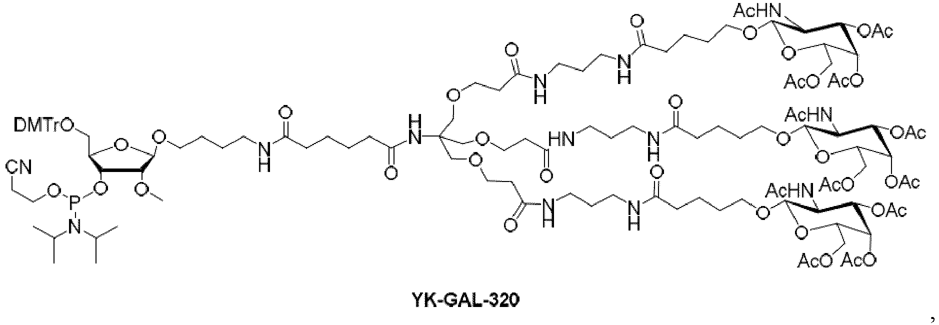 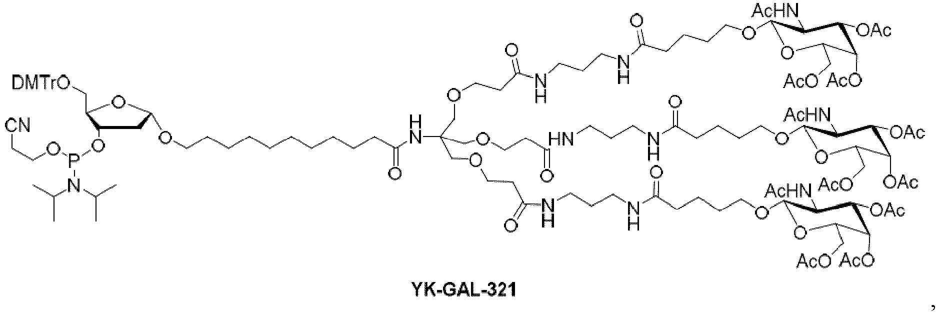 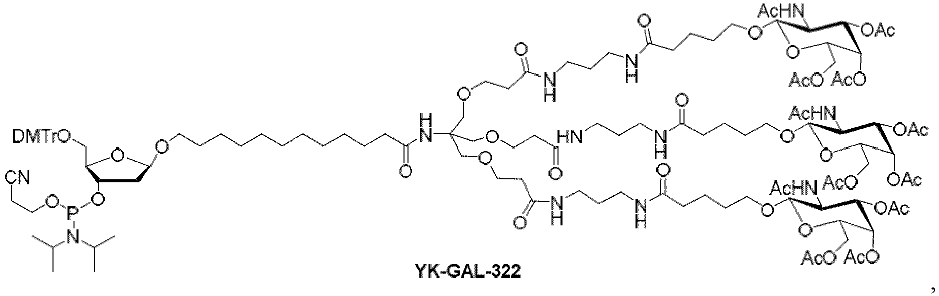 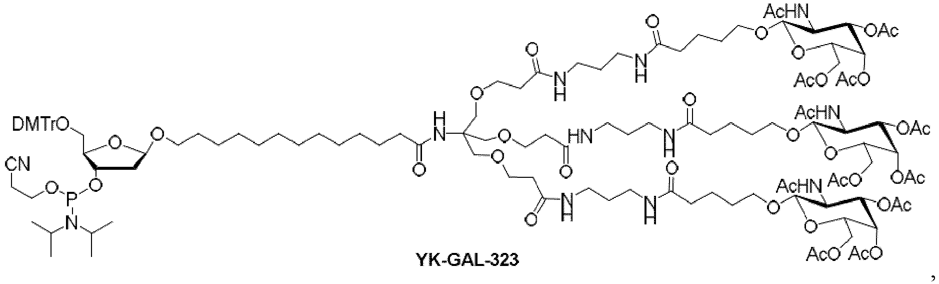 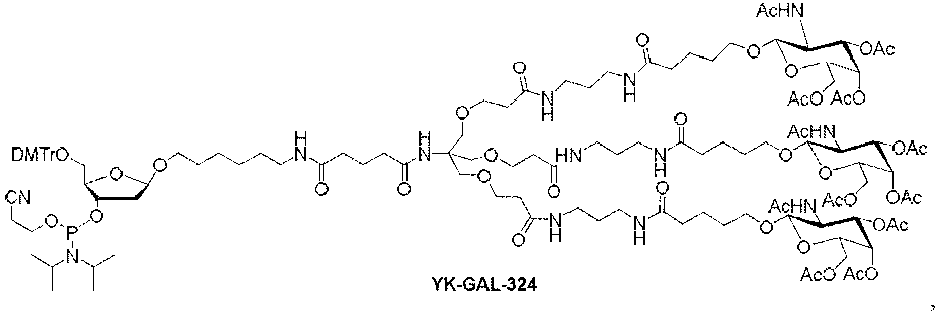 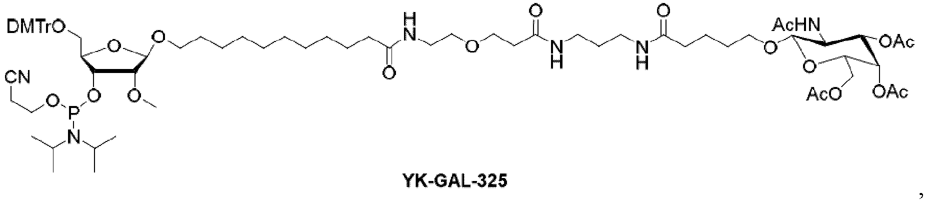 or

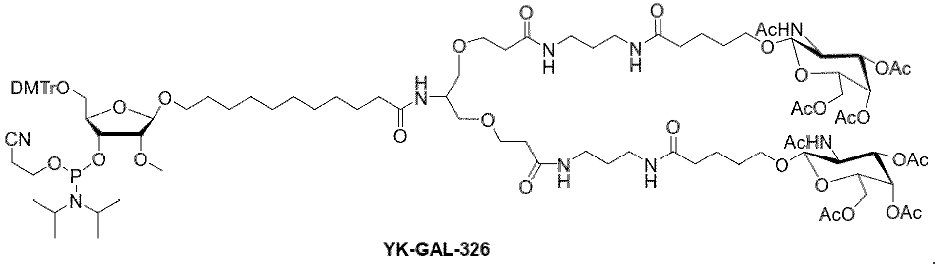

18. The compound represented by formula (I) or the pharmaceutically acceptable salt thereof according to any one of claims 1 to 6, which is capable of binding to asialoglycoprotein receptor (ASGPR).

19. A conjugate comprising an oligonucleotide and a GalNAc moiety, wherein the conjugate has a structure of 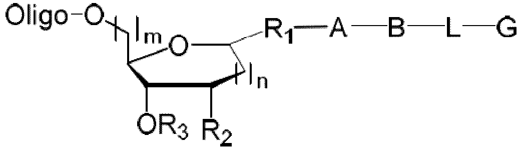 or 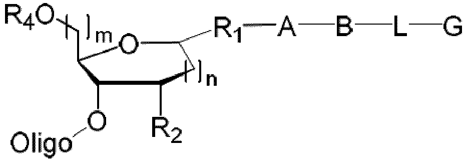 wherein, Oligo represents oligonucleotide, and R₁, R₂, R₃, R₄, A, B, L, G, m and n are the same as those defined in claim 1.

20. The conjugate according to claim 19, wherein the hydroxyl protecting group acetyl in the N-acetyl-galactosamine of G is removed.

21. The conjugate according to claim 19 or 20, wherein the oligonucleotide is a non-thio oligonucleotide or a thio oligonucleotide.

22. The conjugate according to claim 21, wherein the non-thio oligonucleotide and the GalNAc moiety are linked by a phosphate bond.

23. The conjugate according to claim 21, wherein the thio-oligonucleotide and the GalNAc moiety are linked by a phosphorothioate bond.

24. The conjugate according to claim 21, wherein the oligonucleotide is a small interfering RNA (siRNA), a DNA, a micro RNA (miRNA), a small activating RNA (saRNA), a small guide RNA (sgRNA), a transfer RNA (tRNA), an antisense oligonucleotide (ASO), or an aptamer.

25. The conjugate according to claim 24, wherein each nucleotide in the antisense oligonucleotide (ASO) or small interfering RNA (siRNA) is independently a modified or unmodified nucleotide.

26. The conjugate according to claim 21, wherein the oligonucleotide modulates expression of a target gene.

27. A pharmaceutical composition comprising the conjugate according to any one of claims 19 to 26 and at least one pharmaceutically acceptable excipient.

28. Use of the conjugate according to any one of claims 19 to 26 or the pharmaceutical composition according to claim 27 in the manufacture of a medicament for treating and/or preventing a pathological condition or disease caused by the expression of a specific gene in a hepatocyte; optionally, the specific gene is hepatitis B virus gene, angiopoietin 3 gene or apolipoprotein C3 gene.

29. The use according to claim 28, wherein the disease is selected from chronic liver disease, hepatitis, liver fibrosis, liver proliferative disease and dyslipidemia; optionally, the dyslipidemia is hypercholesterolemia, hypertriglyceridemia or atherosclerosis.

30. A method for inhibiting the expression of a specific gene in a hepatocyte, comprising contacting the hepatocyte with an effective amount of the conjugate according to any one of claims 19 to 26 or the pharmaceutical composition according to claim 27;
optionally, the specific gene is selected from one of the following gene: proprotein convertase subtilisin/kexin type 9 gene (PCSK9), Apo B, ApoC, ANGPTL3, SCD1, FVII, p53, HBV, and HCV;
optionally, the specific gene is proprotein convertase subtilisin/kexin type 9 gene (PCSK9), hepatitis B virus gene, angiopoietin-like protein 3 gene, or apolipoprotein C3 gene.

31. A kit comprising the conjugate according to any one of claims 19 to 26.
